(19) Europäisches Patentamt
European Patent Office
Office européen des brevets

(11) **EP 4 410 199 B1**

(12) **EUROPEAN PATENT SPECIFICATION**

(45) Date of publication and mention
of the grant of the patent:
**01.07.2026 Bulletin 2026/27**

(21) Application number: **24182772.4**

(22) Date of filing: **19.11.2013**

(51) International Patent Classification (IPC):
**G16H 40/63** (2018.01)   **G16H 50/30** (2018.01)
**A61B 5/00** (2006.01)   **A61B 5/0205** (2006.01)
**A61B 5/021** (2006.01)   **A61B 5/024** (2006.01)
**A61B 5/145** (2006.01)

(52) Cooperative Patent Classification (CPC):
**A61B 5/7264; A61B 5/7275; A61B 5/743;**
**G16H 40/63; G16H 50/30;** A61B 5/0205

(54) **USER INTERFACE FOR PATIENT RISK ANALYSIS SYSTEM**

BENUTZERSCHNITTSTELLE FÜR EIN PATIENTENRISIKOANALYSESYSTEM

INTERFACE UTILISATEUR POUR SYSTÈME D'ANALYSE DE RISQUE DE PATIENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **19.11.2012 US 201261727820 P**
**07.03.2013 US 201361774274 P**
**14.03.2013 US 201313826441**

(43) Date of publication of application:
**07.08.2024 Bulletin 2024/32**

(62) Document number(s) of the earlier application(s) in
accordance with Art. 76 EPC:
**20187516.8 / 3 767 636**
**13855989.3 / 2 919 644**

(73) Proprietor: **Etiometry Inc.**
**Dedham, MA 02026 (US)**

(72) Inventors:
• **BARONOV, Dimitar V.**
**Weston, MA 02493 (US)**
• **BUTLER, Evan J.**
**New Heaven, CT 06511 (US)**
• **LOCK, Jesse M.**
**Winchester, MA 01890 (US)**

(74) Representative: **Kaminski Harmann**
**Patentanwälte AG**
**Landstrasse 124**
**9490 Vaduz (LI)**

(56) References cited:
**EP-B1- 1 844 725**   **US-A1- 2003 065 535**
**US-A1- 2007 239 043**   **US-A1- 2012 022 336**

**Description**

**Background Art**

The present disclosure relates to systems and methods for risk-based patient monitoring. More particularly, the present disclosure relates to systems and methods for assessing the current and future risks of a patient by combining data of the patient from various different sources.

Practicing medicine is becoming increasingly more complicated due to the introduction of new sensors and treatments. As a result, clinicians are confronted with an avalanche of patient data, which needs to be evaluated and well understood in order to prescribe the optimal treatment from the multitude of available options, while reducing patient risks. One environment where this avalanche of information has become increasingly problematic is the Intensive Care Unit (ICU). There, the experience of the attending physician and the physician's ability to assimilate the available physiologic information have a strong impact on the clinical outcome. It has been determined that hospitals which do not maintain trained intensivists around the clock experience a 14.4% mortality rate as opposed to a 6.0% rate for fully staffed centers. It is estimated that raising the level of care to that of average trained physicians across all ICUs can save 160,000 lives and $4.3 Bn annually. As of 2012, there is a shortage of intensivists, and projections estimate the shortage will only worsen, reaching a level of 35% by 2020.

The value of experience in critical care can be explained by the fact that clinical data in the ICU is delivered at a rate far greater than even the most talented physician can absorb, and studies have shown that errors are six times more likely under conditions of information overload and eleven time more likely with an acute time shortage. Moreover, treatment decisions in the ICU heavily rely on clinical signs that are not directly measurable, but are inferred from other physiologic information. Thus clinician expertise and background play a more significant role in the minute to minute decision making process. Not surprisingly, this leads to a large variance in hidden parameter estimation. As an example, although numerous proxies for cardiac output are continuously monitored in critical care, studies have demonstrated poor correlation between subjective assessment by clinicians, and objective measurement by thermodilution. Experienced intensivists incorporate this inherent uncertainty in their decision process by effectively conducting risk management, i.e. prescribing the treatment not only based on the most probable patient state, but also weighing in the risks of the patient being in other more adverse states. From this perspective, experienced intensivists confront the data overload in intensive care by converting the numerous heterogeneous signals from patient observations into a risk assessment.

Therefore, there is a clear need for a decision support system in the ICU that achieves a paradigm shift from signal-based patient monitoring to risk based patient monitoring, and consequently helps physicians overcome the barrage of data in the ICU.

Document EP 1 844 725 B1, titled "Risk assessment for planned trajectories" by Hartlep et al., relates to a method for planning the placement of a medical device by assessing the risk of a planned trajectory in relation to critical anatomical structures.

Document US 2007/0239043 A1, titled "Method and Apparatus for Arrhythmia Episode Classification" by Patel et al., relates to a method for classifying arrhythmia episodes from an implantable medical device using Bayesian networks to determine the likelihood of a particular diagnosis.

Document US 2012/0022336 A1, titled "Iterative Probabilistic Parameter Estimation Apparatus and Method of Use Therefor" by Teixeira, relates to a system for estimating biomedical parameters by iteratively fusing data from multiple sensors using a probabilistic, dynamic state-space model.

Document US 2003/0065535 A1, titled "Diagnosing Inapparent Diseases from Common Clinical Tests Using Bayesian Analysis" by Karlov et al., relates to a system for diagnosing diseases, particularly inapparent ones, from clinical test data using Bayesian probability analysis.

**Summary of the Embodiments**

A first embodiment teaches a computer-implemented method of graphically communicating, to a user, patient risks for an individual patient, and includes the steps of receiving, from a device, data relating to a plurality of possible clinical patient states in which the patient may be classified; causing the display, on a display device, of a plurality of graphical indicators, each of the plurality of graphical indicators corresponding to one of the plurality of possible clinical patient states, each of the plurality of graphical indicators graphically identifying the probability that the patient is in a corresponding clinical patient state at a given point in a range of time, the plurality of graphical indicators configured to indicate a hazard level; and causing the display, on the display device, of a timeline controller configured to allow a user to dynamically select a plurality of points in time over the range of time, the graphical indicators changing dynamically in response to a specification by the user of one of the plurality of points in time to display the evolution of the plurality of patient states over the range of time.

Graphical properties of the graphical indicators may indicate hazard levels. For example, in some embodiments, the plurality of graphical indicators are arranged on the display device segregated by hazard level so as to indicate a hazard level for each of the graphical indicators and in some embodiments the color of each of the plurality of graphical indicators indicates the hazard level associated with each such graphical indicator.

In some embodiments, the step of receiving, from a device, data relating to a plurality of possible clinical patient states in which the patient may be classified, includes the step of receiving data identifying the plurality of possible clinical patient states in which the patient may be classified, and for each of the plurality of possible clinical patient states, also includes the steps of receiving data identifying the probability that the patient is in each of the possible patient states over the range of time; and receiving data identifying a hazard level into which each of the possible patient states is categorized.

Bar charts may be used as graphical indicators. For example, in some embodiments, the arrangement of graphical indicators on the display device includes a bar chart having a plurality of bars, each bar associated with one of a plurality of hazard levels; and each of the plurality of graphical indicators is a bar in the bar chart. Indeed, in some embodiments, a bar within the bar chart may include a plurality of graphical indicators within a single hazard level. In addition, in some embodiments, each of the bars has a length corresponding to the probability that the patient is in the corresponding clinical patient state at a given point in time. Further, in some embodiments, the hazard level of each of the graphical indicators is expressed by the color of said graphical indicator.

In some embodiments, the display device is a touch screen; and the dynamic controller is a slider displayed on the touch screen.

Some embodiments also include displaying a graphical indicator configured to represent the utility of an invasive measurement, the utility of an invasive measurement expressing a divergence between: a first patient state distribution associated with the plurality of possible clinical patient states based on a plurality of internal state variables associated with a time interval, the plurality of internal state variables including an internal state variable resulting from the invasive measurement; and a simulated patient state distribution associated with the plurality of possible clinical patient states based on the same plurality of internal state variables associate with the same time interval, but excluding the internal state variable resulting from the invasive measurement. Further, in some embodiments, the first patient state distribution is weighted by hazard level.

Some embodiments also include displaying a state variable data control feature that, when activated by the user, causes the display device to display internal state variables on which the patient states are based.

Some embodiments also include displaying a tag creation feature that, when activated by the user, causes the display device to display a tag mark associated with the timeline controller at a selected point within the range of time, and some even include displaying a tag note creation field configured to permit annotation, by the user, of information associated with the tag.

A non-transient computer readable medium having computer-executable code for graphically communicating, to a user, patient risks for an individual patient, includes code for receiving, from a device, data relating to a plurality of possible clinical patient states in which the patient may be classified; code for causing the display, on a display device, of a plurality of graphical indicators, each of the plurality of graphical indicators corresponding to one of the plurality of possible clinical patient states, each of the plurality of graphical indicators graphically identifying the probability that the patient is in a corresponding clinical patient state at a given point in a range of time, the plurality of graphical indicators configured to indicate a hazard level; and code for causing the display, on the display device, of a timeline controller configured to allow a user to dynamically select a plurality of points in time over the range of time, the graphical indicators changing dynamically in response to a specification by the user of one of the plurality of points in time to display the evolution of the plurality of patient states over the range of time.

In some embodiments, the code is configured to arrange the graphical indicators on the display device segregated by hazard level so as to indicate a hazard level for each of the graphical indicators.

In some embodiments, the code is configured to display the graphical indicators in color, such that the color of each of the plurality of graphical indicators indicates the hazard level associated with each such graphical indicator.

In some embodiments, the code for receiving, from a device, data relating to a plurality of possible clinical patient states in which the patient may be classified, includes: code for receiving data identifying the plurality of possible clinical patient states in which the patient may be classified, and for each of the plurality of possible clinical patient states, further includes: code for receiving data identifying the probability that the patient is in each of the possible patient states over the range of time; and code for receiving data identifying a hazard level into which each of the possible patient states is categorized.

Some embodiments use bar charts. For example, in some embodiments, the arrangement of graphical indicators on the display device includes a bar chart having a plurality of bars, each bar associated with one of a plurality of hazard levels; and each of the plurality of graphical indicators is a bar in the bar chart. Further, in some embodiments, a bar within the bar chart may include a plurality of graphical indicators within a single hazard level. In addition, in some embodiments, each of the bars has a length corresponding to the probability that the patient is in the corresponding clinical patient state at a given point in time. Also, in some embodiments, the hazard level of each of the graphical indicators is expressed by the color of said graphical indicator.

In some embodiments, the display device is a touch screen; and the dynamic controller is a slider displayed on the touch screen.

Some embodiments further include code for displaying a graphical indicator configured to represent the utility of an invasive measurement, the utility of an invasive measurement expressing a divergence between: a first patient state distribution associated with the plurality of possible clinical patient states based on a plurality of internal state variables associated with a time interval, the plurality of internal state variables including an internal state variable resulting from the invasive measurement; and a simulated patient state distribution associated with the plurality of possible clinical patient states based on the same plurality of internal state variables associate with the same time interval, but excluding the internal state variable resulting from the invasive measurement. Indeed, in some embodiments, the first patient state distribution is weighted by hazard level.

In some embodiments, the code further includes code for displaying a state variable data control feature that, when activated by the user, causes the display device to display internal state variables on which the patient states are based.

In some embodiments, the code further includes code for displaying a tag creation feature that, when activated by the user, causes the display device to display a tag mark associated with the timeline controller at a selected point within the range of time. Further, in some embodiments, the code further includes code for displaying a tag note creation field configured to permit annotation, by the user, of information associated with the tag.

A system for communicating, to a user, patient risks associated with a specific patient includes a display device; a computer processor configured to display a graphical user interface on the display device, the graphical user interface including: a plurality of graphical indicators, each of the plurality of graphical indicators corresponding to one of a plurality of possible clinical patient states for the patient, and identifying the probability that the patient is in a corresponding clinical patient state at a given point in a range of time, the plurality of graphical indicators configured to indicate a hazard level; and a timeline controller configured to allow the user to dynamically select a plurality of points in time over the range of time, the graphical indicators changing dynamically in response to a specification by the user of one of the plurality of points in time to display the evolution of the plurality of patient states over the range of time.

Further, in some embodiments, the plurality of graphical indicators are arranged on the display device segregated by hazard level so as to indicate a hazard level for each of the graphical indicators.

In some embodiments, the color of each of the plurality of graphical indicators indicates the hazard level associated with each such graphical indicator.

Some embodiments of a system use bar charts. For example, in some embodiments, the arrangement of graphical indicators on the display device includes a bar chart having a plurality of bars, each bar associated with one of a plurality of hazard levels; and each of the plurality of graphical indicators includes a bar in the bar chart. Further, a bar within the bar chart may include a plurality of graphical indicators within a single hazard level. Also, each of the bars may have a length corresponding to the probability that the patient is in the corresponding clinical patient state at a given point in time. Alternately, or in addition, the hazard level of each of the graphical indicators may be expressed by the color of said graphical indicator.

In some embodiments of a system, the display device includes a touch screen; and the dynamic controller is a slider displayed on the touch screen.

Some embodiments of a system for communicating, to a user, patient risks associated with a specific patient according to claim 29, additionally include a graphical indicator configured to represent the utility of an invasive measurement, the utility of an invasive measurement expressing a divergence between: a first patient state distribution associated with the plurality of possible clinical patient states based on a plurality of internal state variables associated with a time interval, the plurality of internal state variables including an internal state variable resulting from the invasive measurement; and a simulated patient state distribution associated with the plurality of possible clinical patient states based on the same plurality of internal state variables associate with the same time interval, but excluding the internal state variable resulting from the invasive measurement. Further, in some embodiments, the first patient state distribution is weighted by hazard level.

In some embodiments, the graphical user interface further includes: a state variable data control feature that, when activated by the user, causes the display device to display internal state variables on which the patient states are based.

In some embodiments, the graphical user interface further includes: a tag creation feature that, when activated by the user, causes the display device to display a tag mark associated with the timeline controller at a selected point within the range of time. Indeed, some embodiments further include a tag note creation field configured to permit annotation, by the user, of information associated with the tag.

## Brief Description of the Drawings

It should be understood at the outset that although illustrative implementations of one or more embodiments of the present disclosure are provided below, the disclosed systems and/or methods may be implemented using any number of techniques, whether currently known or in existence. The disclosure should in no way be limited to the illustrative implementations, drawings, and techniques illustrated below, including the exemplary designs and implementations illustrated and described herein, but may be modified within the scope of the appended claims along with their full scope of equivalents.

In the drawings;

FIG. 1 illustrates conceptually a medical care risk based monitoring environment in accordance with the disclosure;

FIG. 2A illustrates conceptually a basic schematic of the physiology observer module in accordance with the disclosure;

FIGS. 2B-D illustrate conceptually exemplary graphs of probability density functions for select ISVs as generated by the physiology observer module in accordance with the disclosure;

FIG. 3 illustrates conceptually a non-limiting example of a physiology observer process in accordance with the disclosure;

FIG. 4 illustrates conceptually a non-limiting example of the physiology observer process in accordance with the disclosure;

FIG. 5 illustrates conceptually a time line, wherein back propagation is used to incorporate information in accordance with the disclosure;

FIG. 6 illustrates conceptually an example of a process involving mean arterial blood pressure (ABPm) in accordance with the disclosure;

FIG. 7 illustrates conceptually an example of resampling in accordance with the disclosure;

FIG. 8 illustrates conceptually a clinical trajectory interpreter module using joined Probability Density Functions of ISVs and performing state probability estimation to calculate the probabilities of different patient states in accordance with the disclosure;

FIG. 9 illustrates conceptually a non-limiting example of a definition of a patient state employed by the clinical trajectory interpreter module in accordance with the disclosure;

FIG. 10 illustrates conceptually a non-limiting example of how a clinical trajectory interpreter module may employ the definition of patient states to assign probabilities that the patient may be classified under each of the four possible patient states at a particular point of time;

FIG. 11 illustrates conceptually an alternative approach of estimating the probabilities for different patient states in accordance with the disclosure;

FIG. 12 illustrates conceptually a non-limiting example of a definition of patient states assigned with hazard levels by the clinical trajectory interpreter module in accordance with the disclosure;

FIG. 13 illustrates conceptually patient states and their respective probabilities organized into tree graphs called etiologies in accordance with the disclosure;

FIG. 14 illustrates conceptually an exemplary etiology tree for a given set of patient states and physiologic variables in accordance with the disclosure;

FIG. 15 illustrates conceptually a method for calculating the utility of different measurements in accordance with the disclosure;

FIG. 16 illustrates conceptually one possible realization of integration of external computation generated from third party algorithms in accordance with the disclosure;

FIG. 17 illustrates conceptually an example of integration instructions of an external computation in accordance with the disclosure;

FIG. 18 illustrates conceptually an additional example of integration instructions of an external computation in accordance with the disclosure;

FIG. 19 illustrates conceptually example functionalities of the visualization and user interactions module in accordance with the disclosure;

FIG. 20 illustrates conceptually an example of a summary view that may convey on a single screen a risk profile for each patient in a particular hospital unit in accordance with the disclosure;

FIG. 21 illustrates conceptually one possible realization of a view describing the ongoing risks of the patient in accordance with the disclosure;

FIG. 22 illustrates conceptually how the slider on top of the patient view may be utilized in reviewing the history of the patient risks in accordance with the disclosure;

FIG. 23 illustrates how the user can navigate the etiology tree by clicking on the composite patient state and viewing the constituent patient states in accordance with the disclosure;

FIG. 24 illustrates conceptually how in the same framework the user may view the predicted risks for the patient by sliding the slider ahead of current time in accordance with the disclosure;

FIG. 25 illustrates conceptually how the user may choose to set an alarm for a particular risk in accordance with the disclosure;

FIG. 26 illustrates conceptually yet another possible visualization of the patient risk trajectory, i.e., the evolution of the patient states' probabilities associated with particular risks in accordance with the disclosure;

FIG. 27 illustrates conceptually how the system may directly visualize the probability density functions of various internal state variables in accordance with the disclosure;

FIG. 28 illustrates conceptually an example of a tagging feature that a user interface may implement in accordance with the disclosure;

FIG. 29 illustrates conceptually a Newsfeed view of the user interface in accordance with the disclosure;

FIG. 30 illustrates conceptually a Condition Summary View of the user interface in accordance with the disclosure;

FIG. 31 illustrates conceptually the ability for the user interface to include and display reference material, which may be accessed through the Internet; or stored within the system in accordance with the disclosure;

FIG. 32 illustrates conceptually a general Dynamic Bayesian Network (DBN) that may be employed to capture the physiology model of the HLHS stage 1 palliation patients in accordance with the disclosure;

FIG. 33 illustrates conceptually several equations that may be used to model the dynamics of the HLHS stage 1 physiology in accordance with the disclosure;

FIG. 34 illustrates conceptually example equations that may be used to abstract the relationships between the dynamic variables in the model and the derived variables in accordance with the disclosure;

FIG. 35 illustrates conceptually a possible observation model that may be used to relate the derived variables with the available sensor data in accordance with the disclosure;

FIG. 36 illustrates conceptually possible attributes, patient states, and etiology tree that may be used by the clinical trajectory interpreter module in the case of the HLHS Stage 1 population in accordance with the disclosure;

FIG. 37 illustrates conceptually one possible environment in which the risk based monitoring system can be applied to assist clinicians in deciding whether to apply a particular treatment in accordance with the disclosure;

FIG. 38 illustrates conceptually a non-limiting example set of patient states relevant to blood transfusion that may be used to inform the blood transfusion decision in accordance with the disclosure;

FIG. 39 illustrates conceptually another application of the risk-based monitoring system, applying standardized medical plans in accordance with the disclosure;

FIG. 40 illustrates conceptually an example application of the risk based monitoring system combined with a specific type of standardized clinical plan in accordance with the disclosure;

FIG. 41 illustrates conceptually an example risk stratification that may be employed by the system in the context of Nitric Oxide treatment in accordance with the disclosure;

FIG. 42 illustrates conceptually possible patient states that may describe the clinical trajectory of an ADHD patient in accordance with the disclosure;

FIG. 43 lists the available patient evaluation modalities as M1, M2, and M3;

FIG. 44 illustrates conceptually a dynamic model of the patient evolution from state to state abstracted by a Dynamic Bayesian Network in accordance with the disclosure;

FIG. 45 illustrates conceptually an alternative embodiment for two predictions of how the patient state can transition in a single month given medication change or a dosage change in accordance with the disclosure;

FIG. 46 illustrates conceptually one possible embodiment and scenario of visualization displaying the patient clinical trajectory and risks in accordance with the disclosure;

FIG. 47 illustrates conceptually an evaluation of the patient and the patient trajectory at week 9 at which point risk based patient monitoring system determines a probability distribution function for the state of the patient for each of the past nine weeks in accordance with the disclosure;

FIG. 48 shows a follow-up evaluation based on teacher and parent Vanderbilt diagnosis in accordance with the disclosure;

FIG. 49 shows consequent evaluation based on all available measurements--office visit, parent and teacher evaluation, which establishes high probability for significant improvement in accordance with the disclosure;

FIG. 50 shows yet another follow-up at which point it is established that the patient is most probably stably improved, and has been stably improved between the two evaluations in accordance with the disclosure;

FIG. 51 shows a follow-up evaluation of the patient and the patient trajectory in the absence of measurements, wherein due to the lack of recent observation, the uncertainty is increasing in accordance with the disclosure;

FIG. 52 shows the state of this uncertainty given a full patient evaluation (all measurement modalities) in accordance with the disclosure; and

FIG. 53 illustrates yet another possible visualization from the described system output. It shows possible patient state transitions under changes of treatment plan, e.g., change of medication in accordance with the disclosure.

Figure 54. An example implementation of the "Unit Summary View" screen. The screen features a tile for each patient, which can all be viewed via the scroll bar on the right side of the screen. Each patient tile has their location, patient details, and condition over an approximate timeline.

Figure 55. An example implementation of the "Patient View Risks" screen. The screen illustrates the probability of the patient (indicated at the top of the screen) being in each possible state at the current time.

Figure 56. An example implementation of the "Patient View Risks" screen. The screen illustrates the probability of the patient being in each possible state at some past time.

Figure 57. An example implementation of the "Patient View Risks" screen. The screen features the patient state that the user is viewing at the specified red mark on the timeline.

Figure 58. An example implementation of the "Patient View Risks" screen. The screen shows the ability to drag one possible state into another to create a composite state.

Figure 59. An example implementation of the "Patient View Risks" screen. The screen shows the composite state that is resulting from two separate states.

Figure 60. An example implementation of the "Patient View Risks" screen. The screen shows that by clicking on the composite state, the user can view the states that make up the composite state.

Figure 61. An example implementation of the "Patient View Risks" screen. The screen features an overlay over the patient state view. This overlay provides the ability to manually set alarms that will be triggered based on the changes in the probability states and other events of interest, as well as the ability to specify the names of those that will be notified with the alarms.

Figure 62. An example implementation of the "Patient View Risks" screen. The screen shows the alarm that was set corresponding to one of the probability states.

Figure 63. An example implementation of the "Patient View Risk Trajectory" screen. The screen features a graph where the independent axis is time and dependent axis is the probability of the patient being in a particular state. The bars above the graph give the user ability to add or remove possible states.

Figure 64. An example implementation of the "Patient View Measures" screen. The screen features plots that show the patient's physiological measures over time.

Figure 65. An example implementation of the "Patient View Chart" screen. This screen is a digital version of the current patient charts that are utilized by a physician.

Figure 66. An example workflow for the user interface. The expected trajectory of the user's actions is as follows: (1) review the current risks, (2) review risk trajectory, (3) review physiological measurements, (4) review chart, and finally, (5) take notes on the patient.

## Detailed Description of Specific Embodiments

Technologies are provided herein for providing risk-based patient monitoring of individual patients to clinical personnel. The technologies described herein can be embodied as a monitoring system for critical care, which combines data from various bedside monitors, electronic medical records, and other patient specific information to assess the current and the future risks to the patient. The technologies can be also embodied as a decision support system that prompts the user with specific actions according to a standardized medical plan, when patient specific risks pass a predefined threshold. Yet another embodiment of the described technologies is an outpatient monitoring system which combines patient and family evaluation, together with information about medication regiments and physician evaluations to produce a risk profile of the patient, continuously track its clinical trajectory, and provide decision support to clinicians as regarding when to schedule a visit or additional tests.

System Modules And Interaction

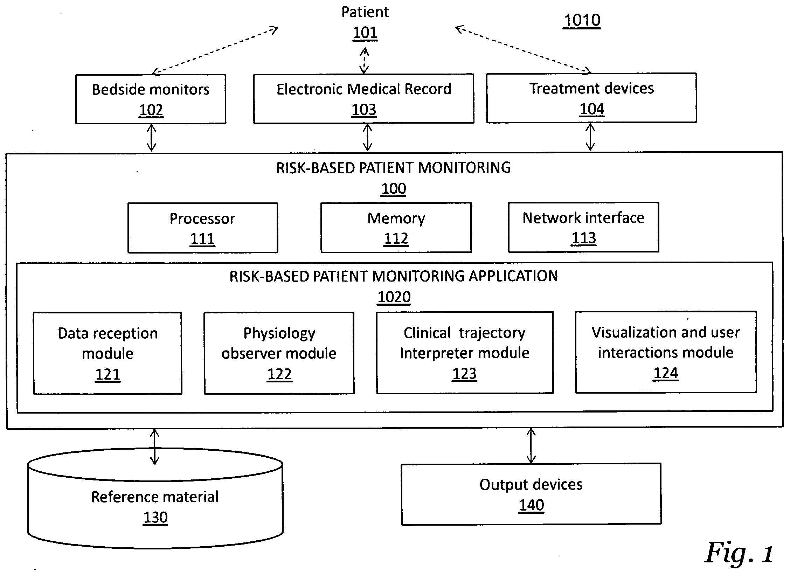

Referring now to the Figures, FIG. 1 illustrates a medical care risk based monitoring environment 1010 for providing health providers, such as physicians, nurses, or other medical care providers, risk-based monitoring in accordance with various embodiments of the present disclosure. A patient 101 may be coupled to one or more physiological sensors or bedside monitors 102 that may monitor various physiological parameters of the patient. These physiological sensors may include but are not limited to, a blood oximeter, a blood pressure measurement device, a pulse measurement device, a glucose measuring device, one or more analyte measuring devices, an electrocardiogram recording device, amongst others. In addition, the patient may be administered routine exams and tests and the data stored in an electronic medical record (EMR) 103. The electronic medical record 103 may include but is not limited to stored information such as hemoglobin, arterial and venous oxygen content, lactic acid, weight, age, sex, ICD-9 code, capillary refill time, subjective clinician observations, patient self-evaluations, prescribed medications, medications regiments, genetics, etc. In addition, the patient 101 may be coupled to one or more treatment devices 104 that are configured to administer treatments to the patient. In various embodiments, the treatments devices 104 may include extracorporeal membrane oxygenator, ventilator, medication infusion pumps, etc.

By way of the present disclosure, the patient 101 may be afforded improved risk-based monitoring over existing methods. A patient specific risk-based monitoring system, generally referred to herein as system 100, may be configured to receive patient related information, including real-time information from bed-side monitors 102, EMR patient information from electronic medical record 103, information from treatment devices 104, such as settings, infusion rates, types of medications, and other patient related information, which may include the patient's medical history, previous treatment plans, results from previous and present lab work, allergy information, predispositions to various conditions, and any other information that may be deemed relevant to make an informed assessment of the possible patient conditions and states, and their associated probabilities. For the sake of simplicity, the various types of information listed above will generally be referred to hereinafter as "patient-specific information". In addition, the system may be configured to utilize the received information, determine the clinical risks, which then can be presented to a medical care provider, including but not limited to a physician, nurse, or other type of clinician.

The system, in various embodiments, includes one or more of the following: a processor 111, a memory 112 coupled to the processor 111, and a network interface 113 configured to enable the system to communicate with other devices over a network. In addition, the system may include a risk-based monitoring application 1020 that may include computer-executable instructions, which when executed by the processor 111, cause the system to be able to afford risk based monitoring of the patients, such as the patient 101.

The risk based monitoring application 1020 includes, for example, a data reception module 121, a physiology observer module 122, a clinical trajectory interpreter module 123, and a visualization and user interaction module 124. In an exemplary embodiment, the data reception module 121 may be configured to receive data from bedside monitors 102, electronic medical records 103, treatment devices 104, and any other information that may be deemed relevant to make an informed assessment regarding the patient's clinical risks, and any combination thereof of the preceding elements.

The physiology observer module 122 utilizes multiple measurements to estimate probability density functions (PDF) of internal state variables (ISVs) that describe the components of the physiology relevant to the patient treatment and condition in accordance with a predefined physiology model. The ISVs may be directly observable with noise (as a non-limiting example, heart rate is a directly observable ISV), hidden (as a non-limiting example, oxygen delivery ($DO_2$) defined as the flow of blood saturated oxygen through the aorta cannot be directly measured and is thus hidden), or measured intermittently (as a non-limiting example, hemoglobin concentration as measured from Complete Blood Count tests is an intermittently observable ISV).

In one embodiment, instead of assuming that all variables can be estimated deterministically without error, the physiology observer module 122 of the present disclosure provides probability density functions as an output. Additional details related to the physiology observer module 122 are provided herein.

The clinical trajectory interpreter module 123 may be configured, for example, with multiple possible patient states, and may determine which of those patient states are probable and with what probability, given the estimated probability density functions of the internal state variables. A patient state is defined as a qualitative description of the physiology at a particular point of a clinical trajectory, which is recognizable by medical practice, and may have implications to clinical decision-making. Examples of particular patient states include, but are not limited to, hypotension with sinus tachycardia, hypoxia with myocardial depression, compensated circulatory shock, cardiac arrest, hemorrhage, amongst others. In addition, these patient states may be specific to a particular medical condition, and the bounds of each of the patient states may be defined by threshold values of various physiological variables and data. In various embodiments, the clinical trajectory interpreter module 123 may determine the patient conditions under which a patient may be categorized using any of information gathered from reference materials, information provided by health care providers, other sources of information. The reference materials may be stored in a database or other storage device 130 that is accessible to the risk based monitoring application 1020 via network interface 113, for example. These reference materials may include material synthesized from reference books, medical literature, surveys of experts, physician provided information, and any other material that may be used as a reference for providing medical care to patients. In some embodiments, the clinical trajectory interpreter module 123 may first identify a patient population that is similar to the subject patient being monitored. By doing so, the clinical trajectory interpreter module 123 may be able to use relevant historical data based on the identified patient population to help determine the possible patient states.

The clinical trajectory interpreter module 123 is capable of also determining the probable patient states under which the patient can be currently categorized, given the estimated probability density functions of the internal state variables, as provided by physiology observer module 122. In this way, each of the possible patient states is assigned a probability value from 0 to 1. The combination of patient states and their probabilities is defined as the clinical risk to the patient. Additional details related to the clinical trajectory interpreter module 123 are provided herein.

Visualization and user interactions module 124 may be equipped to take the outputs of the data reception module 121 the physiology observer module 122, and the clinical trajectory interpreter module 123 and present them to the clinical personnel. The visualization and user interactions module 124 may show the current patient risks, their evolution through time, the probability density functions of the internal state variables as functions of time, and other features that are calculated by the two modules 122 and 123 as by-products and are informative to medical practice. Additionally, visualization and user interactions module 124 enables the users to set alarms based on the patient state probabilities, share those alarms with other users, take notes related to the patient risks and share those notes with other users, and browse other elements of the patient medical history. Additional details related to the visualization and user interactions module 124 are provided herein.

Physiology Observer

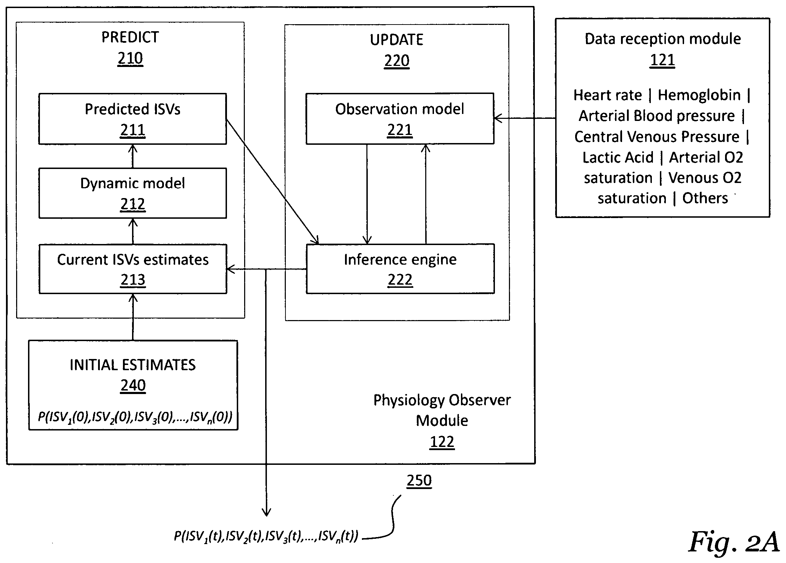

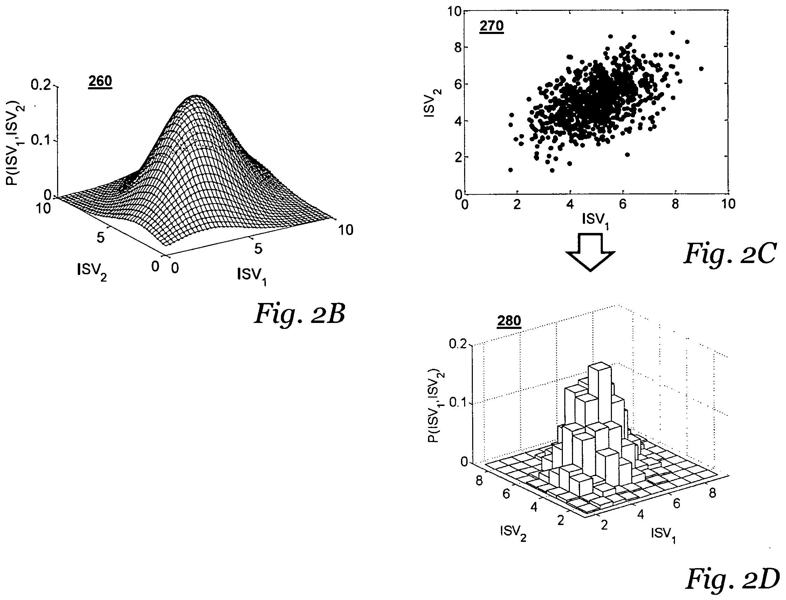

FIG. 2 illustrates a basic schematic of the physiology observer module 122, which utilizes two models of the patient physiology: a dynamic model 212 and an observation model 221. The dynamic model 212 captures the relationship arising between the internal state variables at some time $t_k$ and another close time $t_{k+1}$, thereby enabling modeling of the patient physiology as a system whose present state has information about the possible future evolutions of the system. Given the propensity of the patient physiology to remain at homeostasis through auto-regulation, there is a clear rational of introducing such memory in internal state variables that are indicative of the homeostasis, e.g., oxygen delivery and oxygen consumption.

The observation model 221 may capture the relationships between measured physiology variables and other internal state variables. Examples of such models include: a) the dependence of the difference between systolic and diastolic arterial blood pressures (also called pulse pressure) on the stroke volume; b) the relationship between heart rate, stroke volume, and cardiac output; c) the relationship between hemoglobin concentration, cardiac output and oxygen delivery; d) the relationship between the Vanderbilt Assessment Scale and the clinical state of an attention deficit and hyperactivity disorder patient; and e) any other dependence between measurable and therefore observable parameters and internal state variables.

The physiology observer module 122 functions as a recursive filter by employing information from previous measurements to generate predictions of the internal state variables and the likelihood of probable future measurements and then comparing them with the most recently acquired measurements. Specifically the physiology observer module 122 utilizes the dynamic model 212 in the predict step or mode 210 and the observation model 221 in the update step or mode 220. During the prediction mode 210, the physiology observer module 122 takes the estimated PDFs of ISVs 213 at a current time step $t_k$ and feeds them to the dynamic model 212, which produces predictions of the ISVs 211 for the next time step $t_{k+1}$. The is accomplished using the following equation:

$$P(ISVs(t_{k+1})|M(t_k)) = \int_{ISVs \in ISV} P(ISVs(t_{k+1})|ISVs(t_k))P(ISVs(t_k)|M(t_k))\,dISVs$$

where $ISVs(t_k) = \{ISV_1(t_k), ISV_2(tk), ISV_3(t_k),...ISV_n(t_k)\}$ and $M(t_k)$ is the set of all measurements up to time $t_k$. The probability $(I(t_k+1)|ISVs(t_k))$ defines a transition probability kernel describing the dynamic model 212, which defines how the estimated PDFs evolve with time. The probabilities $P(ISVs(t_k)|M(t_k))$ are provided by the inference engine 222 and are the posterior probabilities of the ISVs given the measurements acquired at the previous time step. During the update mode 210 of the physiology observer module 122, the predicted ISVs 211 are compared against the received measurements from data reception module 121 with the help of the observation model 221, and as a result the ISVs are updated to reflect the new available information. The inference engine 222 of module 122 achieves this update by using the predicted PDFs as a-priori probabilities, which are updated with the statistics of the measurements to achieve the posterior probabilities reflecting the current ISVs PDFs estimates 213. The inference engine 222 accomplished the update step 220 with the following equation which is Bayes' Theorem,

$$P\left(ISVs(t_{k+1})\big|M(t_{k+1})\right) = \frac{P\left(m_1(t_{k+1}), m_2(t_{k+1}),...m_n(t_{k+1})\big|ISVs(t_{k+1})\right)P(ISVs(t_{k+1})|M(t_k))}{P(M(t_{k+1}))}$$

where $P(m_1(t_{k+1}), m_2(t_{k+1}), ...m_n(t_{k+1})|ISVs(t_{k+1}))$
is the conditional likelihood kernel provided by the observation model 221 that determines how likely the currently received measurements are given the currently predicted ISVs.

At the initialization time, e.g., t=0, when no current estimate of ISVs PDFs is available, the physiology observer module 122 may utilize initial estimates 250, which may be derived from an educated guess of possible values for the ISVs or statistical analysis of previously collected patient data.

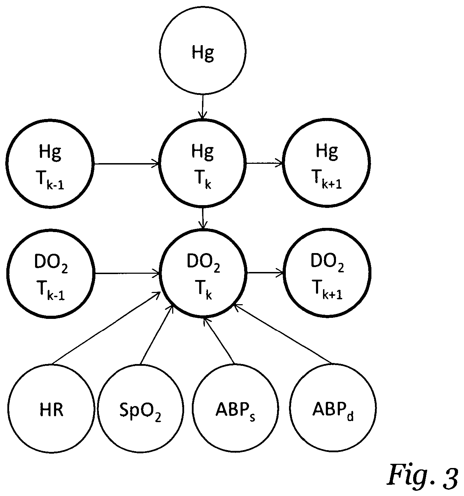

FIG. 3 illustrates a non-limiting example of models that enable the physiology observer in accordance with the present disclosure. While not directly observable, the management of oxygen delivery, DO2, is an important part of critical care. Therefore, precise estimation of DO2 can inform improved clinical practice. In the illustrated example, this estimation is achieved through the measurements of hemoglobin concentration (Hg), heart rate (HR), diastolic and systolic arterial blood pressures, and SpO2. The dynamic model 212 assumes that oxygen delivery is driven by a feedback process which stabilizes it against stochastic disturbances. Similarly, hemoglobin concentration is controlled around the norm value of 15 mg/dL. The observation model 221 takes into account the relationship between arterial oxygen saturation SpO2, hemoglobin concentration and arterial oxygen content CaO2, the dependence of the difference between systolic, ABPs, and diastolic, ABPd, arterial blood pressures (also called pulse pressure) on the stroke volume, and the relationship between heart rate, HR, stroke volume, SV, and cardiac output. The two models are abstracted as a Dynamic Bayesian Network (DBN), and the physiology observer module 122 utilizes the DBN to continuously track the oxygen delivery. A Dynamic Bayesian Network is a systematic way to represent statistical dependencies in terms of a graph whose vertices signify variables (observable and unobservable), and whose edges show causal relationships. Further descriptions of an exemplary DBN for DO2 estimation can be found in U.S. Provisional Application No. 61/699,492, filed on Sep. 11, 2012, entitled SYSTEMS AND METHODS FOR EVALUATING CLINICAL TRAJECTORIES AND TREATMENT STRATEGIES FOR OUTPATIENT CARE, Attorney Docket No. 3816/1003, and U.S. Provisional Application No. 61/684,241, filed on Aug. 17, 2012, entitled SYSTEM AND METHODS FOR PROVIDING RISK ASSESSMENT IN ASSISTING CLINICIANS WITH EFFICIENT AND EFFECTIVE BLOOD MANAGEMENT, Attorney Docket No. 3816/1001.

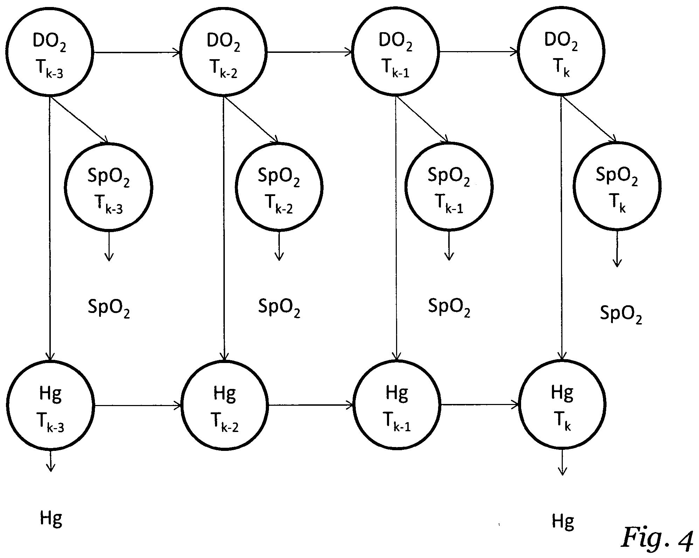

FIG. 4 depicts a non-limiting example of the physiology observer described above tracking DO2, but over a longer time interval, i.e., 4 time steps. In the observer, the main hidden ISV is the oxygen delivery variable (DO2). The two types of measurements, Hemoglobin (Hg) and oximetry (SpO2) are in dashed circles in FIG. 4. SpO2 is an example of the continuous or periodic measurements that the physiology observer module 122 receives from sensors, such as bedside monitors 102 and treatment devices 104 connected to the patient 101 that continuously report information. Hemoglobin (Hg) is an example of an intermittent or aperiodic measurement extracted from patient lab work that is available to the observer on a sporadic and irregular basis, and latent at times, relative to current system time. The physiology observer module 122 is capable of handling both types of measurements because, along with tracking the hidden ISVs, e.g. DO2, module 122 also continuously maintains estimates of the observed values for all types of measurements, even when measurements are not present. FIG. 4 depicts these estimates for the case of SpO2 and Hg. As can be seen, the SpO2 measurements are available regularly at each time step, whereas Hg is only available at two of the time steps.

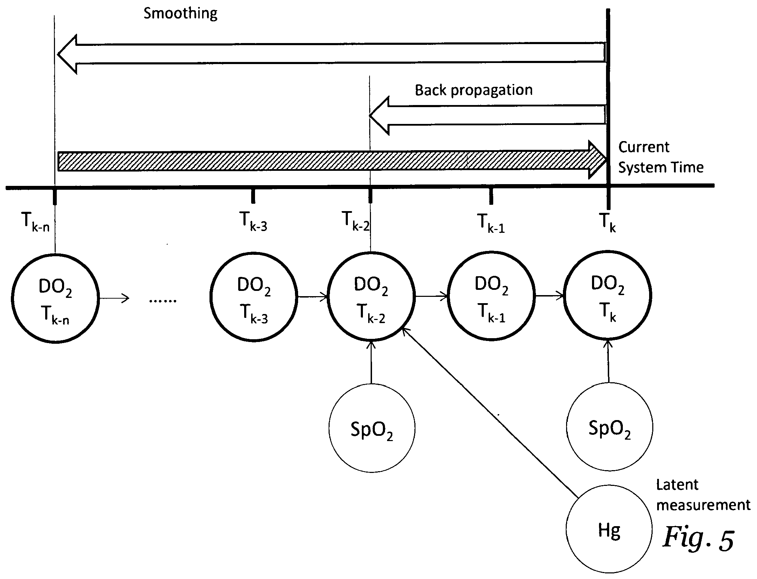

As mentioned above, certain measurements, such as Hemoglobin, are available to the system with an unknown amount of time latency, meaning the measurements are valid in the past relative to the current time and the time they arrive over the data communication links. The physiology observer module 122 may handle such out of sequence measurements using back propagation, in which the current estimates of the ISVs are projected back in time to the time of validity of the measurements, so that the information from the latent measurement can be incorporated correctly. FIG. 5 depicts such time line. In FIG. 5, hemoglobin arrives at the current system time, $t_k$, but is valid and associated back to the ISV (DO2) at time $T_{k-2}$. Back propagation is the method of updating the current ISVs probability estimates $P(ISVs(t_k)|M(t_k))$ with a measurement that is latent relative to the current time, $m(t_{k-n})$ Back propagation is accomplished in a similar manner to the prediction method described previously. There is a transition probability kernel, $P(ISVs(t_{k-n})|ISVs(t_k))$, that defines how the current probabilities evolve backwards in time. This can then be used to compute probabilities of the ISVs at time $t_{k-n}$ given the current set of measurements which excludes the latent measurement, as follows:

$$P(ISVs(t_{k-n})|M(t_k)) = \int_{ISVs \in ISV} P(ISVs(t_{k-n})|ISVs(t_k))P(ISVs(t_k)|M(t_k))dISVs$$

Once these probabilities are computed, the latent measurement information is incorporated using Bayes' rule in the standard update:

$$P(ISVs(t_{k-n})|M(t_k), m(t_{k-n})) = \frac{P\big(m(t_{k-n})\big|ISVs(t_{k-n})\big)P(ISVs(t_{k-n})|M(t_k)}{P(M(t_k),m(t_{k-n}))}$$

The updated probabilities are then propagated back to the current time $t_k$ using the prediction step described earlier. Back propagation can be used to incorporate the information.

Another functionality of the physiology observer module 122 includes smoothing. The care provider using the system 100 may be interested in the patient state at some past time. With smoothing, the physiology observer module 122 may provide a more accurate estimate of the patient ISVs at that time in the past by incorporating all of the new measurements that the system has received since that time, consequently providing a better estimate than the original filtered estimate of the overall patient state at that time to the user, computing $P(ISVs(t_k)|M(t_k))$. This is accomplished using the first step of back propagation in which the probability estimates at time $t_k$ which incorporate all measurements up to that time are evolved backwards to the time of interest $t_{k-n}$ using the defined transition probability kernel. This is also depicted in FIG. 5, in which the user is interested in the patient state at $t_{k-n}$ and the estimates are smoothed back to that time.

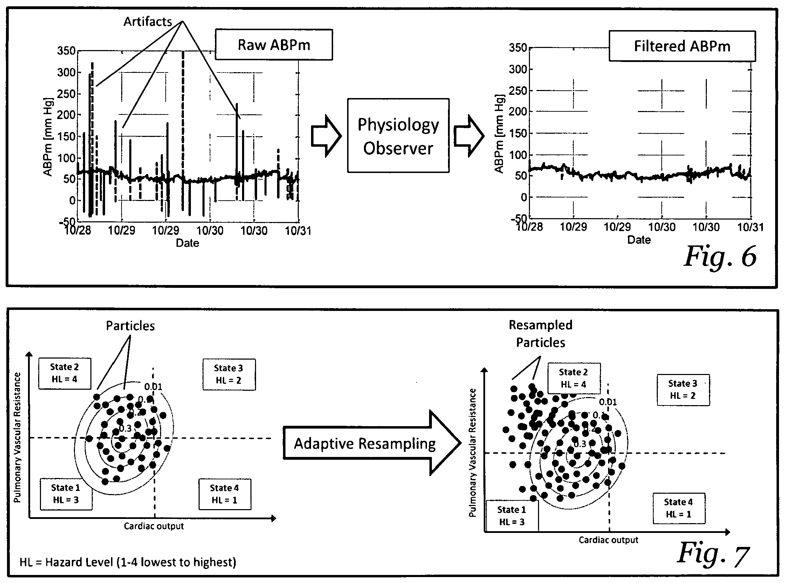

Because physiology observer module 122 maintains estimates of each of the measurements available to the system 100 based on physiologic and statistical models, module 122 may filter artifacts of the measurements that are unrelated to the actual information contained in the measurements. This is performed by comparing the newly acquired measurements with the predicted likelihoods of probable measurements given the previous measurements. If the new measurements are considered highly unlikely by the model, they are not incorporated in the estimation. The process of comparing the measurements with their predicted likelihoods effectively filters artifacts and reduces noise. FIG. 6 shows an example of such a process involving mean arterial blood pressure (ABPm). Because ABPm is collected using an intravenous catheter, the measured signals are often corrupted with artifacts that result in incorrect measurements when the catheter is used for medical procedures such as blood draws or line flushes. FIG. 6 shows the raw ABPm measurements prior to being processed by the physiology observer with the measurement artifacts identified, as well as the filtered measurements after being processed by the physiology observer module 122. As can be seen, the measurement artifacts have been removed and the true signal is left.

In various embodiments, physiology observer module 122 may utilize a number of algorithms for estimation or inference. Depending on the physiology model used, the physiology observer module 122 may use exact inference schemes, such as the Junction Tree algorithm, or approximate inference schemes using Monte Carlo sampling such as a particle filter, or a Gaussian approximation algorithms such as a Kalman Filter or any of its variants.

As discussed, the physiology model used by physiology observer module 122 may be implemented using a probabilistic framework known as a Dynamic Bayesian Network, which graphically captures the causal and probabilistic relationship between the ISVs of the system, both at a single instance of time and over time. Because of the flexibility this type of model representation affords, the physiology observer module 122 may utilize a number of different inference algorithms. The choice of algorithm is dependent on the specifics of the physiology model used, the accuracy of the inference required by the application, and the computational resources available to the system. Used in this case, accuracy refers to whether or not an exact or approximate inference scheme is used. If the physiology observer model is of limited complexity, then an exact inference algorithm may be feasible to use. In other cases, for more complex physiology observer models, no closed form inference solution exists, or if one does exist, it is not computationally tractable given the available resources. In this case, an approximate inference scheme may be used.

The simplest case in which exact inference may be used, is when all of the ISVs in the physiology model are continuous variables, and relationships between the ISVs in the model are restricted to linear Gaussian relationships. In this case, a standard Kalman Filter algorithm can be used to perform the inference. With such algorithm, the probability density function over the ISVs is a multivariate Gaussian distribution and is represented with a mean and covariance matrix.

When all of the ISV's in the model are discrete variables, and the structure of the graph is restricted to a chain or tree, the physiology observer module 122 may use either a Forward-backward algorithm, or a Belief Propagation algorithm for inference, respectively. The Junction Tree algorithm is a generalization of these two algorithms that can be used regardless of the underlying graph structure, and so the physiology observer module 122 may also use this algorithm for inference. Junction Tree algorithm comes with additional computational costs that may not be acceptable for the application. In the case of discrete variables, the probability distribution functions can be represented in a tabular form. It should be noted that in the case where the model consists of only continuous variables with linear Gaussian relationships, these algorithms may also be used for inference, but since it can be shown that in this case these algorithms are equivalent to the Kalman Filter, the |Kalman Filter is used |[hc1] the example algorithm.

When the physiology model consists of both continuous and discrete ISVs with nonlinear relationships between the variables, no exact inference solution is possible. In this case, the physiology observer module 122 may use an approximate inference scheme that relies on sampling techniques. The simplest version of this type of algorithm is a Particle Filter algorithm, which uses Sequential Importance Sampling. Markov Chain Monte Carlo (MCMC) Sampling methods may also be used for more efficient sampling. Given complex and non-linear physiologic relationships, this type of approximate inference scheme affords the most flexibility. A person reasonably skilled in the relevant arts will recognize that the model and the inference schemes employed by the physiology observer module may be any combination of the above described or include other equivalent modeling and inference techniques.

When using particle filtering methods, a resampling scheme is necessary to avoid particle degeneracy. The physiology observer may utilize an adaptive resampling scheme. As described in detail below, regions of the ISV state space may be associated with different patient states, and different levels of hazard to the patient. The higher the number, the more hazardous that particular condition is to the patient's health. In order to ensure accurate estimation of the probability of a particular patient condition, it may be necessary to have sufficient number of sampled particles in the region. It may be most important to maintain accurate estimates of the probability of regions with high hazard level and so the adaptive resampling approach guarantees sufficient particles will be sampled in high hazard regions of the state space. FIG. 7 illustrates an example of this resampling. State 1 and State 2 have the highest hazard level. The left plot depicts the samples generated from the standard resampling. Notice there are naturally more particles in state 1 and state 2 region because these states are most probable. The right plot shows the impact of the adaptive resampling. Notice how the number of samples in the areas of highest risk has increased significantly.

Clinical Trajectory Interpreter

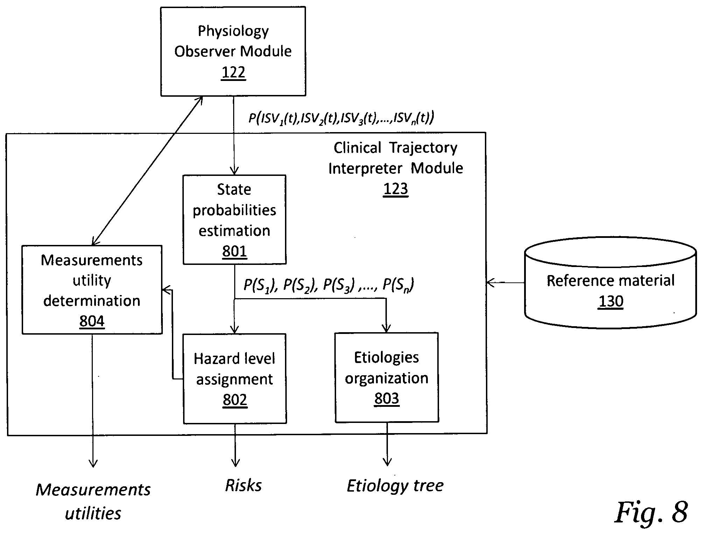

Referring now to FIG. 8, the Clinical Trajectory Interpreter 123 takes the joint Probability Density Functions of the ISVs from physiology observer module 122, and performs state probability estimation 801 to calculate the probabilities of different patient states. The Probability Density Functions of the ISVs may be defined in closed form, for example multidimensional Gaussians 260, or approximated by histogram 280 of particles 270, as illustrated in FIGS. 2B-D. In both cases, the probability density functions of the ISVs can be referred to as: P(ISV1(t), ISV2(t), ... , ISVn(t)), where t is the time they refer to. Given the internal state variables the patient state may be defined by a conditional probability density function: $P(S| ISV1,SV2,...,ISVn)$, where $S \in S_1, S_2,...,S_N$ represents all possible patient states $S_i$

Then determining the probability of the patient being in a particular state $S_i$ may be performed by the equation:

$$P\big(S_i(t)\big) =$$
$$\int_{-\infty}^{\infty} ... \int_{-\infty}^{\infty} P\big(S| ISV_1, ISV_2, ... , ISV_n\big)\, P\big(ISV_1(t), ISV_2(t), ... , ISV_n(t)\big)\, dISV_1 ... dISV_n$$

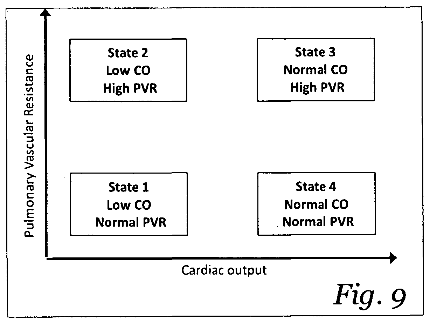

In case that P(ISV1(t), ISV2(t), ... , ISVn(t)) is defined by a closed form function such as multidimensional Gaussian 260, the integration may be performed directly. In case that P(ISV1(t), ISV2(t), ... , ISVn(t) is approximated by a histogram 280 of particles 270 and $P(S| ISV_1, ISV_2, ... , ISV_n)$ is defined by a partition of the space spanned by $ISV_1$, $ISV_2$, ..., $ISV_n$ n into regions as shown in FIG. 9, the probability $P(S_i(t))$ may be calculated by calculating the fraction of particles 270 in each region.

Once patient state probabilities are estimated, the clinical trajectory interpreter module 123 may assign different hazard levels 802 for each patient state or organize the states into different etiologies 803. The clinical trajectory interpreter module 123, in conjunction with the physiology observer module 122, may perform measurements utility determination 804 to determine the utility of different invasive measurements such as invasive blood pressures or invasive oxygen saturation monitoring. In one embodiment, the Clinical trajectory interpreter Module 123 determines the probabilities that the patient is in a particular state, rather than the exact state that the patient is in.

FIG. 9 illustrates a non-limiting example of a definition of a patient state that may be employed by the clinical trajectory interpreter module 123. Specifically, it assumes that the function $P(S| ISV_1, ISV_2, ... , ISV_n)$ may be defined by partitioning the domain spanned by the internal state variables $ISV_1, ISV_2, ... , ISV_n$. The particular example assumes that the patient physiology is described by two internal state variables: Pulmonary Vascular Resistance (PVR) and Cardiac Output (CO). The particular risks and respective etiologies that may be captured by these two ISVs emanate from the effects of increased pulmonary vascular resistance on the circulation. Specifically, high PVR may cause right-heart failure and consequently reduced cardiac output. Therefore, PVR can be used to define the attributes of Normal PVR and High PVR, and CO to define the attributes of Normal CO and Low CO, by assigning thresholds with the two variables. By combining these attributed, four separate states can be defined: State 1: Low CO, Normal PVR; State 2: Low CO, High PVR; State 3: Normal CO, High PVR; State 4: Normal CO Normal PVR.

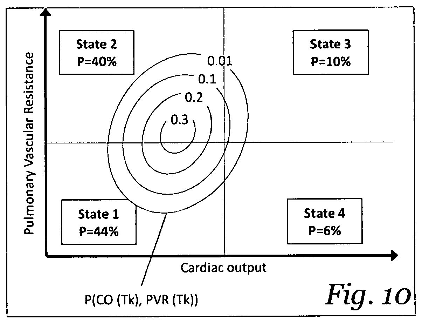

FIG. 10 illustrates a non-limiting example of how the clinical trajectory interpreter module 123 may employ the definition of patient states to assign probabilities that the patient may be classified under each of the four possible patient states at a particular point of time. In the example, the clinical trajectory interpreter module 123 takes the joint probability density function of P(Cardiac Output (Tk), Pulmonary Vascular Resistance (Tk)) and integrates it over the regions corresponding to each particular state, which produces P(S1(Tk)), P(S2(Tk)), P(S3(Tk)), and P(S4(Tk)). In this way, the clinical trajectory interpreter module 123 assigns a probability that a particular patient state is ongoing, given the information provided by the physiology observer module 122. Note that if the output of the physiology observer module 122 is not a closed form function 260 but a histogram 280 of particles 270, the clinical interpreter will not perform integration but just calculate the relative fraction of particles 270 within each region.

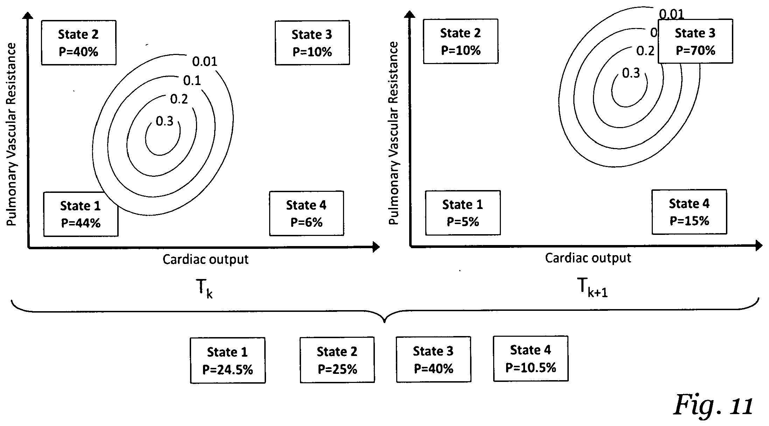

FIG. 11 illustrates an alternative approach of estimating the probabilities for different patient states. In this alternative approach, to calculate the probabilities P(S1), P(S2), P(S3) and P(S4), the clinical trajectory interpreter module 123 employs the joint probability functions of the ISVs for two consecutive time windows $T_k$ and $T_{k+1}$ to calculate a moving window average. Note in the example that the size of the window is doubled for two time instances, which indicates that the window may be of an arbitrary, suitable size. As a result of this moving window averaging, the clinical trajectory interpreter module 123 performs a dynamic analysis of the trajectory of the ISVs. That is, it gives a metric of the probability that the physiology trajectory, as described by the ISVs, may be found in a particular region in a particular time frame. In other words, this probability calculation gives an estimate of the probability that a particular patient state may be ongoing in the chosen time-frame, as opposed to just at a chosen time instance.

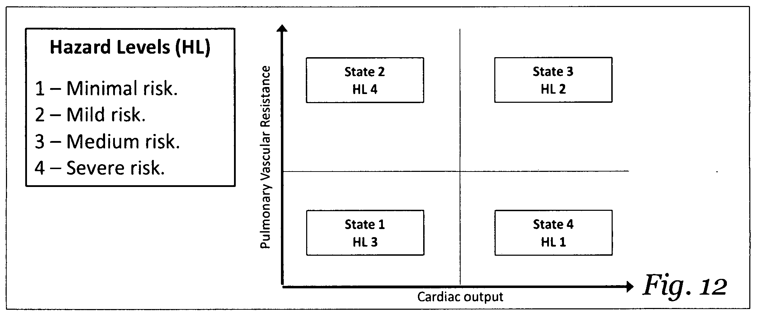

Clinical trajectory interpreter module 123 may also assign hazard levels to each particular state. FIG. 12 illustrates a non-limiting example of a definition of patient states assigned with hazard levels by the clinical trajectory interpreter module 123. The hazard levels may be informed from clinician surveys, reference literature or any other clinical sources. In the particular example, the clinical trajectory interpreter module 123 distinguishes between four different hazard levels: 1--Minimal risk, 2--Mild risk, 3--Medium risk, and 4--Severe risk. The combination of the probability of a patient state and its hazard level will be referred from hereon as a "Patient risk."

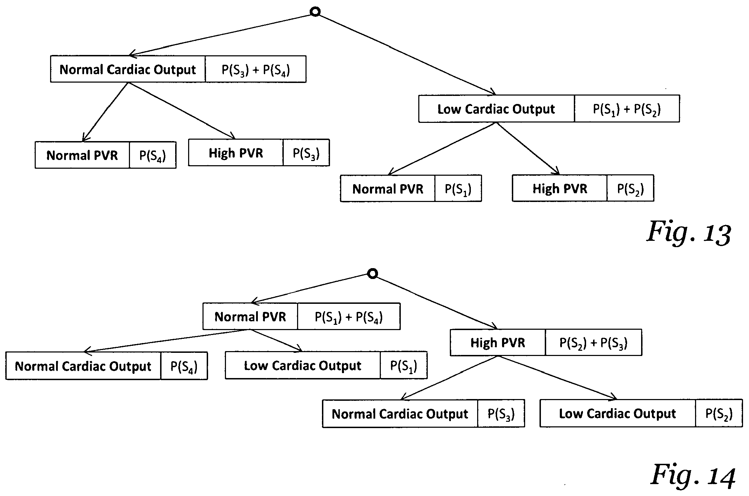

FIG. 13 illustrates how the patient states and their respective probabilities may be organized into tree graphs called etiologies. In particular, the attributes normal and low associated with the cardiac output ISV are the base nodes of the graph. Each of these vertexes has two children associated with the attributes of the pulmonary vascular resistance. This organization leads to each patient state being a leaf (end vertex) on the tree. This particular tree will be referred to as an etiology tree. The etiology tree may be further employed by the visualization and user interaction module 124 to provide a layered view of the various patient risks as further described herein.

FIG. 14 illustrates that the etiology tree may not be unique for a given set of patient states and physiologic variables. Specifically, FIG. 14 provides an alternative etiology tree for the example from FIG. 13. The root of the alternative etiology tree starts from the attributes associated with the pulmonary vascular resistance, instead of the attributes associated with cardiac output. It can be appreciated that different rules may be employed for generating the trees depending on various factors and the context of use. For example, one etiology tree may be preferred against another realization in different clinical situations or depending on the preference of the users. Moreover, the tree may dynamically change as the risks change and the clinical situation evolves.

Utility of Different Measurements

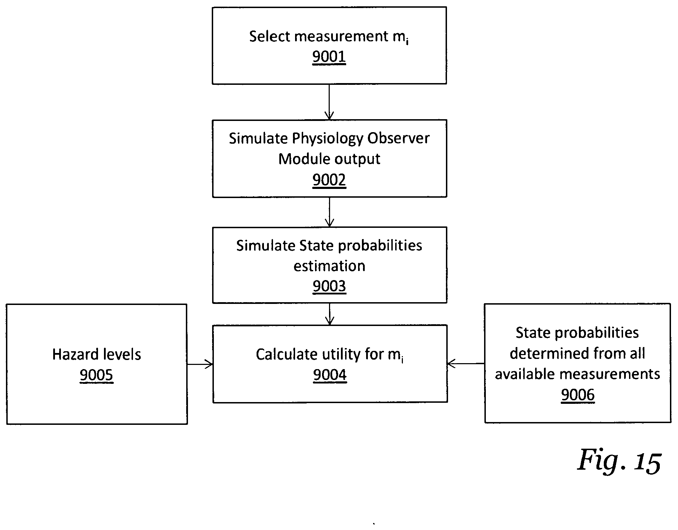

During hospital care, there exist measurements that may harm the patient or slow down their recovery. Examples of such harmful measurements are all measurements coming from catheters such as invasive blood pressures and blood oximetry, which have been shown to significantly increase the risk of infection. Therefore, it may be useful if, during the care process, the clinician is provided with an assessment of the utility of each of the potentially harmful measurements. FIG. 15 illustrates a method for calculating the utility of different measurements.

Referring to FIG. 15, the risk-based system 100 and the clinical trajectory interpreter module 123 may calculate the utility of a particular measurement with the illustrated procedure. Particularly, in step 9001, a measurement $m_i$ may be selected. Given measurement $m_i$ and a current time ($t_{current}$), in step 9002, the clinical trajectory interpreter module 123 may submit an instruction to the physiology observer module 122 to simulate the physiology observer module output (the probability density functions of the internal state variables) from a given arbitrary point back from the current time ($t_{current}-T$) to the current time $t_{current}$ with removal of measurement $m_i$ from the algorithm output. Then, in step 9003, the clinical trajectory interpreter module 123 may simulate the state probabilities estimation given the simulated output of the physiology observer and arrive with a set of patient state probabilities, i.e., $P_{sim}(S_1(t_{current}))$, $P_{sim}(S_2(t_{current}))$,..., $P_{sim}(S_n(t_{current}))$. Then, in step 9004, using the state probabilities determined from all available measurements, i.e., $P(S_1(t_{current}))$, $P(S_2(t_{current}))$,..., $P(S_n(t_{current}))$, the clinical trajectory interpreter module 123 may calculate the utility of the measurement $m_i$ using the formula:

$$U(m_i) = D(P_{sim}|P) = \sum_{i=1}^{n} P(S_i(t_{current}))\log\left(\frac{P(S_i(t_{current}))}{P_{sim}(S_i(t_{current}))}\right),$$

which is also the Kullback-Leibler divergence between the patient state distribution given all available measurements and the patient state distribution given the measurement $m_i$ has been removed for a time interval T.

Alternatively, in step 9005, the clinical trajectory interpreter module 123 may calculate utility for $m_i$ by employing the hazard levels, $r_i$, assigned to each state $S_i$ by the formula:

$$U(m_i) = D_{weighted}(P_{sim}|P) = \sum_{i=1}^{n} r_i P(S_i(t_{current}))\log\left(\frac{P(S_i(t_{current}))}{P_{sim}(S_i(t_{current}))}\right).$$

In a similar manner, the clinical trajectory interpreter module 123 can perform the utility calculation not only for a particular measurement, but also for any group of measurements. The utility calculation can also include a component that captures the potential harm associated with a particular measurement. For example, the invasive catheter measurement described above would have a large level of harm associated with it. In this way, the calculation trades the harm associated with the measurement against the value of information it provides. An example of this modified utility calculation is given by the following formula:

$$U(m_i) = D_{weighted}(P_{sim}|P) - H(m_i),$$

where $H(m_i)$ defines a function that describes the harm of each available measure.

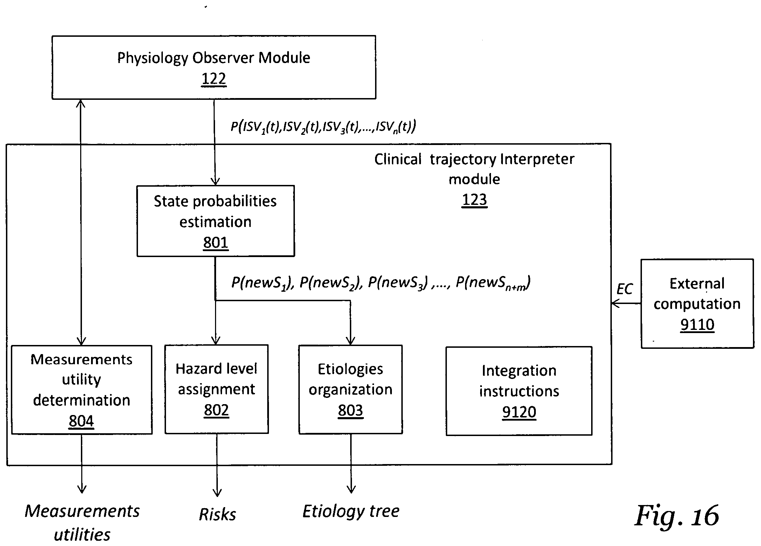

The risk-based monitoring system 100 can also integrate external computation generated from third party algorithms implemented either on the same computation medium as the patient-based monitoring system or as a part of an external device. FIG. 16 illustrates one possible realization of integration of an external computation generated from third party algorithms. Particularly, the output from the external computation 9110 is provided to the clinical trajectory interpreter module 123 which implements integration instructions 9120. As a result the state probability estimation 801 produces new states $P(newS_1)$, $P(newS_2)$, $P(newS_3)$ ,...., $P(newS_{n+m})$, which may result in an increased number of states n+m from the original number of n states. Similarly, integration instructions 9120 may be provided to the hazard level assignment 802 and the etiology organization 803.

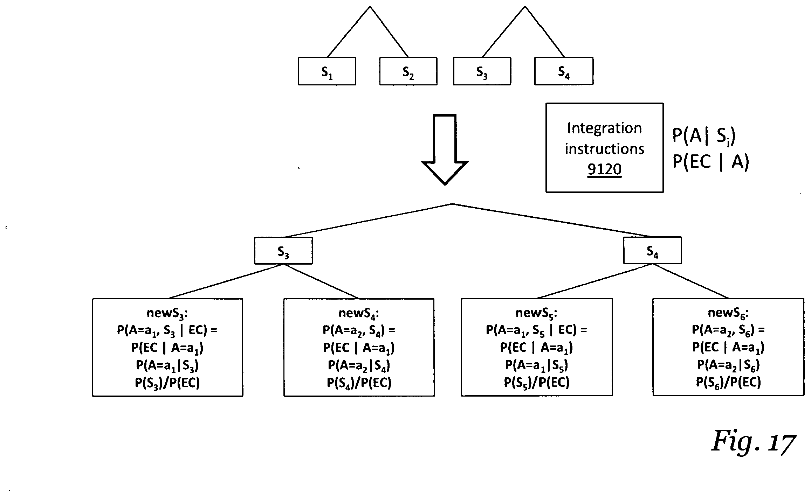

FIG. 17 illustrates an example of integration instructions. In the example, it is assumed that the external computation, EC, provides information about particular binary attributes $A=a_1$ or $A=a_2$, and the specific of how the provided information is captured in the integration instructions by the conditional probability P(EC|A). Also, given four original states $S_1$, $S_2$, $S_3$, and $S_4$, the integration instruction may specify how the states $S_3$ and $S_4$ may be updated with two additional attributes $A=a_1$ and $A=a_2$, and turn into four new states $newS_3$, $newS_4$, $newS_5$, and $newS_6$. To perform this update, the integration instruction may also employ prior probabilities $P(A|S_3)$ and $P(A|S_4)$. These prior probabilities may be derived from retrospective studies by analyzing what fractions of patients exhibiting $S_3$ or $S_4$ have concomitantly exhibited $A=a_1$ or $A=a_2$.

Another way to derive the prior probabilities is by soliciting the opinion of clinicians.

By utilizing the integration instructions, the state probabilities estimation 801 of the new states may then be derived from the formula:

$$P(A=a_j, S_i \mid EC) = P(EC \mid A=a_j)\, P(A=a_j|S_i)\, P(S_i) / P(EC),$$

where i in {3,4} and j in {1,2}, and where $P(S_j)$ are the original patient state probabilities derived from the output of the physiology observer module 122.

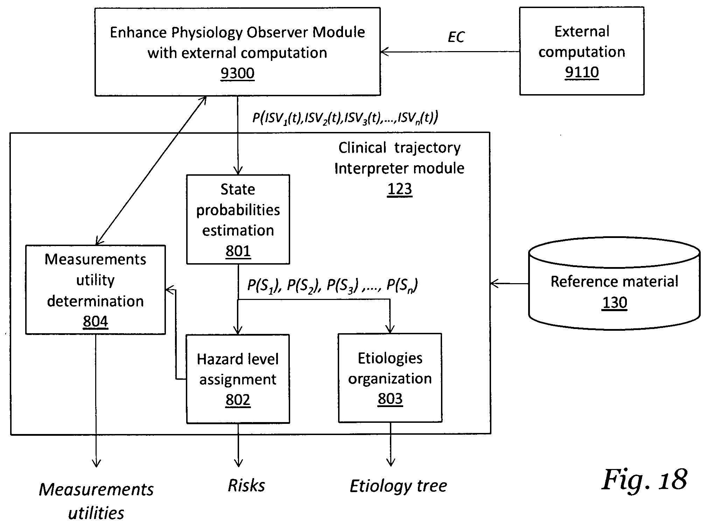

FIG. 18 illustrates an additional example of integration instructions of an external computation. Again, the risk-based monitoring system 100 can perform the integration, as shown in FIG. 18, both in the case that the external computation 9110 is generated on the same computational medium as the patient-based monitoring system, or as a part of an external device. In this case, it is assumed that the external computation 9110 provides direct information about a particular internal state variable estimated by the physiology observer module 122 (or enhanced physiology observer module 9300). Therefore, to integrate the external computation, the physiology observer module 122 can treat the external computation 9110 as an additional measurement and integrate it directly into the observation model 221.

Visualization and User Interaction|[DB2]

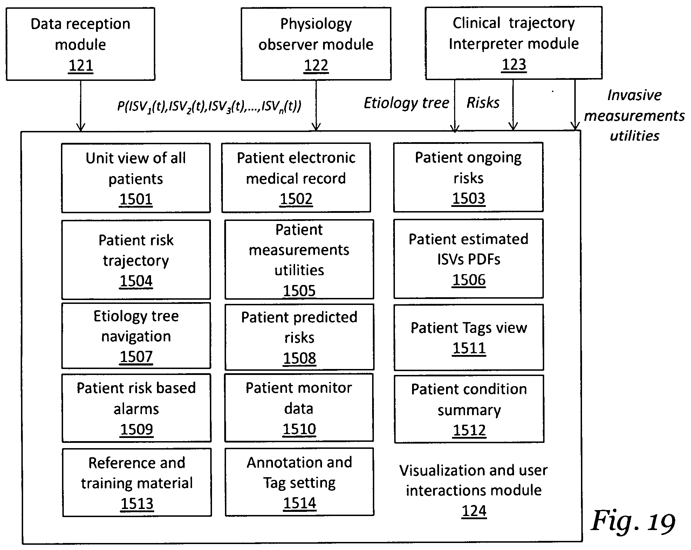

FIG. 19 illustrates example functionalities of the visualization and user interactions module 124. Specifically, module 124 may receive all available patient information and data including, the data from the data reception module 121, the joint probability density function produced by the physiology observer module 122, and the etiology tree, the risks, and the invasive measurements utilities estimated by the clinical trajectory interpreter module 123. By utilizing this information, the visualization and user interactions module 124 may produce: 1) a unit view 1501 of patients describing their risks, diagnoses, etc.; 2) a view 1502 of a patient's electronic medical record including laboratory results, prescribed medication, diagnoses, etc.; 3) a view 1503 of a patient's ongoing risks; 4) a view 1504 of a patient's risk trajectory, i.e., how the probabilities for particular patient states have evolved in a particular time frame; 5) a view 1505 of a patient's measurements utilities in the estimation of the particular patient risks; 6) a plot of a patient estimated ISVs' PDFs 1506 describing the time evolution of the ISVs' PDFs; 7) a view 1507 enabling to navigate through the etiology tree of the patient and thus visualize different levels of the tree; 8) a view 1508 showing a patient's predicted risks; 9) a view 1509 enabling clinicians to view and set patient risk based alarms; 10) a view 1510 of a patient's physiology monitoring data and its evolution against time; and 11) any combination of the above described. In addition, the visualization and user interactions module 124 may also produce a patient tags view 1511, a patient condition summary 1512, reference and training material 1513, and annotation and tag setting 1514.

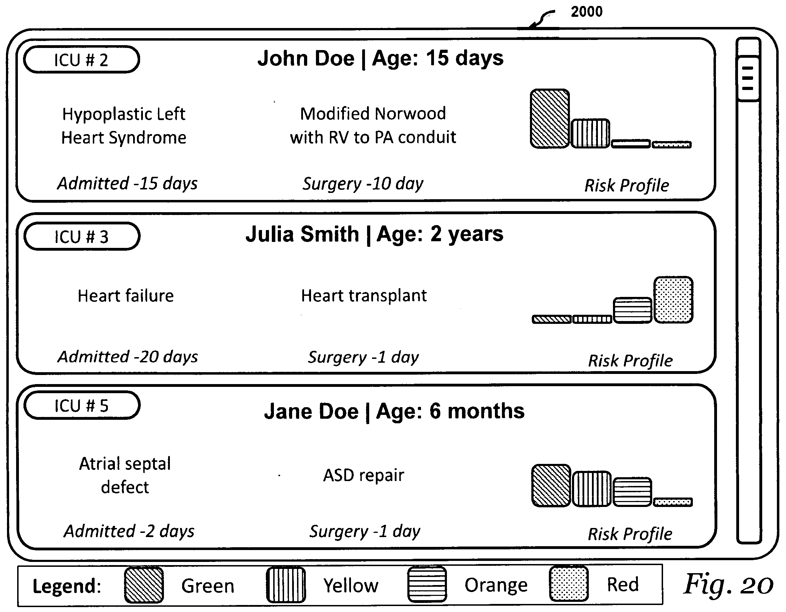

FIG. 20 illustrates an example of a summary view 2000 that may convey in a single screen a risk profile for each patient in a particular hospital unit. The risk profile represents what is the cumulative probability of the patient being in a particular hazard level. It is calculated by summing the current probabilities of all states at particular hazard level. In the example, the summed probabilities, hazard levels are represented by the height of four bars, each bar corresponding to a particular hazard level. In this specific example, these hazard levels may be Green (slanted hatching)--Minimal risk, Yellow (vertical hatching)--Mild risk, Orange (horizontal hatching)--Medium risk, and Red (dotted hatching)--Severe risk.

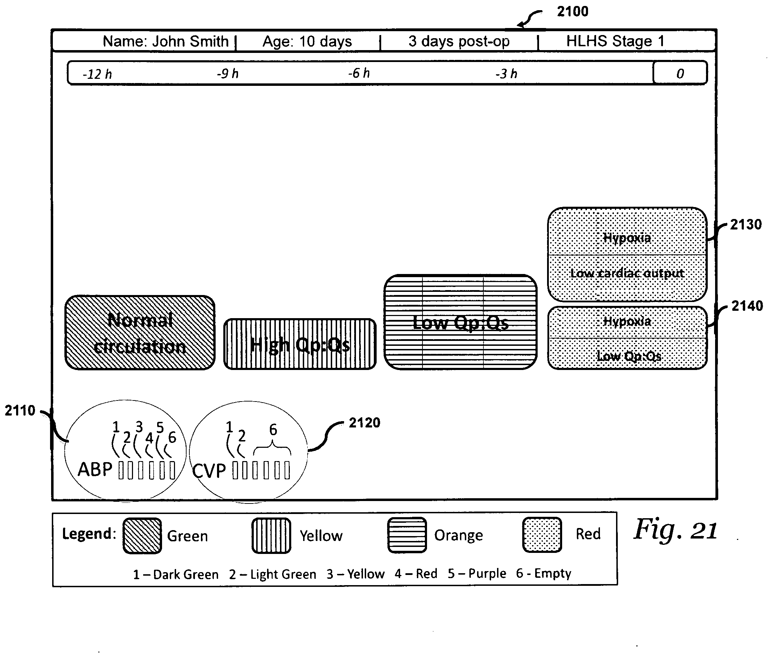

FIG. 21 illustrates one possible realization of a view 2100 describing the ongoing risks of the patient. Each round-cornered box corresponds to a particular risk: the color corresponds to the hazard level with Green (slanted hatching)-Minimal risk, Yellow (vertical hatching)--Mild risk, Orange (horizontal hatching)-Medium risk, and Red (dotted hatching)--Severe risk; the height of the box corresponds to the probability of the particular patient state. Risks are grouped in columns based on their hazard levels. The screen and the respective risks are updated in real-time as new data becomes available.

Still referring to FIG. 21, in addition to the visualization of the ongoing patient risks, the system 100 may provide information about the utility of the various invasive measurements in determining these risks. Specifically, the illustrated example gives the utilities of invasive arterial blood pressure (ABP) and invasive central venous pressure (CVP) measurements. The utility may be represented by filled bars 2110 and 2120, and the maximum utility may correspond to six filled bars. The six filled bars may be displayed in color gradient from 1-dark green, 2-light green, 3-yellow, 4-red, 5-purple, to 6-white or empty. In this particular embodiment, the filled bars 2110 for ABP show all six colors, while two of the filled bars 2120 for CVP respectfully show 1-dark green and 2-light green and the remaining filled bars 2110 show 6-white or empty.

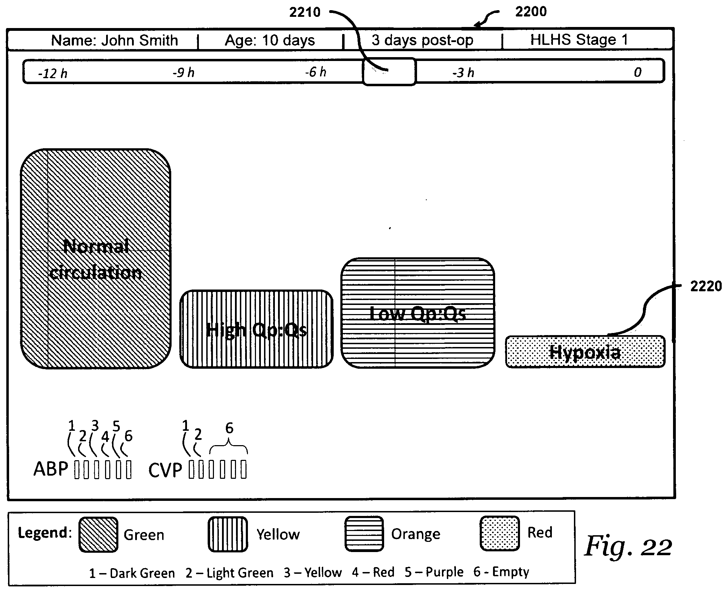

FIG. 22 illustrates a view 2200 and how a slider 2210 or other graphic element on top of the patient view may be utilized in reviewing the history of the patient risks. Specifically, in the example, the slider 2210 is moved to show the patient risks at approximately four hours back from current time. This enables clinicians to review the continuous evolution of the patient risks and compare them with the applied treatment or any other external factors. In various embodiments, slider 2210 may be moved on the user interface with a pointing device, a command, or, if utilized in conjunction with touch sensitive displays, through touching and dragging the slider or other graphic element to designate the desired time period.

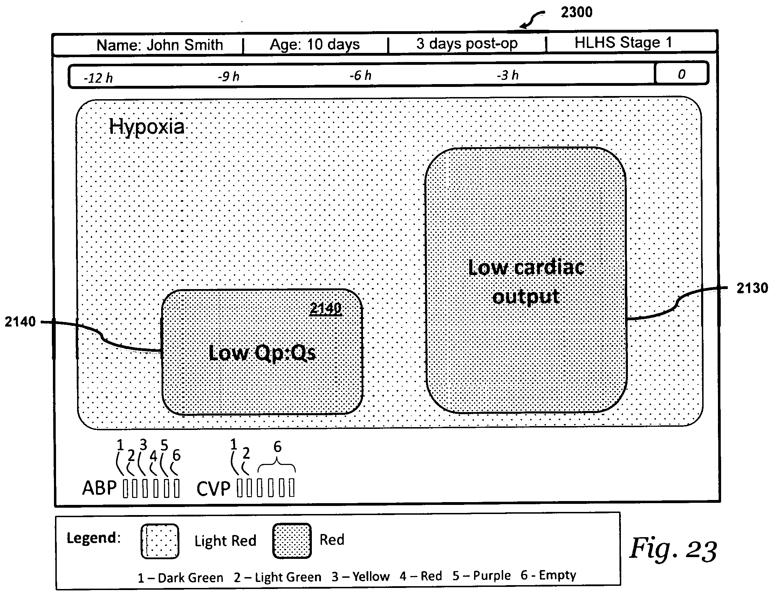

Referring now to both FIGS. 21 and 22, the etiology tree is used to combine the two states State A 2130: Hypoxia with low cardiac output and State B 2140: hypoxia with low Qp:Qs from FIG. 21 to represent them by a single patient state (Hypoxia) 2220. In the particular example, this is used to fit the text into the smaller box of FIG. 22 relative to FIG. 21. The user can navigate the etiology tree in view 2300 by clicking on the composite patient state 2220 and viewing its constituent patient states 2130 and 2140, as illustrated in FIG. 23.

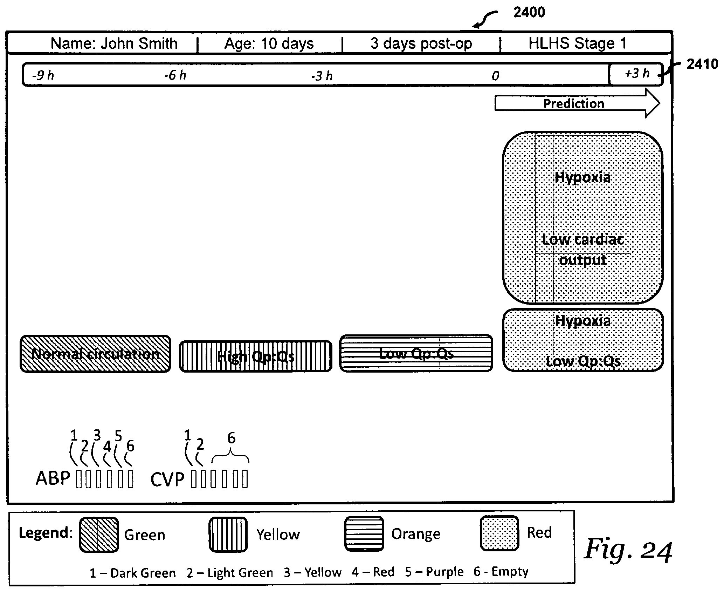

FIG. 24 illustrates in view 2400 how in the same framework the user may view the predicted risks for the patient by moving the slider 2410 ahead of current time. |[DB3]

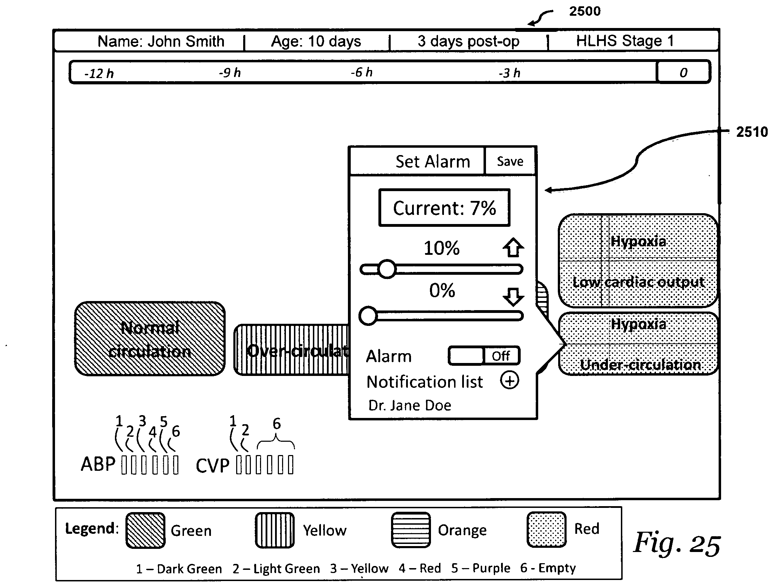

FIG. 25 illustrates in view 2500 an interactive dialog box 2510 through which the user may define the conditions to set an alarm for a particular risk. The user achieves this by selecting the particular risk and then setting upper and lower thresholds for the patient state probability associated with this risk. No alarm is activated as long as the patient state probability is between the upper and the lower threshold. The alarm is activated when the patient state probability crosses the threshold. Once the alarm is activated the system 100 may notify a list of chosen people, or send the notification to another clinical system. Any of module 122-124 may actually store their respective threshold data ranges and initiate the trigger depending on the specific parameter.

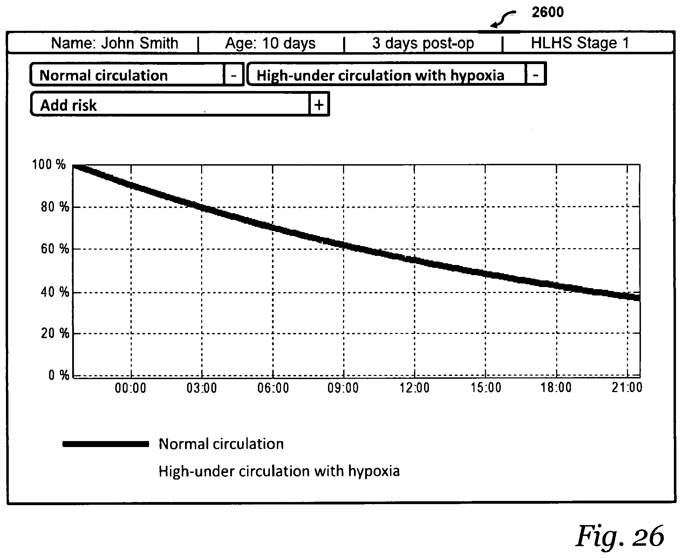

FIG. 26 illustrates in view 2600 yet another possible visualization of the patient risk trajectory, i.e. the evolution of the patient states' probabilities associated with particular risks. The user may choose what time series of patient state probabilities he/she wants to display, and the system plots these probabilities against time.

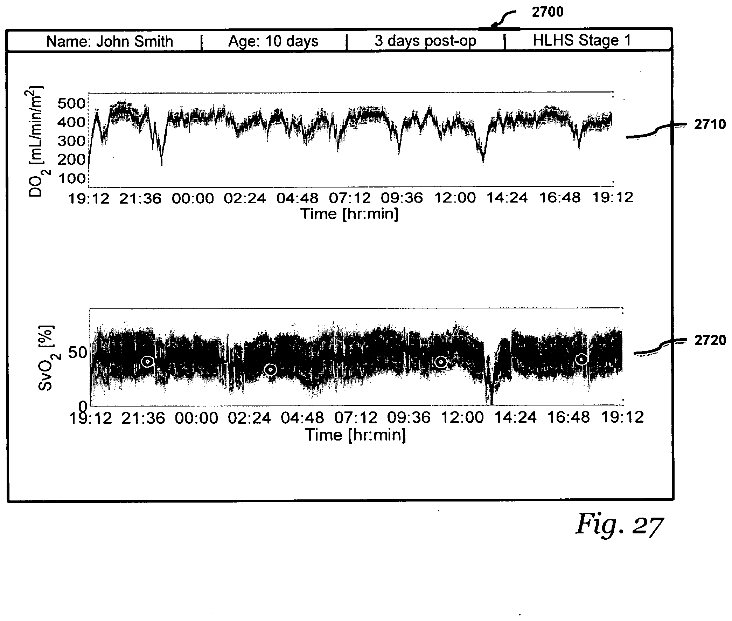

FIG. 27 illustrates in view 2700 how the system 100 may directly present the probability density functions of various internal state variables. Specifically, in the example, the estimated PDF of oxygen delivery is plotted in graph 2710 as a function of time, with darker colors corresponding to higher likelihood. Similarly, the estimated PDF of mixed venous oxygenation saturation (SvO2) is plotted in graph 2720 and compared with actual measurements (dark circles).

Information Sharing Among Users

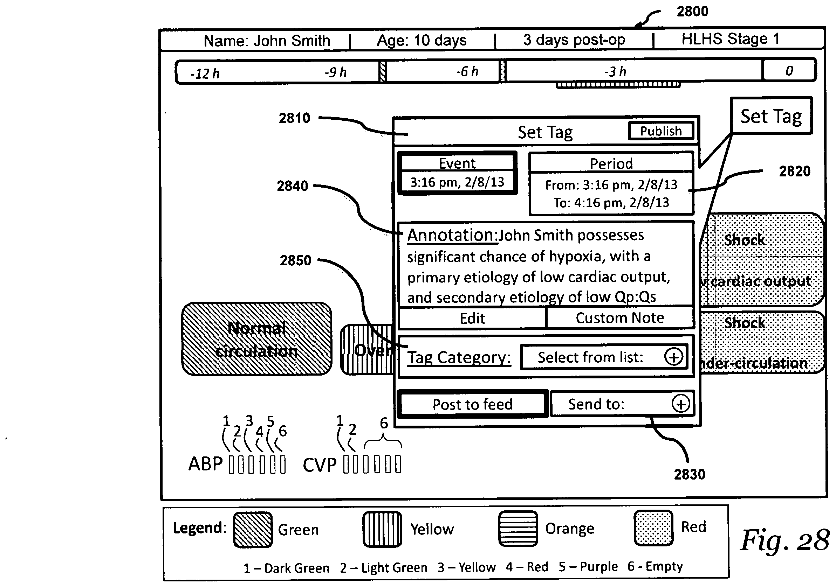

FIG. 28 in view 2800 illustrates an example of a tagging definition interface 2810 that enables clinicians to mark specific instances of time or specific periods of time 2820 that are of interest or represent important points in the clinical course, i.e. a tag. Tags may be shared or sent via dialog box 2830 to specified recipients, or may be included in notes or any other part of the user interface. Users may be able to annotate a tag with particular comments or observations via dialog box 2840, and tags may be classified into categories from menu list 2850, for example, a tag may represent a change in medication dosing, an intervention, a note regarding monitors or measuring equipment, etc. Tags and their respective time-series markings may be color coded to indicate various properties, such as their category. For example, green tag-marks on a time series may represent changes in medication, red tag-marks may represent interventions, and yellow tag marks may represent periods of heightened concern. When setting a tag, the user may be prompted to define time instance or the time period, the category of the tag, the annotation for the tag, and how the tag should be handled by the system. Furthermore, annotations may be suggested by using natural language processing to convert the etiologies of the condition into note form.

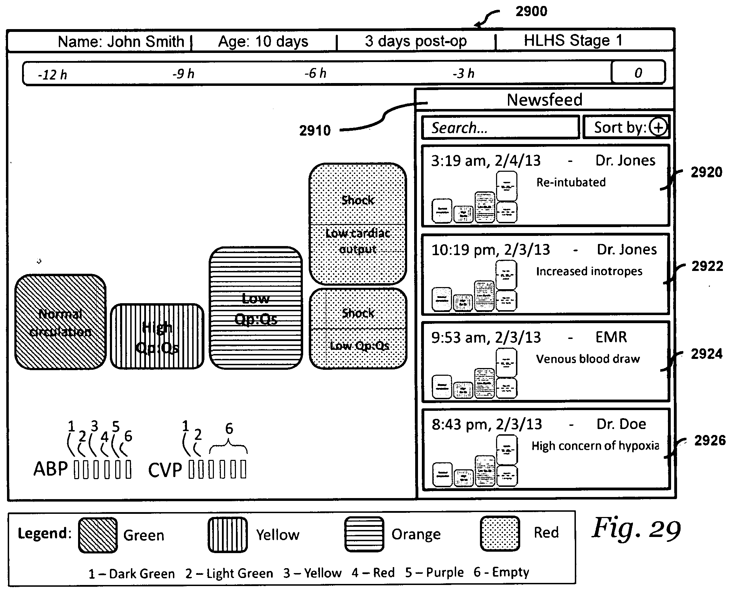

FIG. 29 illustrates in view 2900 a Newsfeed view 2910 comprising tags, notes, or information taken from external sources, such as the time of a blood draw as taken from an electronic medical record (EMR). The Newsfeed 2910 may allow clinicians to view and post events, periods of interest, interventions, notes, tags, etc., which are posted by other clinicians. Clinicians may view the entire Newsfeed, or sort it based on Tag category, hazard level, etc. Further, clinicians may search for tags based on keywords, intervention type, time of stay, source of information, etc. Entries 2920-2926 on Newsfeed 2010 may indicate any of the category, source of tag, and patient overview, either in words, or as a picture, such as the risk profile.

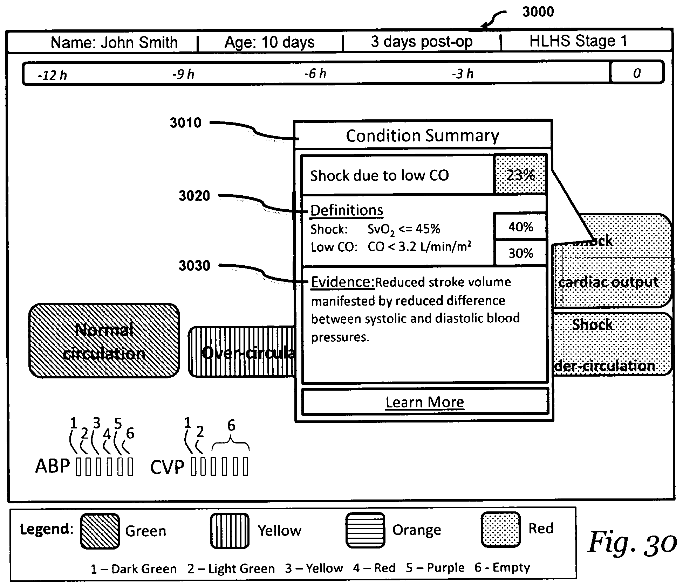

FIG. 30 illustrates in view 3000 a Condition Summary View 3010 through which the clinician may request a condition summary by selecting or clicking a particular patient state. The Condition Summary View 3010 then may present clinicians with a description of a particular state, including both definitions in window 3020 of the state, and information regarding how the system arrived at the conclusion about this patient state probability. This view 3010 may provide the likelihood and hazard level of the patient state, the definition of the patient state in terms of ISV thresholds, and the likelihood of each attribute defining the state, as illustrated and can also provide a natural language description and evidence window 3030 of evidence contributing to the patient state, by translating the ISVs PDFs into a qualitative textual description, or by directly presenting numerical information regarding the evidence. As an example, FIG. 30 illustrates the Condition Summary View 3010 for the patient state shock due to low cardiac output. Here, the Condition Summary View 3010 presents the probability of the "shock due to low cardiac output" state, and the hazard level of the state shown in color or dotted hatching. Further, the definitions of shock (mixed venous saturation below or equal to 45%), and low cardiac output (cardiac output below 3.2 liters per minute per meter squared), along with the probabilities that each of these are satisfied (e.g., 40% for shock and 30% for low CO) is presented. The evidence window 3030 conveys the information which leads to the assessment of "Shock due to low CO". In this example, the system has converted the information regarding the probabilities of the etiologies into a textual form, specifically that the estimated probability is mainly driven by the fact that there is sub nominal pulse pressure (systolic blood pressure minus diastolic blood pressure) indicating reduced stroke volume.

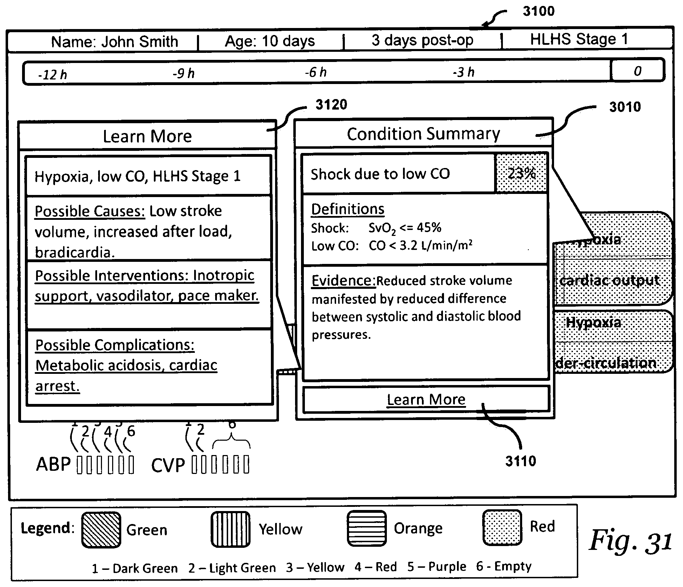

FIG. 31 further illustrates in view 3100 the ability for the user interface to include and display reference material, which may be accessed through the Internet, or stored within the system 100 or remotely accessible thereby. Selecting the Learn More button 3110 in the Condition Summary View 3010 may bring up a Learn More View 3120, which shows reference information associated with the Condition Summary View 3010. Reference information may include causes, interventions, common comorbidities, anatomy, relevant publications, etc. Furthermore, this feature may serve as a training tool to familiarize clinicians with managing the particular patient population, or treatment strategies.

HLHS Stage 1 Example

The following description explains how the disclosed system 100 and techniques can be applied to the modeling of the clinical course of a specific patient population under intensive care--post-operatively recovering Hypoplastic Left Heart Syndrome patients after stage one palliation.

Hypoplastic Left Heart Syndrome is a congenital heart defect, which is manifested by an underdeveloped left ventricle and left atrium. As a result, patients suffering from this condition do not have separated systemic and pulmonary blood flows, but instead the right ventricle is responsible for pumping blood to both the body and the lungs. Therefore, the hemodynamic optimization during intensive care involves managing the fractions of the blood flow that pass through the lungs (pulmonary flow $Q_p$) and the body (systemic flow Qs). The optimal hemodynamic state is reached when, adequate tissue oxygen delivery, $DO_2$, is achieved for a pulmonary to systemic blood flow ratio, denoted $Q_p/Q_s$, of 1. Often, to reach this optimal state, the patient physiology passes through other less beneficial states, and the correct identification of these states and the application of a proper treatment strategy for each one of them define the quality of the post-operative care.

**Table 1**

| Variable | Description | Units | Type |
|---|---|---|---|
| $DO_2$ | Indexed Oxygen Delivery | mL $O_2$/min/m$^2$ | Dynamic |
| $VO_2$ | Indexed Oxygen Consumption | mL $O_2$/min/m$^2$ | Dynamic |
| PVR | Pulmonary Vascular Resistance | mm Hg/L/min/m$^2$ | Dynamic |
| SVR | System Vascular Resistance | mm Hg/L/min/m$^2$ | Dynamic |
| $\Delta$PVR | Change in PVR per time step | mm Hg/L/min/m$^2$ | Dynamic |
| $\Delta$SVR | Change in SVR per time step | mm Hg/L/min/m$^2$ | Dynamic |

(continued)

| Variable | Description | Units | Type |
|---|---|---|---|
| $Hb$ | Hemoglobin | g/dL | Dynamic/Observed |
| $HR$ | Heart Rate | Beats per min | Dynamic/Observed |
| $SpVO_2$ | Pulmonary Venous Oxygen Saturation | % | Dynamic |
| $SaO_2$ | Arterial Oxygen Saturation | % | Derived/Observed |
| $SvO_2$ | Systemic Venous Oxygen Saturation | % | Derived/Observed |
| $SpO_2$ | Pulmonary Venous Oxygen Saturation | % | Observed |
| $\eta$ | Aortic Compliance | | Dynamic |
| $ABPm$ | Mean Arterial Blood Pressure | mm Hg | Derived/Observed |
| $CVP$ | Central Venous Pressure | mm Hg | Dynamic/Observed |
| $LAP$ | Left Atrial Pressure | mm Hg | Dynamic/Observed |
| $RAP$ | Right Atrial Pressure | mm Hg | Dynamic/Observed |
| $\Delta P$ | Pulse Pressure | mm Hg | Derived/Observed |
| $CO$ | Total Cardiac Output | L/min/m$^2$ | Derived |
| $Q_p$ | Pulmonary rate of blood flow | L/min/m$^2$ | Derived |
| $Q_s$ | Systemic rate of blood flow | L/min/m$^2$ | Derived |
| $Q_p{:}Q_s$ | Ratio of $Q_p$ to $Q_s$ | | Derived |
| $\Delta Q_p{:}Q_s$ | Change in Ratio of Qp to Qs per time step | | Derived |
| $c_{DO2}$ | Oxygen Delivery feedback constant | | - |
| $c_{VO2}$ | Oxygen Consumption feedback constant | | - |
| $c_{Hb}$ | Hemoglobin feedback constant | | - |
| $c_3$ | Aortic compliance scaling constant | | - |
| $c_4$ | Aortic compliance offset | | - |
| $c_5$ | Hemoglobin oxygen carrying capacity | | - |

Table 1 lists state variables that may be used in the model of HLHS physiology after stage 1 palliation, the variable description, units, and type of variable. A person reasonably skilled in the relevant arts will recognize that though these variables encompass circulation, hemodynamic, and the oxygen exchange components of HLHS physiology, the models can be altered or enhanced with any additional physiologic components such as ventilation, metabolism, etc. without altering the premise of the disclosed invention.

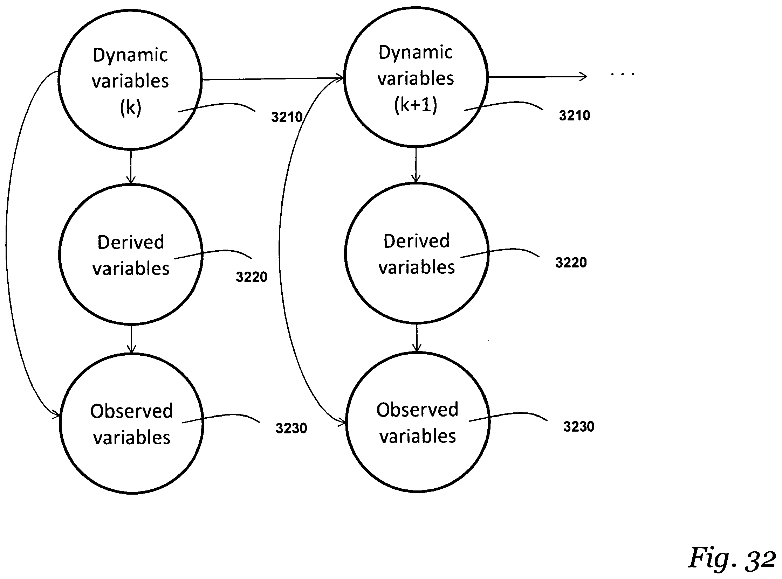

FIG. 32 depicts a general Dynamic Bayesian Network (DBN) that may be employed to capture the physiology model of the HLHS stage 1 palliation patients. The graphical model illustrated conceptually in FIG. 32 captures the causal and probabilistic relationship between the variables of the model. In the DBN, the state variables are organized into three groups: dynamic variables 3210, derived variables 3220, and observed variables 3230. Dynamic variables 3210 are variables whose values change over time based on a dynamic probabilistic model to be described below. Derived variables 3230 are quantities that depend on the dynamic variables with some functional relationship. These variables are computed or derived when required from the latest dynamic variables and are therefore also dynamic in nature. Observed variables 3230 are those variables that are measured directly by one of the sensors connected to the system and the patient. Observed variables 3230 represent instances of the true dynamic or derived states variables that have been observed under noise.

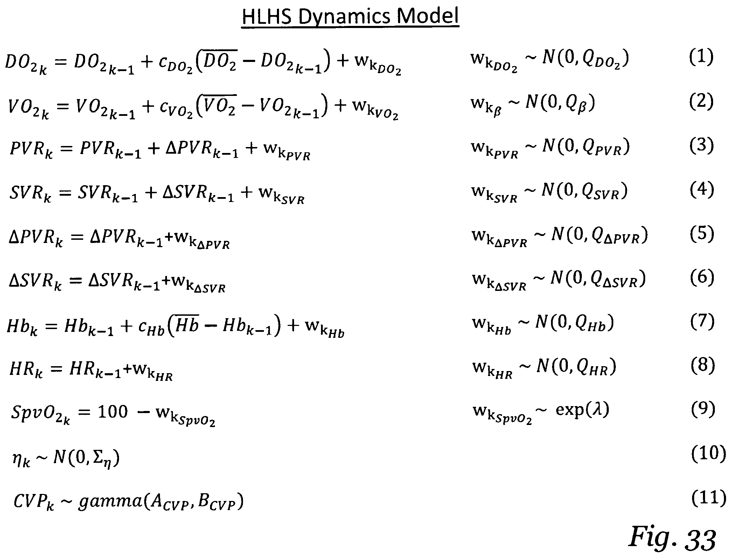

FIG. 33 lists several equations that may be used to model the dynamics of the HLHS stage 1 physiology. The model consists of four main types of stochastic models. The first type of model is a stochastic feedback control model (eqs. 1, 2, and 7). These variables have a nominal value that the body maintains, but are disturbed by some random process off of this nominal value. The strength at which the body attempts to maintain these values is decided by the feedback constant. The second type of model is a drift diffusion process (eqs, 3 and 4). These variables are driven over time by a random white noise process and a drift rate process. The third type of model is a simple random walk process (eqs. 5, 6, and 8). The last type of dynamic model is a memory-less process model in which the variable has no relationship to the variable at the previous time period, but is simply a random variable which value changes at each time instance according to some predefined distribution over the proper support of the variable, i.e. a gamma distribution over the entire positive real line with parameters A and B. (eqs. 9, 10, and 11). With the exception of equations 9, 10, and 11, the driving noise for each dynamic model is independent white Gaussian noise.

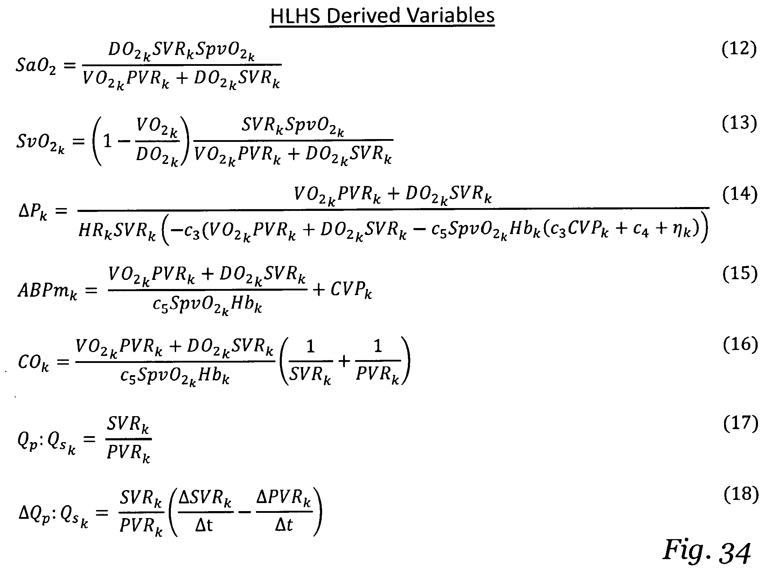

FIG. 34 depicts example equations that may be used to abstract the relationships between the dynamic variables in the model and the derived variables. Some of these functional relationships are true for general human physiology, but many are the result of the parallel circulation physiology that is specific to the HLHS population. Equations 12-15 describe relationships for variables that are measured directly. Equations 16-18 describe functional relationships for variables that are of highest interest when managing care of HLHS patients post-surgery, specifically Cardiac Output (CO) and Pulmonary to Systemic Flow ratio (Qp:Qs). These variables cannot be measured directly without complex procedures.

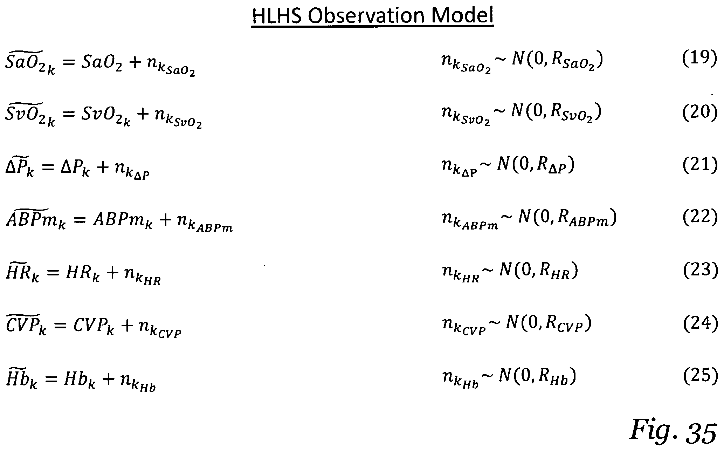

Given these functional relationships and the definition of the dynamic states, FIG. 35 now provides a possible observation model that may be used to relate the derived variables with the available sensor data. Each observation model is a conditional Gaussian relationship. Under this model, the measurement received from the sensor represents a direct observation of the underlying state variable corrupted by additional independent Gaussian White noise with some variance. The figure depicts the observed quantity as the underlying state variable with a tilde over the variable name. In this implementation, different sensors can map to the same underlying state variable, but with potentially different noise levels. For example, SpO2 as reported by a pulse oximeter measures the underlying physiology state, SaO2 or arterial oxygen saturation non-invasively. An intravenous catheter inserted directly into the arterial blood stream also measures this quantity but in an invasive way. The catheter measurement should be a more accurate measurement than the pulse oximetry. In this model, this is handled with a smaller measurement variance, R.

In the HLHS physiology observer, inference over the DBN is performed using a particle filter. As described earlier, a particle filter is an example of an approximate inference scheme that uses Monte Carlo samples of the internal state variables to approximate the probability density function of each state variables with an empirical distribution based on the number of particles. The filter uses a process known as Sequential Importance Sampling (SIS) to continuously resample particles from the most recent approximate probability distribution. In the filter, each particle is assigned a weight. When a new observation or measurement arrives, the weights of each particle are updated based on the likelihood of the particular particle given the observation. The particles are then resampled based on their relative updated weights, the particles with the highest weights being more likely to be resampled than those with lower weights.

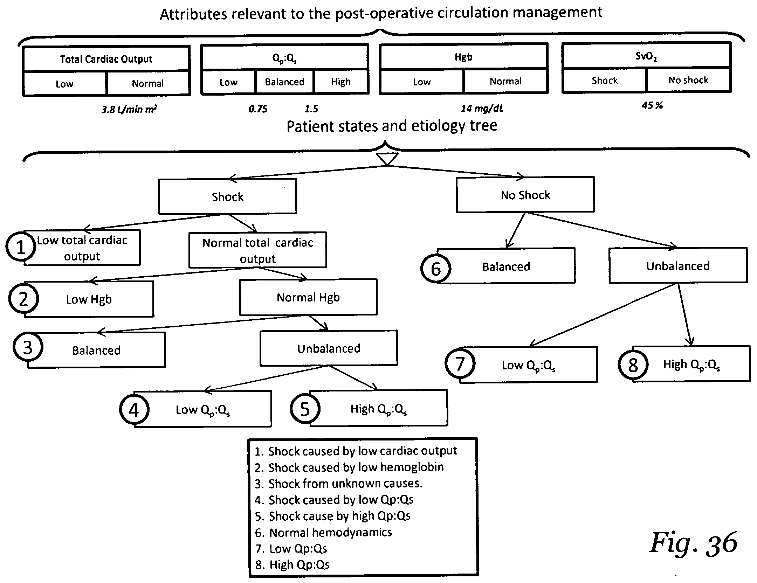

FIG. 36 illustrates possible attributes, patient states, and an etiology tree that may be used by the clinical trajectory interpreter module 123 in the case of the HLHS Stage 1 population. The variable total cardiac output defined as the sum of the systemic and the pulmonary blood flows is used to define low and normal total cardiac output, the Qp:Qs ratio is used to derive low, balanced, and high Qp:Qs ratio, the value of hemoglobin concentration Hgb is used to derive low and normal hemoglobin, and the value of the mixed venous oxygen saturation, SvO2, is used to derive the attributes hemodynamic shock and no hemodynamic shock. This results in eight possible states defined as follows: 1) Shock caused by low total cardiac output, as the presence of both of the attributes Shock and low total cardiac output; 2) Shock caused by low hemoglobin as the state with attributes shock, normal total cardiac output, and low hemoglobin; 3) shock from unknown causes as the state with the attributes shock, normal total cardiac output, normal hemoglobin, and balanced circulation; 4) shock cause by low Qp:Qs as the state with the attributes shock, normal total cardiac output, normal hemoglobin, and low Qp:Qs; 5) shock cause by high Qp:Qs as the state with the attributes shock, normal total cardiac output, normal hemoglobin, and high Qp:Qs; 6) normal circulation as a state with the attributes of no shock and normal circulation; 7) low Qp:Qs as the state defined by the attributes of no shock and low Qp:Qs; and 8) high Qp:Qs as the state defined by the attributes of no shock and high Qp:Qs. FIG. 35 also illustrates a possible realization of an etiology tree describing the relationships between the attributes and the patient states. Using the particles approximation of internal state variables the probability of the eight states can be calculated by calculating the relative fraction of particles within each state.

Example of Applying The Risk-Based Monitoring System In Conjunction With Evaluating Consequences Of A Possible Treatment

Another possible application of the risk based monitoring system is to assist clinicians when deciding whether to apply a particular treatment, one example being blood transfusion. Transfusion of blood and blood products is a common in-hospital procedure. Despite that blood transfusion indications and policies are neither well established nor consistently applied within or between medical centers. Multiple studies have demonstrated variation in transfusion practices among different hospitals, practitioners, and procedures. This variation persists even when applied to a single procedure (e.g. coronary artery bypass graft surgery).

Moreover, blood transfusion has been increasingly recognized as an independent risk factor for morbidity and mortality. Specific events and outcomes associated with transfusion include sepsis, organ ischemia, increased time on ventilation support, increased hospital length of stay, and short- and long-term morbidity. This relationship is proportional to the transfusion volume, and evidence suggests that high hematocrit values may be detrimental. Understandably, researchers conventionally recommend transfusion policies aimed at achieving an informed tradeoff between the risks and benefits.

Setting robust and effective transfusion policies has been proven to be a difficult task. The consensus in the medical community is that simple policies--such as hemoglobin threshold policies--do not provide adequate guidance. This is due to the compensatory nature of hemodynamic physiology; patients have a variable capacity to tolerate low hemoglobin. Consequently, effective transfusion decision-making must integrate factors such as compensatory reserve, intravascular volume, hemodynamic stability, procedure type, and other patient data. Thus, there is an essential need for blood management policies that will utilize the full spectrum of relevant clinical variables and determine the risk/benefit ratio of transfusion. This is exactly afforded by applying the risk based monitoring system.

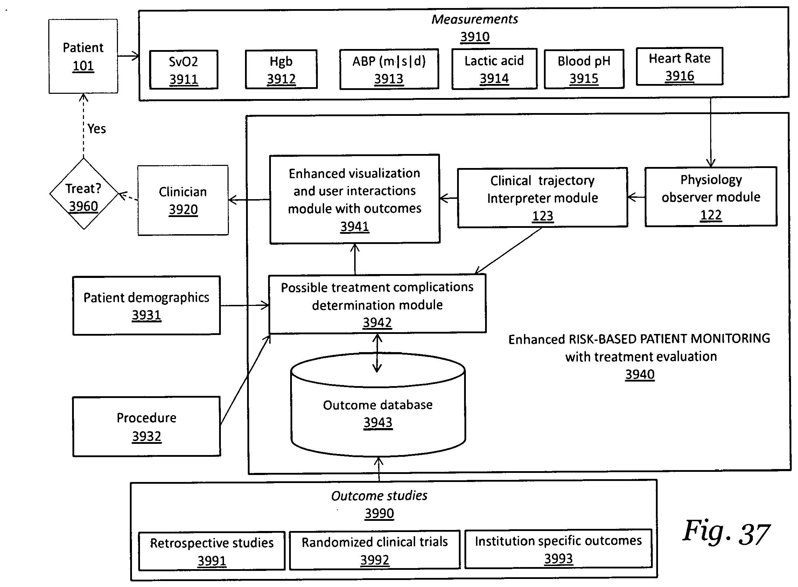

FIG. 37 illustrates one possible environment in which the risk based monitoring system can be applied to assist clinicians in deciding whether to apply a particular treatment. Consistent with the disclosure, a patient 101 is being monitored with multiple measurements 3910, both intermittently and persistently. The persistent measurements may include mixed venous oxygen saturation (SvO2) 3911, systolic, diastolic and mean arterial blood pressures (ABP s|d|m) 3913, heart rate 3916, monitored via a bedside monitor. The intermittent measurements may include blood pH 3915, hemoglobin concentration (Hgb) 3912, and lactic acid concentration 3914 monitored through periodic blood works. These measurements 3910 are fed into an enhanced risk based monitoring system 3940 with treatment evaluation, which, in addition to the previously disclosed physiology observer module 122 and clinical trajectory interpreter module 123, consists of several other modules. A possible treatment complications determination module 3924 receives information from the clinical trajectory interpreter module 123, together with information about the patient demographics 3931 and type of procedure 3932. With the information, this module 3924 queries an outcome database 3943 and receives back information of what the probability of different complications can be given that a) the patient is in particular patient states with particular probabilities; b) the patient is of certain demographics (age, sex, etc); c) the patient has had a particular type of procedure; d) and any combinations thereof of a) b) and c). On the other hand, the outcome database 3943 can be populated by using outcome studies 3990 derived from retrospective studies 3991, randomized clinical trials 3992, institution specific outcomes 3993 determined from previously collected patient data for a particular institution, and any combination thereof of the proceeding elements.

When the possible treatment complications determination module 3942 determines the possible complications, it feeds this information back to an enhanced visualization and user interactions module 3941. The enhanced visualization and user interactions module 3941 combines the patient-specific risk based monitoring performed by the physiology observer module 122 and the clinical trajectory interpreter module 122, with the evaluation of probable complication. This affords the system to provide a superior vantage point from which the clinician 3920 can better recognize risks and benefits of treatments such as blood transfusion, and respectively more efficiently and effectively decide whether to administer this treatment 3960 or not.

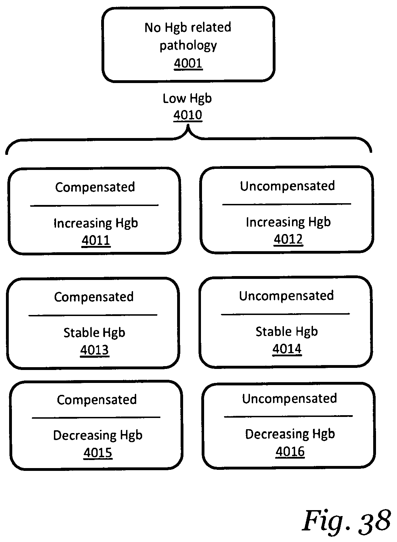

FIG. 38 shows a non-limiting example set of patient states relevant to blood transfusion that may be used to inform the blood transfusion decision. The states contain information about the dynamics of the hemoglobin (decreasing/stable/increasing) and the hemodynamic compensation for reduced blood oxygen carrying capacity. In uncompensated patients, the hemodynamic auto-regulation mechanisms become incapable of overcoming the depleted blood oxygen carrying capacity, marking the onset of anaerobic metabolism. These seven states can be determined through three internal state variables: oxygen delivery, hemoglobin, and rate of hemoglobin production/loss. Specifically, when hemoglobin is above 13 mg/dL, it is assumed that there is no Hgb related pathology 4001. When Hgb is lower than 13 mg/dL, there are six other states, determined through five different attributes. From the oxygen delivery ISV, the system can determine whether the patient is compensated or uncompensated, e.g., it may be assumed that DO2 above 400 ml/min/m.sup.2, for ventilated and paralyzed patient, indicates compensation, and below this value uncompensated patient. The other three attributes are determined from the Hgb rate ISV and are stable 4013 and 4014 (the rate is close to zero), increasing 4011 and 4012 (the rate is positive), and decreasing 4015 and 4016 (the rate is negative).

Using The Risk Based Monitoring System With Standardized Clinical Plan

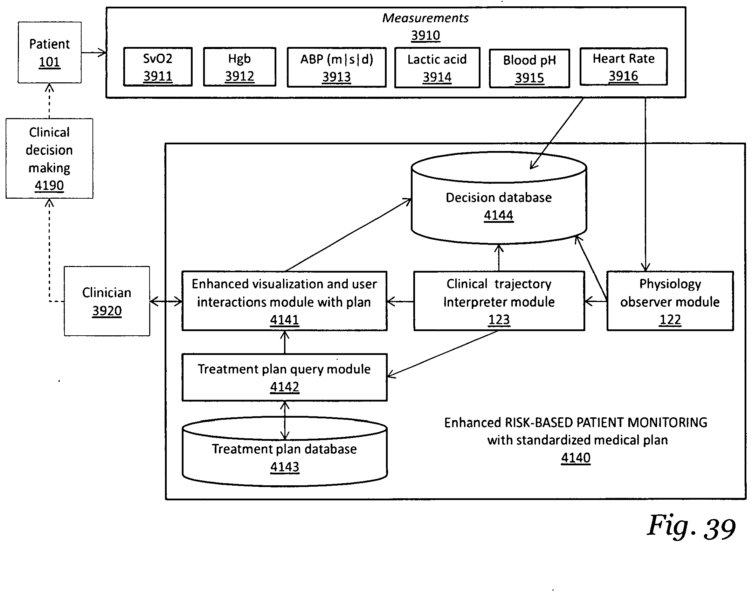

Yet another application of the risk based monitoring system is in applying standardized medical plans. FIG. 39 illustrates one possible embodiment of this application. Specifically, the data from the clinical trajectory interpreter module 123 is fed to a treatment query module 4142. The treatment query module 4142 queries a treatment plan database 4143 based on the determined patient risks. The treatment plan database 4143 specifies a map between patient risks and treatments. When the database 4143 returns a treatment plan, it is represented to the clinician 3920 by an enhanced visualization and user interactions module 4141 with plan. The clinician 3920 can then make clinical decision 4190 with respect to patient 101. The user decision, the context under which it was taken, (the calculated patient risks, the estimated ISVs, and other possible patient data at the time of the decision) are then recorded to a decision data base 4144. The decision database 4144 then can be compared to patient outcomes and utilized in the improvement of the treatment plan.

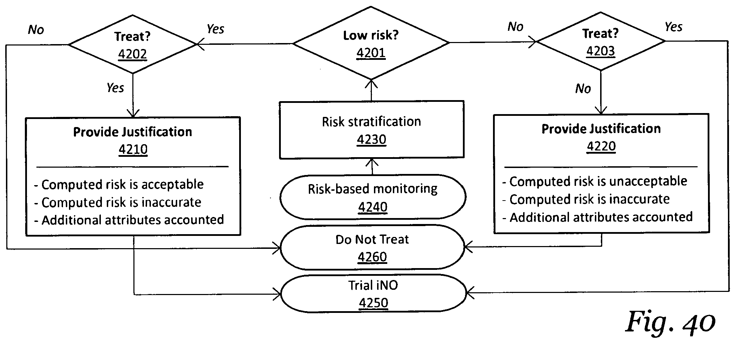

FIG. 40 illustrates an example application of the risk based monitoring system 4240 combined with a specific type of standardized clinical plan. The particular example considers the medical decision whether to treat the patient with nitric oxide. Nitric oxide is a pulmonary vasodilator and is used to treat high pulmonary vascular resistance and ensuing pulmonary hypertension, which can cause reduced cardiac output. In the example, the medical plan uses the risks calculated by the clinical trajectory interpreter module 123 and stratifies 4230 them into two categories: low risk and high risk. If the risks are low 4201 the recommended decision is not to treat 4202, respectively, if the patient is classified as being in high risk the recommended decision is to treat 4203. The provider can then make a decision to either follow the recommendations 4250 or disregard them 4260. If the provider chooses to disregard the treatment recommendation for a high risk patient, he needs to provide justification 4220. Likewise, if the provider chooses to treat a low risk patient, he also needs to provide justification 4210. Justifications 4210 and 4220 in conjunction with patient outcomes may be utilized to refine the risk stratification 4230 and risk-based monitoring system 100.

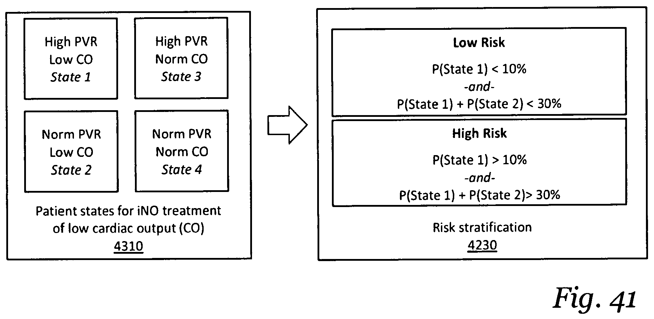

FIG. 41 illustrates the example risk stratification that may be employed by the system in the context of Nitric Oxide treatment. Specifically, it assumes that the patient can be in four different states: State 1: Low CO, Normal PVR; State 2: Low CO, High PVR; State 1 Normal CO, High PVR; State 4: Normal CO Normal PVR. A patient being in low risk may be defined as P(State 1)<10% and P(State 1)+P(State 2)<30%. Similarly high risk may be defined as: P(State 1)>10% and P(State 1)+P(State 2)>30%.

Using The Clinical Risk Assessment System In Outpatient Care Of Chronic Conditions

Yet another embodiment of the present disclosure allows the clinical trajectory tracking in outpatient care. Outpatient care of chronic conditions involves sporadic patient assessment from intermittent visits, patient self-evaluations, and observations from caregivers. This leads to uncertainties in determining the patient clinical course and the efficiency of the prescribed treatment strategy. To achieve effective patient care management, clinicians must understand and reduce these uncertainties. They have two main decisions at their disposal: 1) schedule visits, prescribe tests, or solicit self-evaluation (or caregiver evaluations) to improve their understanding of the clinical trajectory; and/or 2) prescribe changes of medication or medication dosing to achieve a better trade-off between the likelihood of improvement and possible side-effects. To inform this decision making process, there is a need for processing the available patient information in a way that conveys the clinical trajectory, the uncertainty in its estimation, and the expected effect that different treatment strategies may have on the future evolution of the clinical trajectory.

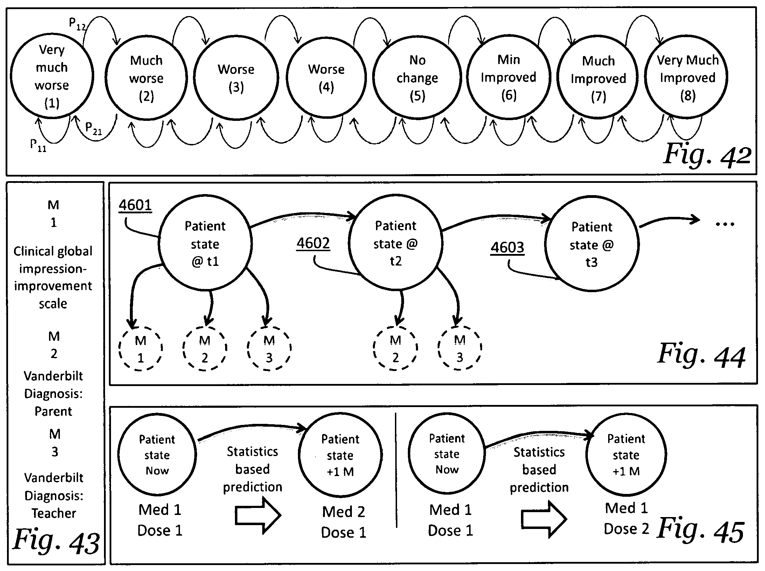

As a non-limiting example embodiment of the risk based monitoring system to outpatient clinical trajectory tracking, we consider its application to the outpatient care of Attention Deficit and Hyperactivity Disorder (ADHD) of pediatric patients. FIG. 42 illustrates possible patient states that may describe the clinical trajectory of an ADHD patient. They are the same as the ones used by the Clinical global impression-improvement scale: 1) very much worse; 2) much worse; 3) worse; 4) No change; 5) Minimally Improved; 6) Much improved; 7) Very much improved. The Patient State Distribution (PSD) is the set of probabilities that the patient is in any of the seven states, given all available information and observations.

To evaluate the patient state, a clinician may either schedule an office visit for direct examination, or may request a Vanderbilt diagnostic test from family members or teachers (the test is modified depending on the respondent, teacher or parent). FIG. 43 lists the available patient evaluation modalities as M1. M2 and M3. Models may be used to map the test questions and answers into the states of the patient. Both the clinical evaluation and the test-based evaluation are associated with uncertainty that prohibits the exact determination in which of the seven states the patient currently resides.

The dynamic model or the patient evolution from state to state may be abstracted by a Dynamic Bayesian Network (DBN) as the one shown in FIG. 44. In FIG. 44, the arcs' directions signify statistical dependence, i.e., the connection from "Patient state @ t1" 4601 to "M1", signifies the probability density function (PDF): P(M1|Patient state @ t1). Similarly, the depicted DBN illustrates that "Patient state @ t2" 4602 (the patient state at a particular time t2), is conditioned on the "Patient state @ t1" (the patient state at the previous time increment t1). In the spirit of the present disclosure, this model enables the estimation of the patient state distribution even in the absence of some or all possible measurements, e.g., as illustrated in the figure at time instance t2 when M1 is missing, and at time instance t3 ("Patient state @ t3" 4603), when all measurements are missing.

FIG. 45 illustrates an alternative embodiment for two predictions of how the patient state can transition in a single month given medication change or a dosage change. This prediction is performed based on a statistical model derived in the following fashion: Step 1: Isolate a group of patients from retrospective data which at some point of their treatment have passed through State A and received a change of treatment (Med1 Dose1->Med 2 Dose 2); Step 2: For each patient, set the time instance that this particular event occurred to t0; 3) step 3: for each patient identify what is the patient state at time t0+1 Month (1M) (or any desired time step unit). 4) calculate the fraction of patients that transition State A->State i where i stands for all seven possible patient states; 5) set the fraction as the probabilities for transition under the particular treatment change.

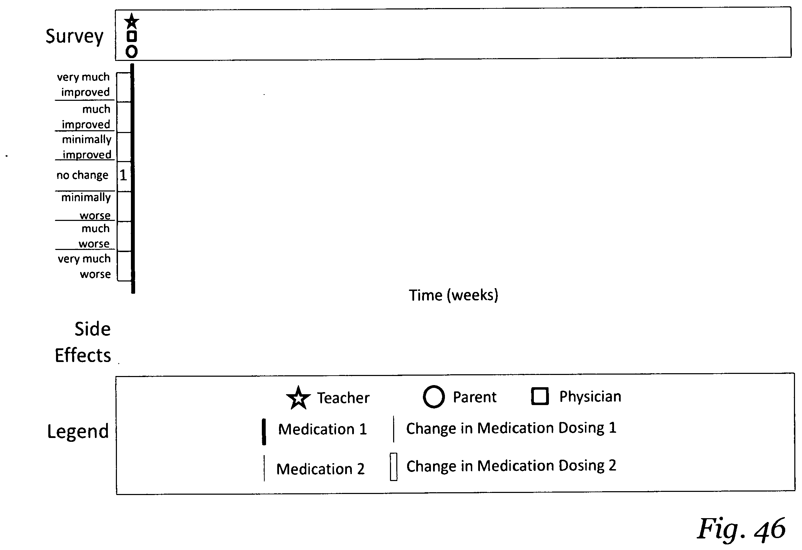

FIG. 46 shows one possible embodiment and scenario of visualization displaying the patient clinical trajectory and risks. The user interface denotes that the patient is at "no change" state, and that this has been established by three separate measurements: office visit, teacher based Vanderbilt diagnosis, and parent based Vanderbilt diagnosis. The solid line on the screen signifies that a medication has been prescribed to the patient (Medication 1) at week 1 of the treatment.

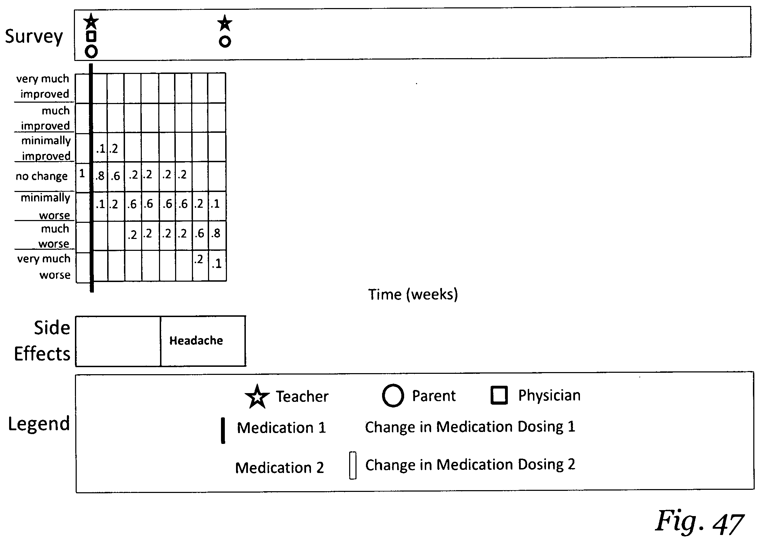

FIG. 47 shows an evaluation of the patient and the patient trajectory at week 9 at which point the clinical risk assessment system determines a probability density function for the state of the patient for each of the past six weeks. The available measurements at this point are teacher and parent Vanderbilt diagnosis. In the illustrated example, due to a high probability of a deteriorating patient state, the clinician prescribes a change of medication dosing, which is depicted by a dashed red line. Additionally, the user interface shows the side effect reported by the patient--headache.

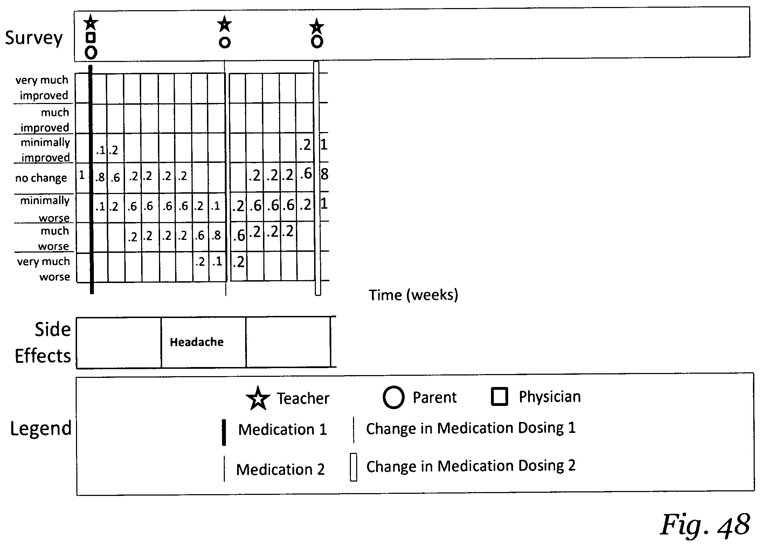

FIG. 48 shows a follow-up evaluation based on teacher and parent Vanderbilt diagnosis. In the example, the clinician decides a medication change depicted by a hollow line.

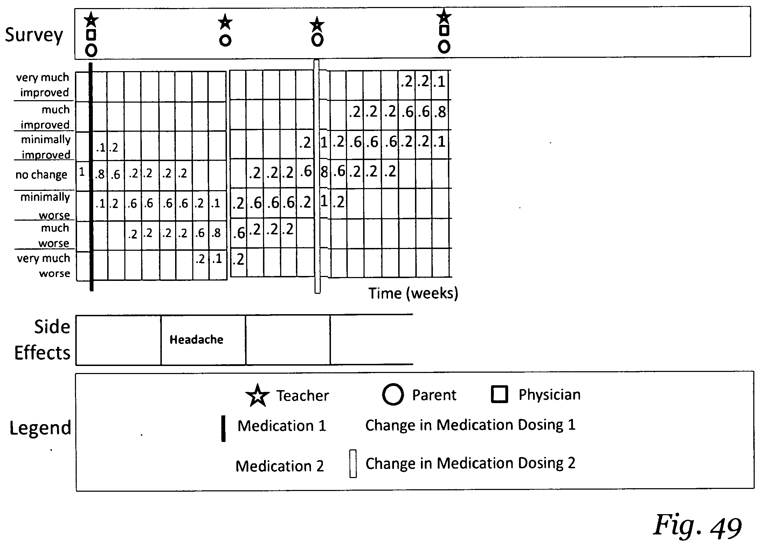

FIG. 49 shows consequent evaluation based on all available measurements--office visit, parent and teacher evaluation, which establishes high probability for significant improvement.

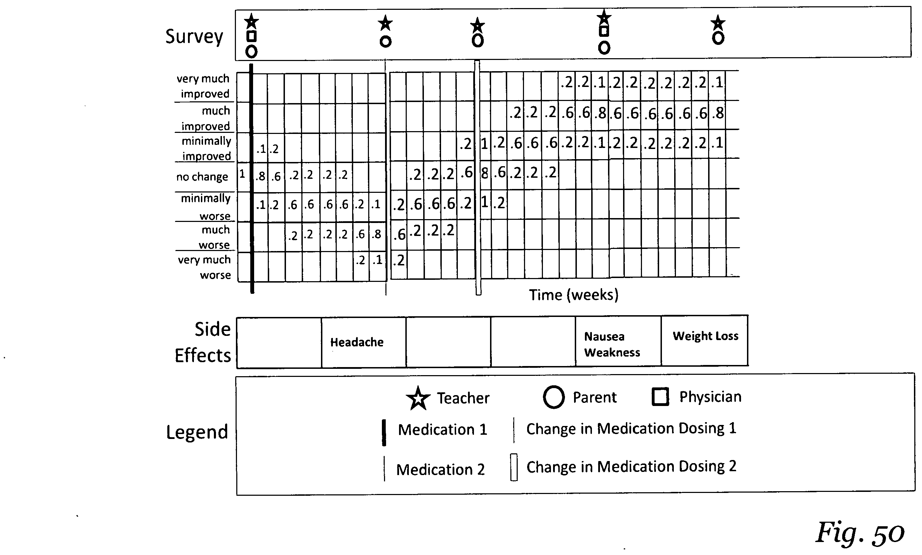

FIG. 50 shows yet another follow-up at which point it is established that the patient is most probably stably improved, and has been stably improved between the two evaluations. Note that due to the applied inference, the PDF for the patient trajectory is continuously estimated. However, the precision (the concentration of the PDF) is higher in the presence of a measurement.

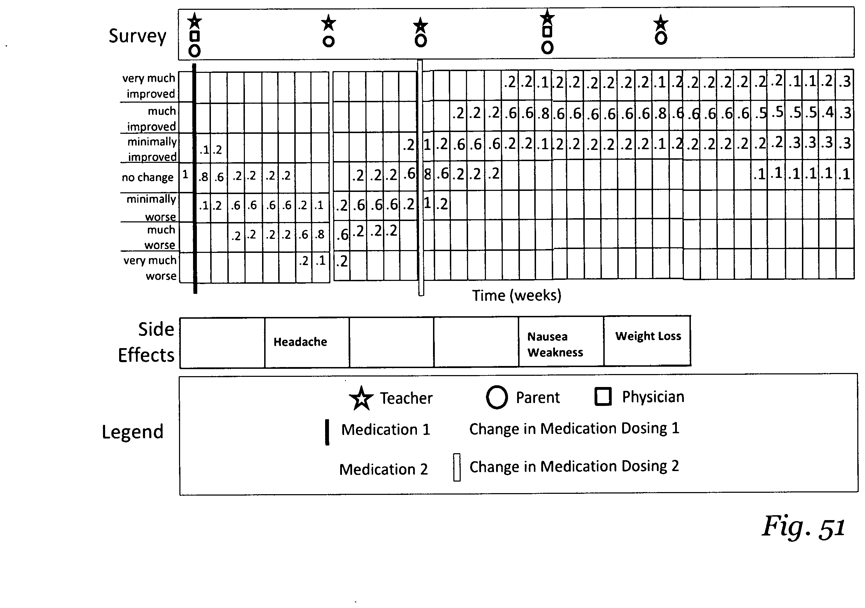

FIG. 51 shows a follow-up evaluation of the patient and the patient trajectory in the absence of measurements. Due to the lack of recent observations, the uncertainty is increasing.

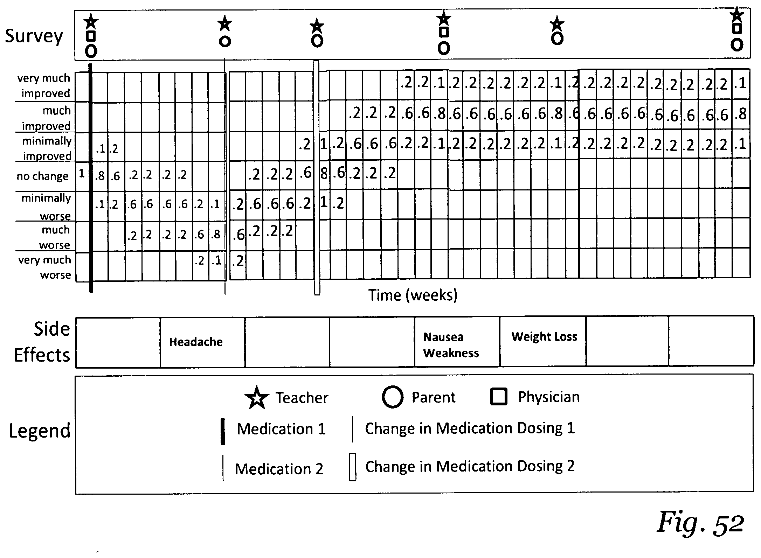

FIG. 52 shows the state of this uncertainty given a full patient evaluation (all measurement modalities). The inference engine propagates this uncertainty back in time to produce a more precise estimation of the patient trajectory, which helps the clinician to deduct that the patient is stable.

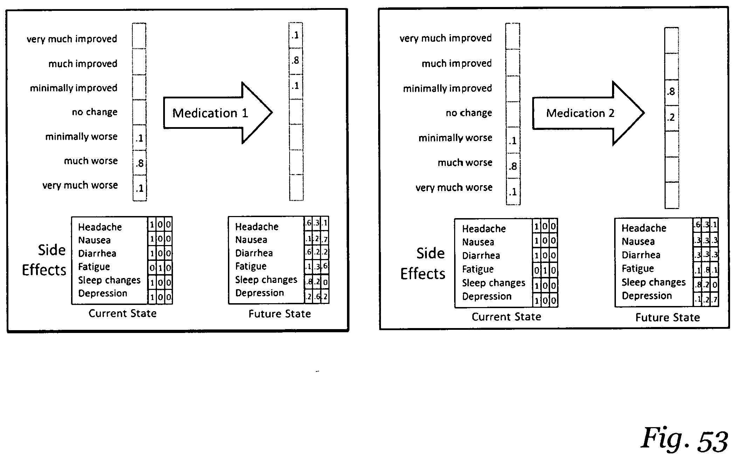

FIG. 53 illustrates yet another possible visualization from the described system output. It shows possible patient state transitions under changes of treatment plan, e.g., change of medication. It also conveys what possible side-effects can be expected. For every side effect, there are three stages of manifestation--mild, moderate, and severe represented with the three boxes next to each side effect in the figure. The coloring corresponds to the probability of a particular severity manifestation for each particular side-effect, with darker colors indicating higher probability.

The evaluation and monitoring of adequate oxygen delivery is possibly the most important aspect of pediatric CICU care [1], especially in postoperative patients. Unfortunately, oxygen demand, delivery, and consumption cannot be measured directly. Instead, physicians rely on physiologic proxy measurements (e.g. oxygen saturation, serum lactate) and assumptions to provide information about a patient's status [1], [2]. In postoperative cardiac patients, one of the most important proxies is the mixed venous oxygen saturation, which is obtained from a catheter placed in the SVC. This measurement is used to derive systemic perfusion [3], and subsequently detect anaerobic metabolism [4]. Despite the invasive nature of this physiologic measurement, it is commonly used because of its success as a predictor of outcome [5]. However, indwelling catheters contribute significantly to patient morbidity and mortality [6], leading to prolonged ICU stays and increased medical costs [7]. It is therefore unsurprising that there is a national drive to venous oxygen saturation in pediatric postoperative patients [9], [10], [11], [12], [13]. However, the variable and uncertain nature of this relationship has reduced the utility of NIRS as a noninvasive tool for hemodynamic assessment [8]. Additionally, assumptions in the proxy models further limit the utility of this measurement. As an example, when evaluating the ratio of pulmonary to systemic flow in a single ventricle patient (a calculation that requires $SvO_2$) the pulmonary venous saturation is assumed to be higher than 95%, despite clinical evidence showing that this is not always true [14]. Thus propagating multiple uncertainties leads to an inaccurate - if not clinically dangerous - evaluation of a patient's physiologic status.

Evaluation and management of tissue oxygen delivery ($DO_2$) is a cornerstone of care in the CICU. The estimation of this physiologic variable is dependent on proxy measurements using invasive catheters. The risks associated with these catheters often offset the utility of the measurements. One such proxy is venous oxygen saturation ($SvO_2$) measured using a superior vena cava (SVC) catheter. A noninvasive alternative is cerebral blood oxygenation ($rSO_2$) as measured through Near Infrared Spectroscopy (NIRS). However, the clinical benefits of using NIRS are offset by the inherent measurement uncertainty and resultant medical risks.

Accordingly, there is a need for methods and systems for producing stochastic cellular materials in a simpler, easier, and more accurate manner than existing methods. Additionally, there is the need for novel methods and systems for representing this information in a simpler, more intuitive, more integrated manner than existing methods.

The invention described herein provides a system and method for representing patient risks and other clinical information. The risks correspond to the probabilities that the patient exhibits particular conditions and respective etiologies. In addition the invention discusses a method for representing the utility of different possibly harmful modes of physiologic monitoring in estimating these risks.

The invention also discusses a method for applying this risk representation in the care of patients.

In a different possible embodiment of the discussed invention, it can employ methods and systems for computing the patient risks as the ones described in the following provisional patents: (61/614,861), (61/614,846), (61/620,144), (61/684,241).

The invention described herein provides a system and method for representing patient risks and other clinical information.

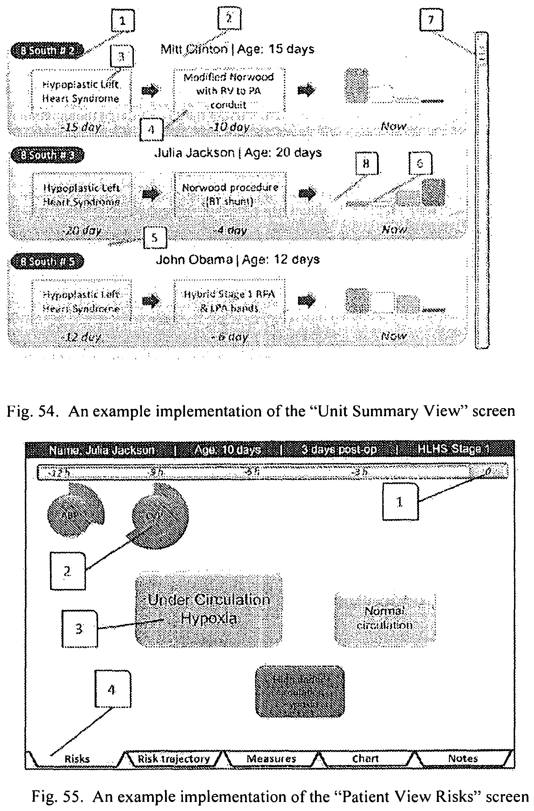

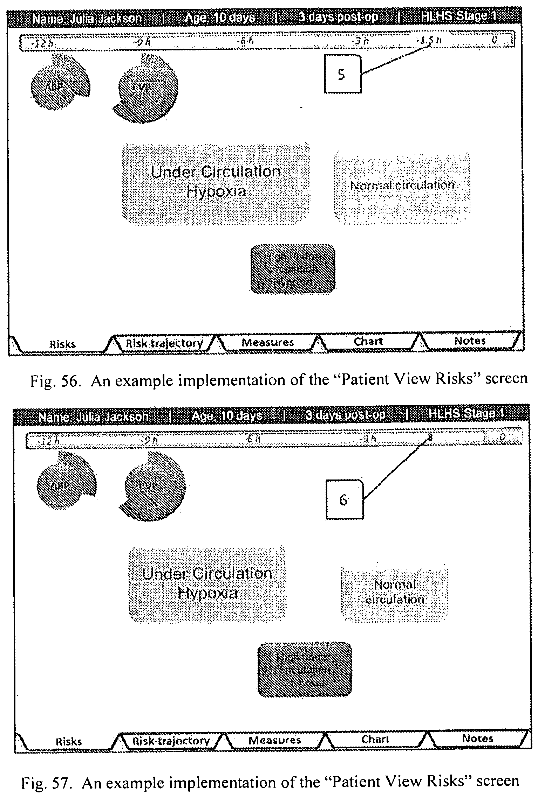

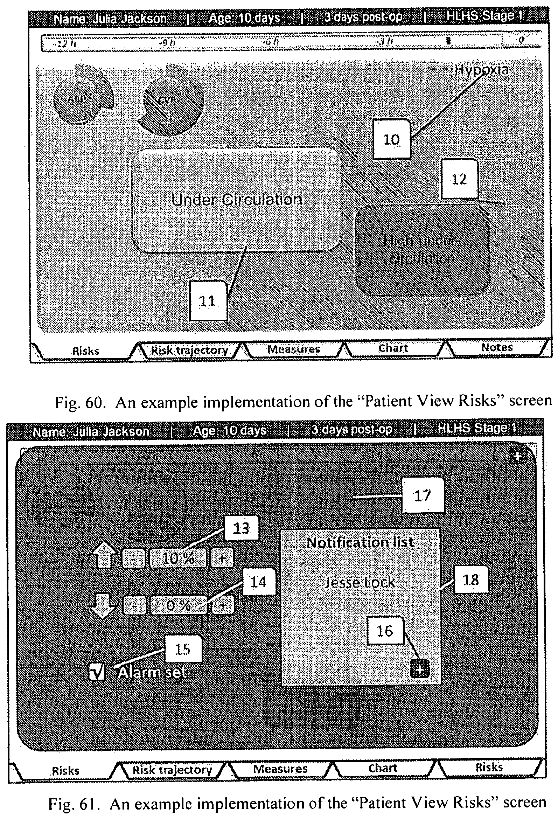

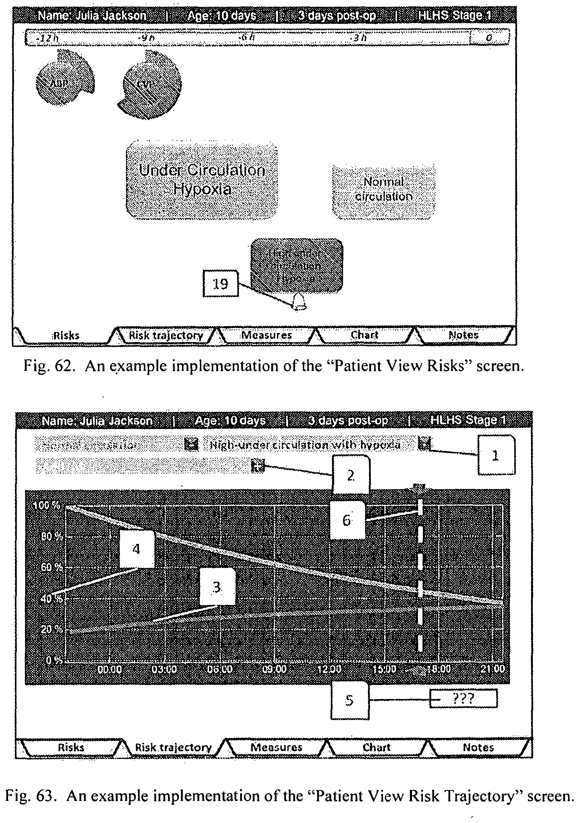

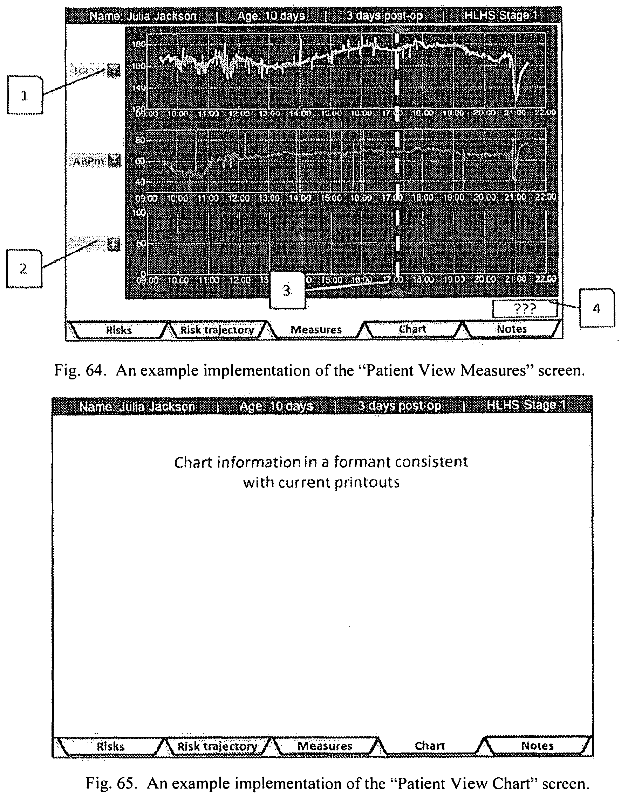

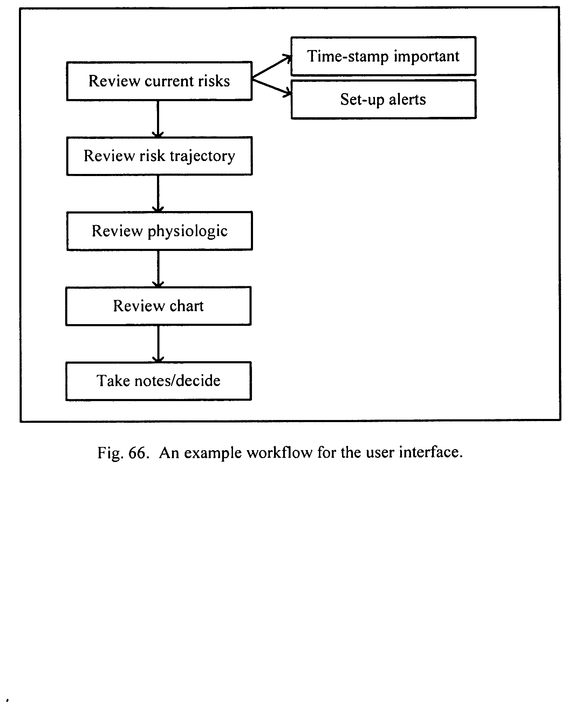

In one possible embodiment of the invention, the invention enables a user interface with a workflow as the one described in Figure 66. In this workflow, the user first chooses a specific patient from a list of patients (Figure 54). The user then transitions to a view that enables them to review the current risks to the patient (Figure 55), and provides them with the ability to set alerts for specific risks (Figure 61) or put timestamps for important events (Figure 57). The user can then review a historic timeline of the risks (Figure 63), which enables them to review how the risks have changed over a specific time period. Following this, the user can review the physiology measurements which were used as the basis for the calculated risks (Figure 64), review the patient chart (Figure 65), and take notes on the patient.

The screen depicted in Figures 55 through 62 is called the "Patient View Risks". The purpose of this screen is to present the probability of the patient being in each possible clinical state, the hazard level associated with each of these states, as well as the utility of each available measurement to the user.

With this screen, the user also has the ability to set alarms. These alarms can be set up to trigger either when one of the probabilities of the patient states crosses a threshold set by the user or by any other event of interest (e.g. change of medication). When an alarm triggers, it can also automatically set a time-stamped tag. An example implementation is illustrated by the markers on Figures 55 through 62. In Figure 55, the markers represent the following: (1) sliding button allows visualizing how the patient risks have changed with time, (2) the utility of each measurement (full halo corresponds to maximum utility), (3) possible patient state given the currently available measurements, and (4) navigation tab. In marker #3, the color represents hazard level of the state (four possible states: Minimum Hazard, Mild Hazard, Moderate Hazard, Severe Hazard, ranging from green to red respectively) and the size of the box corresponds to probability. In Figure 56, marker #5 illustrates that dragging the sliding window and pressing it down produces the result of marker #6 (shown in Figure 57).

The user also has the ability to replay portions of the patient's timeline. This gives the user manual control in viewing the history of the patient's stay.

The current physiological measures currently in use are also displayed on the screen. These measures are represented with the name of the measure encircled by a bar chart in the shape of a halo. The color of the halo corresponds to the extent to which that measure contributes to the currently viewed state of the patient. Potential measurements that are available but not in use can also be displayed on the screen.

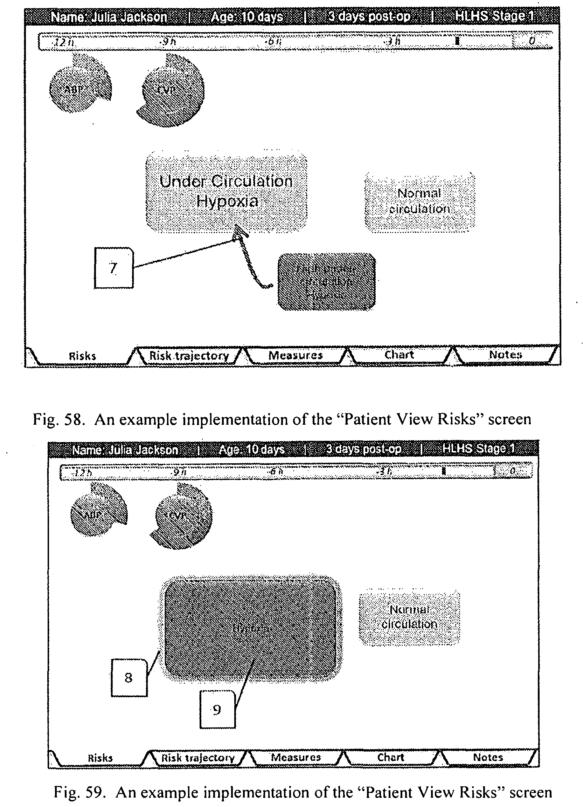

In Figure 58, the user has the ability to customize the clinical states that are viewed through several methods. Marker #7 shows that dragging one state into another state produces a composite state, highlighted by marker #8 (in Figure 59). Clicking on this composite state (denoted by marker #9) allows the user to see the states that the composite state consists of. In Figure 60, the name of the composite state is denoted by marker #10. The naming of the states themselves is based on hierarchical organization that is handled by the backend (one of which is denoted by marker #11). Marker #12 highlights the color, which captures the risk of the composite state.

As part of an additional alarm screen (shown in Figure 61), the user can set upper (marker #13) and lower (marker #14) limits for each risk displayed on the "Patient View Risks" screen. The alarm can be set via the checkmark box shown by marker #15. Marker #16 designates the button where the user can choose people to be notified when the alarm is triggered. The alarm screen is overlaid on top of the "Patient View Risks" screen (highlighted by marker #17). The box represented by marker #18 is a list which shows people to be notified on the onset of an alarm. Finally, Figure 62 shows the states associated with alarms are marked (marker #19).

The next screen is called the "Patient View Risk Trajectory" as shown in Figure 63. The purpose of this screen is to allow the user to display the risks for the patient being in a particular state as functions of time. The user is able to choose: (1) the probability of what states or combination of states to display, and (2) at what time scale to display them. An example implementation is shown in Figure 63, where marker #1 shows that by clicking the minus button the user deletes a particular state probability history and marker #2 shows by clicking the plus button the user can add additional states or combination of states. Marker #3 illustrates the probability evolution of a particular state, which is measured by the probability axis (denoted by marker #4). Marker #5 shows a tool for adjusting the time scale. Marker #6 designates a line shows time stamps corresponding to features 5 (illustrated by marker #5 in Figure 56) and 6 (illustrated by marker #6 in Figure 57) of "Patient View Risks".

A crucial feature is the ability to "tag" events that the user desires to view at a later time. The user can search events to tag based on medication, specific times of the patient's stay, etc. These tags can mark time-series for all views, can be shared, can be included in notes, etc. Moreover, the user can annotate tags. These tags can be accessed in note form, where the patient state corresponding to the time tagged will be displayed as a screenshot. This also gives the user the ability to search through the tags, which can include tags that correspond to state probability thresholds.

The next screen is called "Patient View Chart". The purpose of this screen is to present the user with the same information as the one provided to them by the rounding chart. An example implementation is shown in Figure 65, where the content of this view is pulled directly from the clinician's rounding chart.

The final screen is called "Patient View Notes". The purpose of this screen is to present the user with the ability to take notes. Figure 66 provides an example workflow that gives an order of actions through the interface. For each patient, the user reviews the current risks to the patient. At this time, the user can tag a desired point in time for future reference and can set up alerts as described in the alert feature description above. Next, the user can review risk trajectory for the patient. From there, the user can choose to review the physiological measurements as well as the patient's chart. Finally, using the notes tab, the user can take notes on the patient.

**REFERENCES**

[1] P. A. Chechia, P.C. Laussen., "The cardiac intensive unit perspective on hemodynamic monitoring of oxygen transport balance." Pediatric Critical Medicine, 2011, Issue 4, Vol. 12, pp. S69-S71.

[2] Spenceley, Neil, et al., "Monitoring in pediatric cardiac critical care: A worldwide perspective." Pediatric Critical Care Medicine, 2011, Issue 4, Vol. 12, pp. S76-S80.

[3] Bohn, D., "Objective assesment of cardiac output in infants after cardiac surgery." Seminars in Thoracic and Cardiovascular Surgery: Pediatric Cardiac Surgery Annual, 2011, Issue 1, Vol. 14, pp. 19-23.

[4] G. M. Hoffman, N. S. Ghanayem, J. M. Kampine, S. Berger, Kathleen A Mussatto, S.B. Litwin, J. S. Tweddell., "Venous saturation and the anaerobic threshold in neonates after the Norwood procedure for hypoplastic left heart syndrome." The Annals of Thoracic Surgery, 2000, Issue 5, Vol. 70.

[5] S. M. Bradley, A. M. Atz., "Postoperative management: The role of mixed venous oxygen saturation monitoring." Seminars in Thoracic and Cardiovascular Surgery: Pediatric Cardiac Surgery Annual, 2005, Issue 1, Vol. 8, pp. 22-27.

[6] Pinilla, J, et al., "Study of the incidence of intravascular catheter infection and associated septicemia in critically ill patients." Critical Care Medicine, 1983, Vol. 11, pp. 21-25.

[7] DK, Warren, WW, Quadir and CS, Hollenbeak., "Attributable cost of catheter-associated bloodstream infections among intensive care patients in a nonteaching hospital." s.l. : Critical Care Medicine, 2006, Vol. 34, pp. 2084-9.

[8] S. Chakravarti, S Srivastava, A. J. C. Mitnacht., "Near Infrared Spectroscopy (NIRS) in Children ." Seminars in Cardiothorasic and Vascular Anesthesia, 2008, Issue 70, Vol. 12.

[9] Tortoriello TA, Stayer SA, Mott AR, McKenzie ED, Fraser CD, Andropoulos DB, Chang AC., "A noninvasive estimation of mixed venous oxygen saturation using near-infrared spectroscopy by cerebral oximetry in pediatric cardiac surgery patients." Pediatric Anesthesia, 2005, Issue 6, Vol. 15, pp. 495-503.

[10] P. M. Kirshbom, J. M. Forbess, B. E. Kogon, J. M. Simsic, et al., "Cerebral Near Infrared Spectroscopy Is a Reliable Marker of Systemic Perfusion in Awake Single Ventricle Children." PEDIATRIC CARDIOLOGY, 2007, Issue 1, Vol. 28, pp. 42-45.

[11] N. Nagdyman, T. Fleck, S. Barth, H. Abdul-Khaliq, B. Stiller., "Relation of cerebral tissue oxygenation index to central venous oxygen saturation in children." INTENSIVE CARE MEDICINE, 2004, Issue 3, Vol. 30, pp. 468-471.

[12] McQuillen PS, Nishimoto MS, Bottrell CL, Fineman LD, Hamrick SE, Glidden DV, Azakie A, Adatia I, Miller SP., "Regional and central venous oxygen saturation monitoring following pediatric cardiac surgery: concordance and association with clinical variables." Pediatric Critical Care Medicine, 2007, Issue 2, Vol. 8, pp. 154-160.

[13] Bhutta AT, Ford JW, Parker JG, Prodhan P, Fontenot EE, Seib PM, Stroope BI, Frazier EA, Schmitz ML, Drummond-Webb JJ, Morrow WR., "Noninvasive cerebral oximeter as a surrogate for mixed venous saturation in children." Pediatric Cardiology, 2007, Issue 1, Vol. 28, pp. 34-41.

[14] Taeed, R., Schwartz, S. M. and J. M. Pearl, et al., "Unrecognized Pulmonary Venous Desaturation Early After Norwood Palliation Confounds p:s Assessment and Compromises Oxygen Delivery." Circulation, 2001, Vol. 103, pp. 2699-2704.

Various examples and embodiments consistent with the present disclosure have be described in detailed above. It is to be understood that these examples and embodiments of the present disclosure are provided for exemplary and illustrative purposes only. Various modifications and changes may be made to the disclosed embodiments by persons skilled in the art without departing from the scope of the present disclosure as defined in the appended claims.

**Claims**

1. A computer-implemented method of
graphically communicating, to a clinician, an assessment of the utility of invasive measurements that may harm the
patient, the method comprising:

    receiving, from a device, data relating to a plurality of possible clinical patient states in which the patient may be
    classified;
    determining the utility of a particular invasive catheter measurement $m_i$, by employing hazard levels, $r_i$, assigned
    to each patient state ($S_i$) by:

       submitting an instruction to a physiology observer module (122) to simulate physiology observer module
       output (probability density functions of internal state variables) from a point back from the current time
       ($t_{current}-T$) to the current time ($t_{current}$) with removal of measurement $m_i$;
       in a clinical trajectory interpreter module (123), simulate a state probability estimation given the simulated
       output of the physiology observer module, to arrive at a set of patient state probabilities $P_{sim}(S_1(t_{current}))$, $P_{sim}$
       $(S_2(t_{current}))$ . . . $P_{sim}(S_n(t_{current}))$;
       in the clinical trajectory interpreter module (123), using state probabilities determined from all available
       measurements P(S1(tcurrent), P(S2tcurrent), ... P(Sn(tcurrent)), calculating the utility [U(mi)] of the measurement mi using the formula:

$$U(m_i) = D_{weighted}(P_{sim}|P) = \sum_{i=1}^{n} r_i P(S_i(t_{current})) \log \left( \frac{P(S_i(t_{current}))}{P_{sim}(S_i(t_{current}))} \right)$$

       and;

    displaying a graphical indicator configured to represent the utility of the invasive measurement.

2. The computer-implemented method of graphically communicating the utility of invasive measurements according to
claim 1, wherein the receiving, from the device, the data relating to the plurality of possible clinical patient states in
which the patient may be classified, comprises:
receiving data identifying the plurality of possible clinical patient states in which the patient may be classified, and for
each of the plurality of possible clinical patient states, further comprises:

    receiving data identifying the probability that the patient is in each of the possible patient states over the range of
    time; and
    receiving data identifying a hazard level into which each of the possible patient states is categorized.

3. The computer-implemented method of graphically communicating the utility of invasive measurements according to
claim 1, wherein:

    the displaying of graphical indicators comprises a bar chart having a plurality of bars, each bar associated with one
    of a plurality of hazard levels; wherein particularly a bar within the bar chart may comprise a plurality of graphical
    indicators within a single hazard level; and
    each of the plurality of graphical indicators comprises a bar in the bar chart.

4. The computer-implemented method of graphically communicating the utility of invasive measurements according to
claim 3, wherein

    each of the bars has a length corresponding to the probability that the patient is in the corresponding clinical
    patient state at a given point in time
    or
    wherein the hazard level of each of the graphical indicators is expressed by the color of said graphical indicator.

5. The computer-implemented method of graphically communicating the utility of invasive measurements according to
claim 1, further comprising:
displaying a state variable data control feature that, when activated by a user, causes a display device to display internal state variables on which the patient states are based.

6. The computer-implemented method of graphically communicating the utility of invasive measurements according to claim 1, further comprising:

displaying a tag creation feature that, when activated by a user, causes a display device to display a tag mark associated with the timeline Controller at a selected point within the range of time and
particularly displaying a tag note creation field configured to permit annotation, by the user, of information associated with the tag.

7. A non-transient Computer readable medium having computer-executable code for graphically communicating, to a clinician, utility of invasive measurements that may harm the patient, the computer-executable code comprising code for executing the method according to one of the claims 1 to 6.

8. A system comprising:

a display device;
a Computer processor configured to display a graphical user interface for communicating, to a clinican, utility of invasive measurements that may harm the patient according to the method defined in claims 1 to 6.

9. The System for communicating, to a user, utility of invasive measurements that may harm the patient according to claim 8, wherein:
an arrangement of graphical indicators on the display device comprises a bar chart having a plurality of bars, each bar associated with one of a plurality of hazard levels; and each of the plurality of graphical indicators comprises a bar in the bar chart, in particular wherein a bar within the bar chart may comprise a plurality of graphical indicators within a single hazard level.

10. The System for communicating, to a user, utility of invasive measurements that may harm the patient according to claim 9, wherein

each of the bars has a length corresponding to the probability that the patient is in the corresponding clinical patient state at a given point in time
or
wherein the hazard level of each of the graphical indicators is expressed by the color of said graphical indicator.

11. The System for communicating, to a user, utility of invasive measurements that may harm the patient according to claim 8, wherein:

the display device comprises a touch screen; and
the dynamic Controller is a slider displayed on the touch screen.

12. The System for communicating, to a user, utility of invasive measurements that may harm the patient according to claim 8, the graphical user interface further comprising:
a state variable data control feature that, when activated by the user, causes the display device to display internal state variables on which the patient states are based.

13. The System for communicating, to a user, utility of invasive measurements that may harm the patient according to claim 8, the graphical user interface further comprising:

a tag creation feature that, when activated by the user, causes the display device to display a tag mark associated with the timeline Controller at a selected point within the range of time and
particularly a tag note creation field configured to permit annotation, by the user, of information associated with the tag.

**Patentansprüche**

1. Computerimplementiertes Verfahren zur grafischen Kommunikation einer Bewertung der Nützlichkeit von invasiven Maßnahmen, die einem Patienten schaden können, an einen Kliniker, wobei das Verfahren umfasst:

Empfangen von Daten in Bezug auf eine Vielzahl von möglichen klinischen Patientenzuständen, in welche der Patient klassifiziert werden kann, von einer Vorrichtung;

Bestimmen der Nützlichkeit einer konkreten invasiven Katheter-Messung $m_i$ unter Einsatz von Risikoniveaus $r_i$, die jedem Patientenzustand (Si) zugeordnet sind, durch:

Eingeben einer Anweisung in ein Physiologie-Beobachtermodul (122), um eine Physiologie-Beobachtermodul-Ausgabe (Wahrscheinlichkeitsdichtefunktionen von internen Zustandsvariablen) von einem Punkt vor der aktuellen Zeit ($t_{current}$-T) bis zu der aktuellen Zeit ($t_{current}$) unter Entfernung der Messung $m_i$ zu simulieren;

Simulieren einer Zustands-Wahrscheinlichkeitsschätzung bei der gegebenen simulierten Ausgabe des Physiologie-Beobachtermoduls in einem Modul zur Interpretation des klinischen Verlaufs (123), um zu einer Menge von Patientenzustands-Wahrscheinlichkeiten $P_{sim}(S_1(t_{current}))$, $P_{sim}(S_2(t_{current}))$ ... $P_{sim}(S_n(t_{current}))$ zu gelangen;

Berechnen der Nützlichkeit [U(mi)] der Messung mi in dem Modul zur Interpretation des klinischen Verlaufs (123) unter Verwendung von Zustandswahrscheinlichkeiten, die aus allen verfügbaren Messungen P(S1(tcurrent), P(S2tcurrent), ... P(Sn(tcurrent) bestimmt wurden, unter Verwendung der Formel:

$$U(m_i) = D_{weighted}(P_{sim}|P) = \sum_{i=1}^{n} r_i P(S_i(t_{current})) \log\left(\frac{P(S_i(t_{current}))}{P_{sim}(S_i(t_{current}))}\right)$$

und;

Anzeigen eines grafischen Indikators, der dazu ausgestaltet ist, die Nützlichkeit der invasiven Messung darzustellen.

**2.** Computerimplementiertes Verfahren zur grafischen Kommunikation der Nützlichkeit von invasiven Maßnahmen nach Anspruch 1, wobei das Empfangen von Daten in Bezug auf die Vielzahl von möglichen klinischen Patientenzuständen, in welche der Patient klassifiziert werden kann, von der Vorrichtung umfasst:
Empfangen von Daten, welche die Vielzahl von möglichen klinischen Patientenzuständen identifizieren, in welche der Patient klassifiziert werden kann, und wobei das Verfahren für jeden der Vielzahl von möglichen klinischen Patientenzuständen ferner umfasst:

Empfangen von Daten, welche die Wahrscheinlichkeit identifizieren, dass der Patient sich über den Zeitbereich in jedem der möglichen Patientenzustände befindet; und
Empfangen von Daten, welche ein Risikoniveau identifizieren, in welches jeder der möglichen Patientenzustände kategorisiert wird.

**3.** Computerimplementiertes Verfahren zur grafischen Kommunikation der Nützlichkeit von invasiven Maßnahmen nach Anspruch 1, wobei:

das Anzeigen von grafischen Indikatoren ein Balkendiagramm umfasst, das eine Vielzahl von Balken aufweist, wobei jeder Balken einem einer Vielzahl von Risikoniveaus zugeordnet ist; wobei insbesondere ein Balken innerhalb des Balkendiagramms eine Vielzahl von grafischen Indikatoren innerhalb eines einzelnen Risikoniveaus umfassen kann; und
jeder der Vielzahl von grafischen Indikatoren einen Balken in dem Balkendiagramm umfasst.

**4.** Computerimplementiertes Verfahren zur grafischen Kommunikation der Nützlichkeit von invasiven Maßnahmen nach Anspruch 3, wobei

jeder der Balken eine Länge aufweist, die der Wahrscheinlichkeit entspricht, dass der Patient sich zu einem gegebenen Zeitpunkt in dem entsprechenden klinischen Patientenzustand befindet,
oder
wobei das Risikoniveau eines jeden der grafischen Indikatoren durch die Farbe des grafischen Indikators ausgedrückt wird.

5. Computerimplementiertes Verfahren zur grafischen Kommunikation der Nützlichkeit von invasiven Maßnahmen nach Anspruch 1, ferner umfassend:
Anzeigen eines Zustandsvariablen-Datenkontrollmerkmals, das, wenn es von einem Benutzer aktiviert wird, eine Anzeigevorrichtung veranlasst, interne Zustandsvariablen anzuzeigen, auf welchen die Patientenzustände basieren.

6. Computerimplementiertes Verfahren zur grafischen Kommunikation der Nützlichkeit von invasiven Maßnahmen nach Anspruch 1, ferner umfassend:

Anzeigen eines Markierungserstellungsmerkmals, das, wenn es von einem Benutzer aktiviert wird, eine Anzeigevorrichtung veranlasst, eine Markierung, die der Zeitleisten-Steuerung zugeordnet ist, an einem ausgewählten Punkt innerhalb des Zeitbereichs anzuzeigen, und
insbesondere Anzeigen eines Markierungs-Anmerkungserstellungs-Felds, das dazu ausgestaltet ist, die Anmerkung von Informationen, die der Markierung zugeordnet sind, durch den Benutzer zu erlauben.

7. Nicht-transitorisches computerlesbares Medium, das computerausführbaren Code aufweist, um an einen Kliniker grafisch die Nützlichkeit von invasiven Messungen zu kommunizieren, die dem Patienten schaden können, wobei der computerausführbare Code Code zum Ausführen des Verfahrens nach einem der Ansprüche 1 bis 6 umfasst.

8. System, umfassend:

eine Anzeigevorrichtung;
einen Computerprozessor, der dazu ausgestaltet ist, eine grafische Benutzerschnittstelle anzuzeigen, um an einen Kliniker grafisch die Nützlichkeit von invasiven Messungen, die dem Patienten schaden können, nach dem in den Ansprüchen 1 bis 6 definierten Verfahren zu kommunizieren.

9. System zur Kommunikation der Nützlichkeit von invasiven Messungen, die dem Patienten schaden können, an einen Benutzer nach Anspruch 8, wobei:
eine Anordnung von grafischen Indikatoren auf der Anzeigevorrichtung ein Balkendiagramm umfasst, das eine Vielzahl von Balken aufweist, wobei jeder Balken einem einer Vielzahl von Risikoniveaus zugeordnet ist; und wobei jeder der Vielzahl von grafischen Indikatoren einen Balken in dem Balkendiagramm umfasst, wobei insbesondere ein Balken innerhalb des Balkendiagramms eine Vielzahl von grafischen Indikatoren innerhalb eines einzelnen Risikoniveaus umfassen kann.

10. System zur Kommunikation der Nützlichkeit von invasiven Messungen, die dem Patienten schaden können, an einen Benutzer nach Anspruch 9, wobei

jeder der Balken eine Länge aufweist, die der Wahrscheinlichkeit entspricht, dass der Patient sich zu einem gegebenen Zeitpunkt in dem entsprechenden klinischen Patientenzustand befindet,
oder
wobei das Risikoniveau eines jeden der grafischen Indikatoren durch die Farbe des grafischen Indikators ausgedrückt wird.

11. System zur Kommunikation der Nützlichkeit von invasiven Messungen, die dem Patienten schaden können, an einen Benutzer nach Anspruch 8, wobei:

die Anzeigevorrichtung einen Berührungsbildschirm umfasst; und
die dynamische Steuerung ein Schieberegler ist, der auf dem Berührungsbildschirm angezeigt wird.

12. System zur Kommunikation der Nützlichkeit von invasiven Messungen, die dem Patienten schaden können, an einen Benutzer nach Anspruch 8, wobei die grafische Benutzerschnittstelle ferner umfasst:
ein Zustandsvariablen-Datenkontrollmerkmal, das, wenn es von dem Benutzer aktiviert wird, die Anzeigevorrichtung veranlasst, interne Zustandsvariablen anzuzeigen, auf welchen die Patientenzustände basieren.

13. System zur Kommunikation der Nützlichkeit von invasiven Messungen, die dem Patienten schaden können, an einen Benutzer nach Anspruch 8, wobei die grafische Benutzerschnittstelle ferner umfasst:

ein Markierungserstellungsmerkmal, das, wenn es von dem Benutzer aktiviert wird, die Anzeigevorrichtung veranlasst, eine Markierung, die der Zeitleisten-Steuerung zugeordnet ist, an einem ausgewählten Punkt innerhalb des Zeitbereichs anzuzeigen, und

insbesondere ein Markierungs-Anmerkungserstellungs-Feld, das dazu ausgestaltet ist, die Anmerkung von Informationen, die der Markierung zugeordnet sind, durch den Benutzer zu erlauben.

## Revendications

1. Procédé mis en œuvre par ordinateur de communication graphique, à un clinicien, d'une évaluation de l'utilitaire de mesures invasives qui peuvent nuire au patient , le procédé comprenant:

la réception, en provenance d'un dispositif, de données relatives à une pluralité d'états cliniques possibles de patient dans lesquels le patient peut être classé ;

la détermination de l'utilitaire d'une mesure invasive particulière de cathéter $m_i$, en employant des niveaux de risques, $r_i$, attribués à chaque état de patient (Si) par :

soumission d'une instruction à un module d'observation de physiologie (122) pour simuler un résultat de module d'observation de physiologie (fonctions de densité de probabilité de variables d'état interne) à partir d'un moment en arrière du temps courant ($t_{courant}$-T) jusqu'au temps courant ($t_{courant}$) avec suppression de la mesure $m_i$ ;

dans un module d'interprétation de trajectoire clinique (123), simulation d'une estimation de probabilité d'état étant donné le résultat simulé du module d'observation de physiologie, pour parvenir à un ensemble de probabilité d'état de patient $P_{sim}(S_1(t_{courant}))$, $P_{sim}(S_2(t_{courant}))$, ..., $P_{sim}(S_n(t_{courant}))$ ;

dans le module d'interprétation de trajectoire clinique (123), en utilisant les probabilités d'état déterminées à partir de toutes les mesures disponibles $P(S_1(t_{courant}))$, $P(S_2(t_{courant}))$, ..., $P(S_n(t_{courant}))$, calcul de l'utilitaire $[U(m_i)]$ de la mesure $m_i$ en utilisant la formule :

$$U(m_i) = \mathbf{D_{pondéré}} \ (P_{sim}|P) = \sum_{i=1}^{n} r_i P(S_i \ (t_{courant}) \ )\log\left(\frac{P(S_i \ (t_{courant})}{P_{sim}(S_i \ _{(t_{courant})})}\right)$$

et ;

l'affichage d'un indicateur graphique configuré pour représenter l'utilitaire de la mesure invasive.

2. Procédé mis en œuvre par ordinateur de communication graphique de l'utilitaire de mesures invasives selon la revendication 1, dans lequel la réception, en provenance du dispositif, des données relatives à la pluralité d'états cliniques possibles de patient dans lesquels le patient peut être classé, comprend :

la réception de données identifiant la pluralité d'états cliniques possibles de patient dans lesquels le patient peut être classé, et pour chacun de la pluralité d'états cliniques possibles de patient, comprend en outre :

la réception de données identifiant la probabilité que le patient soit dans chacun des états possibles de patient sur la plage de temps ; et

la réception de données identifiant un niveau de risque dans lequel chacun des états possibles de patient est catégorisé.

3. Procédé mis en œuvre par ordinateur de communication graphique de l'utilitaire de mesures invasives selon la revendication 1, dans lequel :

l'affichage des indicateurs graphiques comprend un diagramme à barres comportant une pluralité de barres, chaque barre étant associée à l'un de la pluralité de niveaux de risque ; dans lequel en particulier une barre au sein du diagramme à barres peut comprendre une pluralité d'indicateurs graphiques au sein d'un niveau de risque unique ; et

chacun de la pluralité d'indicateurs graphiques comprend une barre dans le diagramme à barres.

4. Procédé mis en œuvre par ordinateur de communication graphique de l'utilitaire de mesures invasives selon la revendication 3, dans lequel

chacune des barres a une longueur correspondant à la probabilité que le patient soit dans l'état clinique de patient correspondant à un moment donné dans le temps ou
dans lequel le niveau de risque de chacun des indicateurs graphiques est exprimé par la couleur dudit indicateur graphique.

5. Procédé mis en œuvre par ordinateur de communication graphique de l'utilitaire de mesures invasives selon la revendication 1, comprenant en outre :
l'affichage d'une fonction de contrôle de données de variables d'état qui, lorsqu'elle est activée par un utilisateur, amène un dispositif d'affichage à afficher des variables d'état interne sur lesquelles les états de patient sont basés.

6. Procédé mis en œuvre par ordinateur de communication graphique de l'utilitaire de mesures invasives selon la revendication 1, comprenant en outre :

l'affichage d'une fonction de création d'étiquette qui, lorsqu'elle est activée par un utilisateur, amène un dispositif d'affichage à afficher une marque d'étiquette associée au contrôleur de chronologie à un moment choisi au sein de la plage de temps et
en particulier l'affichage d'un champ de création de note d'étiquette configuré pour permettre une annotation, par l'utilisateur, d'informations associées à l'étiquette.

7. Support non transitoire lisible par ordinateur comportant un code exécutable par ordinateur permettant la communication graphique, à un clinicien, d'un utilitaire de mesures invasives qui peuvent nuire au patient, le code exécutable par ordinateur comprenant un code permettant d'exécuter le procédé selon l'une des revendications 1 à 6.

8. Système comprenant :

un dispositif d'affichage ;
un processeur d'ordinateur configuré pour afficher une interface utilisateur graphique permettant la communication, à un clinicien, d'un utilitaire de mesures invasives qui peuvent nuire au patient selon le procédé défini dans les revendications 1 à 6.

9. Système permettant la communication, à un utilisateur, d'un utilitaire de mesures invasives qui peuvent nuire au patient selon la revendication 8, dans lequel :
un agencement d'indicateurs graphiques sur le dispositif d'affichage comprend un diagramme à barres comportant une pluralité de barres, chaque barre étant associée à l'un de la pluralité de niveaux de risque ; et chacun de la pluralité d'indicateurs graphiques comprend une barre dans le diagramme à barres, en particulier dans lequel une barre au sein du diagramme à barres peut comprendre une pluralité d'indicateurs graphiques au sein d'un niveau de risque unique.

10. Système permettant la communication, à un utilisateur, d'un utilitaire de mesures invasives qui peuvent nuire au patient selon la revendication 9, dans lequel

chacune des barres a une longueur correspondant à la probabilité que le patient soit dans l'état clinique de patient correspondant à un moment donné dans le temps ou
dans lequel le niveau de risque de chacun des indicateurs graphiques est exprimé par la couleur dudit indicateur graphique.

11. Système permettant la communication, à un utilisateur, d'un utilitaire de mesures invasives qui peuvent nuire au patient selon la revendication 8, dans lequel :

le dispositif d'affichage comprend un écran tactile ; et
le contrôleur dynamique est un curseur affiché sur l'écran tactile.

12. Système permettant la communication, à un utilisateur, d'un utilitaire de mesures invasives qui peuvent nuire au patient selon la revendication 8, l'interface utilisateur graphique comprenant en outre :
une fonction de contrôle de données de variables d'état qui, lorsqu'elle est activée par l'utilisateur, amène le dispositif d'affichage à afficher des variables d'état interne sur lesquelles les états de patient sont basés.

13. Système permettant la communication, à un utilisateur, d'un utilitaire de mesures invasives qui peuvent nuire au patient selon la revendication 8, l'interface utilisateur graphique comprenant en outre :

une fonction de création d'étiquette qui, lorsqu'elle est activée par l'utilisateur, amène le dispositif d'affichage à afficher une marque d'étiquette associée au contrôleur de chronologie à un moment choisi au sein de la plage de temps et

en particulier un champ de création de note d'étiquette configuré pour permettre une annotation, par l'utilisateur, d'informations associées à l'étiquette.

*Fig. 1*

EP 4 410 199 B1

**PREDICT**
**210**

Predicted ISVs
**211**

Dynamic model
**212**

Current ISVs estimates
**213**

**UPDATE**
**220**

Observation model
**221**

Inference engine
**222**

Data reception module
**121**

Heart rate | Hemoglobin | Arterial Blood pressure | Central Venous Pressure | Lactic Acid | Arterial O2 saturation | Venous O2 saturation | Others

INITIAL ESTIMATES
**240**

$P(ISV_1(0), ISV_2(0), ISV_3(0), ..., ISV_n(0))$

Physiology Observer Module
**122**

**250**

$P(ISV_1(t), ISV_2(t), ISV_3(t), ..., ISV_n(t))$

*Fig. 2A*

*Fig. 2B*

*Fig. 2C*

*Fig. 2D*

*Fig. 3*

Fig. 4

Fig. 5

*Fig. 6*

*Fig. 7*

Fig. 8

*Fig. 9*

Fig. 10

Fig. 11

**Hazard Levels (HL)**

1 – Minimal risk.
2 – Mild risk.
3 – Medium risk.
4 – Severe risk.

Pulmonary Vascular Resistance

State 2
HL 4

State 3
HL 2

State 1
HL 3

State 4
HL 1

Cardiac output

*Fig. 12*

Fig. 13

Fig. 14

Select measurement $m_i$
9001

Simulate Physiology Observer Module output
9002

Simulate State probabilities estimation
9003

Calculate utility for $m_i$
9004

State probabilities determined from all available measurements
9006

Hazard levels
9005

Fig. 15

Physiology Observer Module
122

$P(ISV_1(t), ISV_2(t), ISV_3(t), ..., ISV_n(t))$

Clinical trajectory Interpreter module
123

State probabilities estimation
801

$P(newS_1), P(newS_2), P(newS_3), ..., P(newS_{n+m})$

EC

External computation
9110

Measurements utility determination
804

Hazard level assignment
802

Etiologies organization
803

Integration instructions
9120

Measurements utilities

Risks

Etiology tree

*Fig. 16*

| $S_1$ | $S_2$ | $S_3$ | $S_4$ |
|---|---|---|---|

Integration
instructions
**9120**

$P(A|\ S_i)$
$P(EC\ |\ A)$

$S_3$

$S_4$

**newS$_3$:**
$P(A=a_1, S_3 \mid EC) =$
$P(EC \mid A=a_1)$
$P(A=a_1|S_3)$
$P(S_3)/P(EC)$

**newS$_4$:**
$P(A=a_2, S_4) =$
$P(EC \mid A=a_1)$
$P(A=a_2|S_4)$
$P(S_4)/P(EC)$

**newS$_5$:**
$P(A=a_1, S_5 \mid EC) =$
$P(EC \mid A=a_1)$
$P(A=a_1|S_5)$
$P(S_5)/P(EC)$

**newS$_6$:**
$P(A=a_2, S_6) =$
$P(EC \mid A=a_1)$
$P(A=a_2|S_6)$
$P(S_6)/P(EC)$

*Fig. 17*

Enhance Physiology Observer Module with external computation
9300

EC

External computation
9110

$P(ISV_1(t), ISV_2(t), ISV_3(t), ..., ISV_n(t))$

Clinical trajectory Interpreter module
123

State probabilities estimation
801

Measurements utility determination
804

$P(S_1), P(S_2), P(S_3), ..., P(S_n)$

Reference material
130

Hazard level assignment
802

Etiologies organization
803

Measurements utilities

Risks

Etiology tree

Fig. 18

EP 4 410 199 B1

Data reception module 121

Physiology observer module 122

Clinical trajectory Interpreter module 123

$P(ISV_1(t), ISV_2(t), ISV_3(t), ..., ISV_n(t))$

Etiology tree

Risks

Invasive measurements utilities

| | | |
|---|---|---|
| Unit view of all patients 1501 | Patient electronic medical record 1502 | Patient ongoing risks 1503 |
| Patient risk trajectory 1504 | Patient measurements utilities 1505 | Patient estimated ISVs PDFs 1506 |
| Etiology tree navigation 1507 | Patient predicted risks 1508 | Patient Tags view 1511 |
| Patient risk based alarms 1509 | Patient monitor data 1510 | Patient condition summary 1512 |
| Reference and training material 1513 | Annotation and Tag setting 1514 | |

Visualization and user interactions module 124

*Fig. 19*

**Fig. 20**

Fig. 21

EP 4 410 199 B1

50

Fig. 22

2200

2210

2220

| Name: John Smith | Age: 10 days | 3 days post-op | HLHS Stage 1 |

-12 h   -9 h   -6 h   -3 h   0

Normal circulation

High Qp:Qs

Low Qp:Qs

Hypoxia

ABP □□□□□□  1 2 3 4 5 6
CVP □□□□□□  1 2 6

Legend:  Green   Yellow   Orange   Red

1 – Dark Green   2 – Light Green   3 – Yellow   4 – Red   5 – Purple   6 - Empty

Fig. 23

EP 4 410 199 B1

Fig. 24

Fig. 25

2600

| Name: John Smith | Age: 10 days | 3 days post-op | HLHS Stage 1 |

Normal circulation [-] High-under circulation with hypoxia [-]

Add risk [+]

Normal circulation

High-under circulation with hypoxia

*Fig. 26*

Fig. 27

EP 4 410 199 B1

Fig. 28

Fig. 29

3000

| Name: John Smith | Age: 10 days | 3 days post-op | HLHS Stage 1 |

-12 h   -9 h   -6 h   -3 h   0

3010 — Condition Summary

Shock due to low CO    23%

3020 — Definitions
Shock:    SvO$_2$ <= 45%    40%
Low CO:   CO < 3.2 L/min/m²    30%

3030 — Evidence:Reduced stroke volume manifested by reduced difference between systolic and diastolic blood pressures.

Learn More

Normal circulation   Over-circula...   ...ck cardiac output   Shock ...der-circulation

1 2 3 4 5 6
ABP □□□□□   CVP □□□□□

1 2 6

Legend:  ▨ Green    ▥ Yellow    ▤ Orange    ▦ Red

1 – Dark Green    2 – Light Green    3 – Yellow    4 – Red    5 – Purple    6 - Empty

Fig. 30

EP 4 410 199 B1

59

3100

| Name: John Smith | Age: 10 days | 3 days post-op | HLHS Stage 1 |

| -12 h | -9 h | -6 h | -3 h | 0 |

3120

3010

## Learn More

Hypoxia, low CO, HLHS Stage 1

Possible Causes: Low stroke volume, increased after load, bradicardia.

Possible Interventions: Inotropic support, vasodilator, pace maker.

Possible Complications: Metabolic acidosis, cardiac arrest.

## Condition Summary

Shock due to low CO | 23%

Definitions
Shock:    $SvO_2 <= 45\%$
Low CO:   $CO < 3.2$ L/min/m$^2$

Evidence: Reduced stroke volume manifested by reduced difference between systolic and diastolic blood pressures.

Learn More

3110

Hypoxia

cardiac output

Hypoxia

der-circulation

ABP □□□□□□   CVP □□□□□□

Legend: ▨ Green   ▥ Yellow   ▤ Orange   ▦ Red

1 – Dark Green   2 – Light Green   3 – Yellow   4 – Red   5 – Purple   6 – Empty

*Fig. 31*

EP 4 410 199 B1

Fig. 32

## HLHS Dynamics Model

$$DO_{2_k} = DO_{2_{k-1}} + c_{DO_2}\left(\overline{DO_2} - DO_{2_{k-1}}\right) + w_{k_{DO_2}} \qquad w_{k_{DO_2}} \sim N(0, Q_{DO_2}) \qquad (1)$$

$$VO_{2_k} = VO_{2_{k-1}} + c_{VO_2}\left(\overline{VO_2} - VO_{2_{k-1}}\right) + w_{k_{VO_2}} \qquad w_{k_\beta} \sim N(0, Q_\beta) \qquad (2)$$

$$PVR_k = PVR_{k-1} + \Delta PVR_{k-1} + w_{k_{PVR}} \qquad w_{k_{PVR}} \sim N(0, Q_{PVR}) \qquad (3)$$

$$SVR_k = SVR_{k-1} + \Delta SVR_{k-1} + w_{k_{SVR}} \qquad w_{k_{SVR}} \sim N(0, Q_{SVR}) \qquad (4)$$

$$\Delta PVR_k = \Delta PVR_{k-1} + w_{k_{\Delta PVR}} \qquad w_{k_{\Delta PVR}} \sim N(0, Q_{\Delta PVR}) \qquad (5)$$

$$\Delta SVR_k = \Delta SVR_{k-1} + w_{k_{\Delta SVR}} \qquad w_{k_{\Delta SVR}} \sim N(0, Q_{\Delta SVR}) \qquad (6)$$

$$Hb_k = Hb_{k-1} + c_{Hb}\left(\overline{Hb} - Hb_{k-1}\right) + w_{k_{Hb}} \qquad w_{k_{Hb}} \sim N(0, Q_{Hb}) \qquad (7)$$

$$HR_k = HR_{k-1} + w_{k_{HR}} \qquad w_{k_{HR}} \sim N(0, Q_{HR}) \qquad (8)$$

$$SpvO_{2_k} = 100 - w_{k_{SpvO_2}} \qquad w_{k_{SpvO_2}} \sim \exp(\lambda) \qquad (9)$$

$$\eta_k \sim N(0, \Sigma_\eta) \qquad (10)$$

$$CVP_k \sim gamma(A_{CVP}, B_{CVP}) \qquad (11)$$

*Fig. 33*

EP 4 410 199 B1

## HLHS Derived Variables

$$SaO_2 = \frac{DO_{2_k}SVR_k SpvO_{2_k}}{VO_{2_k}PVR_k + DO_{2_k}SVR_k} \tag{12}$$

$$SvO_{2_k} = \left(1 - \frac{VO_{2_k}}{DO_{2_k}}\right)\frac{SVR_k SpvO_{2_k}}{VO_{2_k}PVR_k + DO_{2_k}SVR_k} \tag{13}$$

$$\Delta P_k = \frac{VO_{2_k}PVR_k + DO_{2_k}SVR_k}{HR_k SVR_k \left(-c_3(VO_{2_k}PVR_k + DO_{2_k}SVR_k - c_5 SpvO_{2_k}Hb_k(c_3 CVP_k + c_4 + \eta_k)\right)} \tag{14}$$

$$ABPm_k = \frac{VO_{2_k}PVR_k + DO_{2_k}SVR_k}{c_5 SpvO_{2_k}Hb_k} + CVP_k \tag{15}$$

$$CO_k = \frac{VO_{2_k}PVR_k + DO_{2_k}SVR_k}{c_5 SpvO_{2_k}Hb_k}\left(\frac{1}{SVR_k} + \frac{1}{PVR_k}\right) \tag{16}$$

$$Q_p{:}Q_{s_k} = \frac{SVR_k}{PVR_k} \tag{17}$$

$$\Delta Q_p{:}Q_{s_k} = \frac{SVR_k}{PVR_k}\left(\frac{\Delta SVR_k}{\Delta t} - \frac{\Delta PVR_k}{\Delta t}\right) \tag{18}$$

*Fig. 34*

## HLHS Observation Model

$$\widetilde{SaO_2}_k = SaO_2 + n_{k_{SaO_2}} \qquad\qquad n_{k_{SaO_2}} \sim N(0, R_{SaO_2}) \qquad (19)$$

$$\widetilde{SvO_2}_k = SvO_{2_k} + n_{k_{SvO_2}} \qquad\qquad n_{k_{SvO_2}} \sim N(0, R_{SvO_2}) \qquad (20)$$

$$\widetilde{\Delta P}_k = \Delta P_k + n_{k_{\Delta P}} \qquad\qquad n_{k_{\Delta P}} \sim N(0, R_{\Delta P}) \qquad (21)$$

$$\widetilde{ABPm}_k = ABPm_k + n_{k_{ABPm}} \qquad\qquad n_{k_{ABPm}} \sim N(0, R_{ABPm}) \qquad (22)$$

$$\widetilde{HR}_k = HR_k + n_{k_{HR}} \qquad\qquad n_{k_{HR}} \sim N(0, R_{HR}) \qquad (23)$$

$$\widetilde{CVP}_k = CVP_k + n_{k_{CVP}} \qquad\qquad n_{k_{CVP}} \sim N(0, R_{CVP}) \qquad (24)$$

$$\widetilde{Hb}_k = Hb_k + n_{k_{Hb}} \qquad\qquad n_{k_{Hb}} \sim N(0, R_{Hb}) \qquad (25)$$

*Fig. 35*

Attributes relevant to the post-operative circulation management

| Total Cardiac Output | |
| --- | --- |
| Low | Normal |

3.8 L/min m²

| $Q_p:Q_s$ | | |
| --- | --- | --- |
| Low | Balanced | High |

0.75    1.5

| Hgb | |
| --- | --- |
| Low | Normal |

14 mg/dL

| SvO$_2$ | |
| --- | --- |
| Shock | No shock |

45 %

Patient states and etiology tree

Shock

No Shock

① Low total cardiac output

Normal total cardiac output

⑥ Balanced

Unbalanced

② Low Hgb

Normal Hgb

⑦ Low $Q_p:Q_s$

⑧ High $Q_p:Q_s$

③ Balanced

Unbalanced

④ Low $Q_p:Q_s$

⑤ High $Q_p:Q_s$

1. Shock caused by low cardiac output
2. Shock caused by low hemoglobin
3. Shock from unknown causes.
4. Shock caused by low Qp:Qs
5. Shock cause by high Qp:Qs
6. Normal hemodynamics
7. Low Qp:Qs
8. High Qp:Qs

*Fig. 36*

**Measurements**
3910

| Patient 101 | SvO2 3911 | Hgb 3912 | ABP (m|s|d) 3913 | Lactic acid 3914 | Blood pH 3915 | Heart Rate 3916 |

Yes

Treat? 3960

Clinician 3920

Enhanced visualization and user interactions module with outcomes 3941

Clinical trajectory Interpreter module 123

Physiology observer module 122

Patient demographics 3931

Possible treatment complications determination module 3942

Enhanced RISK-BASED PATIENT MONITORING with treatment evaluation 3940

Procedure 3932

Outcome database 3943

**Outcome studies**
3990

| Retrospective studies 3991 | Randomized clinical trials 3992 | Institution specific outcomes 3993 |

*Fig. 37*

EP 4 410 199 B1

No Hgb related
pathology
4001

Low Hgb
4010

Compensated
_____
Increasing Hgb
4011

Uncompensated
_____
Increasing Hgb
4012

Compensated
_____
Stable Hgb
4013

Uncompensated
_____
Stable Hgb
4014

Compensated
_____
Decreasing Hgb
4015

Uncompensated
_____
Decreasing Hgb
4016

*Fig. 38*

Fig. 39

EP 4 410 199 B1

No   **Treat?** 4202   Yes

**Low risk?** 4201

No   **Treat?** 4203   Yes

Yes

No

**Provide Justification** 4210

- Computed risk is acceptable
- Computed risk is inaccurate
- Additional attributes accounted

Risk stratification 4230

**Provide Justification** 4220

- Computed risk is unacceptable
- Computed risk is inaccurate
- Additional attributes accounted

Risk-based monitoring 4240

Do Not Treat 4260

Trial iNO 4250

*Fig. 40*

| High PVR<br>Low CO<br>*State 1* | High PVR<br>Norm CO<br>*State 3* |
|---|---|
| Norm PVR<br>Low CO<br>*State 2* | Norm PVR<br>Norm CO<br>*State 4* |

Patient states for iNO treatment
of low cardiac output (CO)
<u>4310</u>

**Low Risk**

P(State 1) < 10%
*-and-*
P(State 1) + P(State 2) < 30%

**High Risk**

P(State 1) > 10%
*-and-*
P(State 1) + P(State 2)> 30%

Risk stratification
<u>4230</u>

*Fig. 41*

Very much worse (1)

Much worse (2)

Worse (3)

Worse (4)

No change (5)

Min Improved (6)

Much Improved (7)

Very Much Improved (8)

$P_{12}$

$P_{21}$

$P_{11}$

*Fig. 42*

M 1

Clinical global impression-improvement scale

M 2

Vanderbilt Diagnosis: Parent

M 3

Vanderbilt Diagnosis: Teacher

*Fig. 43*

4601

Patient state @ t1

4602

Patient state @ t2

4603

Patient state @ t3

...

M 1

M 2

M 3

M 2

M 3

*Fig. 44*

Patient state Now

Statistics based prediction

Patient state +1 M

Med 1 Dose 1

Med 2 Dose 1

Patient state Now

Statistics based prediction

Patient state +1 M

Med 1 Dose 1

Med 1 Dose 2

*Fig. 45*

Survey

| very much improved | |
| much improved | |
| minimally improved | |
| no change | 1 |
| minimally worse | |
| much worse | |
| very much worse | |

Time (weeks)

Side Effects

Legend

☆ Teacher   ◯ Parent   ☐ Physician

❙ Medication 1   ❙ Change in Medication Dosing 1

❙ Medication 2   ▯ Change in Medication Dosing 2

*Fig. 46*

**Survey** ★ □ O ★ O

| | | | | | | | | |
|---|---|---|---|---|---|---|---|---|
| very much improved | | | | | | | | |
| much improved | | | | | | | | |
| minimally improved | .1 | .2 | | | | | | |
| no change | 1 | .8 | .6 | .2 | .2 | .2 | .2 | |
| minimally worse | | .1 | .2 | .6 | .6 | .6 | .6 | .2 | .1 |
| much worse | | | | .2 | .2 | .2 | .2 | .6 | .8 |
| very much worse | | | | | | | | .2 | .1 |

Time (weeks)

**Side Effects** | Headache

**Legend**

★ Teacher   O Parent   □ Physician

▌ Medication 1   Change in Medication Dosing 1

Medication 2   ▯ Change in Medication Dosing 2

*Fig. 47*

EP 4 410 199 B1

**Survey** ★ □ ○ ★ ○ ★ ○

| | | | | | | | | | | | | | | |
|---|---|---|---|---|---|---|---|---|---|---|---|---|---|
| very much improved | | | | | | | | | | | | | |
| much improved | | | | | | | | | | | | | |
| minimally improved | .1 | .2 | | | | | | | | | | .2 | 1 |
| no change | 1 | .8 | .6 | .2 | .2 | .2 | .2 | | | .2 | .2 | .2 | .6 | 8 |
| minimally worse | | .1 | .2 | .6 | .6 | .6 | .6 | .2 | .1 | .2 | .6 | .6 | .6 | .2 | 1 |
| much worse | | | | .2 | .2 | .2 | .2 | .6 | .8 | .6 | .2 | .2 | .2 |
| very much worse | | | | | | | | .2 | .1 | .2 |

Time (weeks)

**Side Effects**

| | Headache | |
|---|---|---|

**Legend**

★ Teacher  ○ Parent  □ Physician

| Medication 1 | Change in Medication Dosing 1 |
| Medication 2 | Change in Medication Dosing 2 |

*Fig. 48*

Survey

| | | | | | | | | | | | | | | | | | | |
|---|---|---|---|---|---|---|---|---|---|---|---|---|---|---|---|---|---|---|
| very much improved | | | | | | | | | | | | | | | | .2 | .2 | .1 |
| much improved | | | | | | | | | | | | .2 | .2 | .2 | .6 | .6 | .8 |
| minimally improved | .1 | .2 | | | | | | | .2 | 1 | .2 | .6 | .6 | .6 | .2 | .2 | .1 |
| no change | 1 | .8 | .6 | .2 | .2 | .2 | .2 | | .2 | .2 | .2 | .6 | 8 | .6 | .2 | .2 | .2 |
| minimally worse | | .1 | .2 | .6 | .6 | .6 | .6 | .2 | .1 | .2 | .6 | .6 | .6 | .2 | 1 | .2 | |
| much worse | | | | .2 | .2 | .2 | .2 | .6 | .8 | .6 | .2 | .2 | .2 | | | | |
| very much worse | | | | | | .2 | .1 | .2 | | | | | | | | | |

Time (weeks)

Side Effects

| | Headache | | |
|---|---|---|---|

Legend

☆ Teacher    ◯ Parent    ☐ Physician

▮ Medication 1    Change in Medication Dosing 1

Medication 2    Change in Medication Dosing 2

*Fig. 49*

Survey

| | very much improved | | | | | | | | | | | | | | | | .2 | .2 | .1 | .2 | .2 | .2 | .2 | .2 | .2 | .2 | .1 |
|---|---|

very much improved
much improved
minimally improved
no change
minimally worse
much worse
very much worse

Time (weeks)

Side Effects

| Headache | | Nausea Weakness | Weight Loss |
|---|---|---|---|

Legend

☆ Teacher   ○ Parent   ☐ Physician

▮ Medication 1   | Change in Medication Dosing 1

| Medication 2   ▯ Change in Medication Dosing 2

*Fig. 50*

**Survey**

☆ ◻ ○    ☆ ○    ☆ ○    ☆ ◻ ○    ☆ ○

| | very much improved | much improved | minimally improved | no change | minimally worse | much worse | very much worse |
|---|---|---|---|---|---|---|---|

very much improved: .2 .2 .1 .2 .2 .2 .2 .2 .2 .1 .2 .2 .2 .2 .2 .2 .2 .1 .1 .2 .3

much improved: .2 .2 .2 .6 .6 .8 .6 .6 .6 .6 .6 .6 .8 .6 .6 .6 .6 .6 .5 .5 .5 .5 .4 .3

minimally improved: .1 .2 ... .2 1 .2 .6 .6 .6 .2 .2 .1 .2 .2 .2 .2 .2 .2 .1 .2 .2 .2 .2 .2 .2 .2 .3 .3 .3 .3

no change: 1 .8 .6 .2 .2 .2 .2 ... .2 .2 .2 .6 8 .6 .2 .2 .2 ... .1 .1 .1 .1 .1 .1

minimally worse: .1 .2 .6 .6 .6 .6 .2 .1 .2 .6 .6 .6 .2 1 .2

much worse: .2 .2 .2 .2 .6 .8 .6 .2 .2 .2

very much worse: .2 .1 .2

**Time (weeks)**

**Side Effects**

| | Headache | | | Nausea Weakness | Weight Loss |
|---|---|---|---|---|---|

**Legend**

☆ Teacher    ○ Parent    ◻ Physician

| Medication 1    Change in Medication Dosing 1

Medication 2    Change in Medication Dosing 2

*Fig. 51*

*Fig. 52*

EP 4 410 199 B1

very much improved

much improved

minimally improved

no change

minimally worse .1

much worse .8

very much worse .1

**Medication 1** →

.1

.8

.1

| Side Effects | | | | |
|---|---|---|---|---|
| Headache | 1 | 0 | 0 |
| Nausea | 1 | 0 | 0 |
| Diarrhea | 1 | 0 | 0 |
| Fatigue | 0 | 1 | 0 |
| Sleep changes | 1 | 0 | 0 |
| Depression | 1 | 0 | 0 |

Current State

| Headache | .6 | .3 | .1 |
|---|---|---|---|
| Nausea | .1 | .2 | .7 |
| Diarrhea | .6 | .2 | .2 |
| Fatigue | .1 | .3 | .6 |
| Sleep changes | .8 | .2 | 0 |
| Depression | .2 | .6 | .2 |

Future State

very much improved

much improved

minimally improved

no change

minimally worse .1

much worse .8

very much worse .1

**Medication 2** →

.8

.2

| Side Effects | | | | |
|---|---|---|---|---|
| Headache | 1 | 0 | 0 |
| Nausea | 1 | 0 | 0 |
| Diarrhea | 1 | 0 | 0 |
| Fatigue | 0 | 1 | 0 |
| Sleep changes | 1 | 0 | 0 |
| Depression | 1 | 0 | 0 |

Current State

| Headache | .6 | .3 | .1 |
|---|---|---|---|
| Nausea | .3 | .3 | .3 |
| Diarrhea | .3 | .3 | .3 |
| Fatigue | .1 | .8 | .1 |
| Sleep changes | .8 | .2 | 0 |
| Depression | .1 | .2 | .7 |

Future State

*Fig. 53*

Fig. 54. An example implementation of the "Unit Summary View" screen

Fig. 55. An example implementation of the "Patient View Risks" screen

Fig. 56. An example implementation of the "Patient View Risks" screen

Fig. 57. An example implementation of the "Patient View Risks" screen

Fig. 58. An example implementation of the "Patient View Risks" screen

Fig. 59. An example implementation of the "Patient View Risks" screen

82

Fig. 60. An example implementation of the "Patient View Risks" screen

Fig. 61. An example implementation of the "Patient View Risks" screen

Fig. 62. An example implementation of the "Patient View Risks" screen.

Fig. 63. An example implementation of the "Patient View Risk Trajectory" screen.

Fig. 64. An example implementation of the "Patient View Measures" screen.

Fig. 65. An example implementation of the "Patient View Chart" screen.

Fig. 66. An example workflow for the user interface.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1844725 B1, Hartlep
- US 20070239043 A1, Patel
- US 20120022336 A1
- US 20030065535 A1, Karlov
- US 61699492
- US 38161003 B

- US 61684241
- US 8161001 B
- WO 61614861 A
- WO 61614846 A
- WO 61620144 A
- WO 61684241 A

**Non-patent literature cited in the description**

- **P. A. CHECHIA** ; **P.C. LAUSSEN.** The cardiac intensive unit perspective on hemodynamic monitoring of oxygen transport balance. *Pediatric Critical Medicine*, 2011, vol. 12 (4), S69-S71
- **SPENCELEY, NEIL et al.** Monitoring in pediatric cardiac critical care: A worldwide perspective. *Pediatric Critical Care Medicine*, 2011, vol. 12 (4), S76-S80
- **BOHN, D.** Objective assesment of cardiac output in infants after cardiac surgery. *Seminars in Thoracic and Cardiovascular Surgery: Pediatric Cardiac Surgery Annual*, 2011, vol. 14 (1), 19-23
- **G. M. HOFFMAN** ; **N. S. GHANAYEM** ; **J. M. KAMPINE** ; **S. BERGER** ; **KATHLEEN A MUSSAT-TO** ; **S.B. LITWIN** ; **J. S. TWEDDELL.** Venous saturation and the anaerobic threshold in neonates after the Norwood procedure for hypoplastic left heart syndrome. *The Annals of Thoracic Surgery*, 2000, vol. 70 (5)
- **S. M. BRADLEY** ; **A. M. ATZ**. Postoperative management: The role of mixed venous oxygen saturation monitoring. *Seminars in Thoracic and Cardiovascular Surgery: Pediatric Cardiac Surgery Annual*, 2005, vol. 8 (1), 22-27
- **PINILLA, J et al.** Study of the incidence of intravascular catheter infection and associated septicemia in critically ill patients. *Critical Care Medicine*, 1983, vol. 11, 21-25
- **DK, WARREN** ; **WW, QUADIR** ; **CS, HOLLENBEAK.** Attributable cost of catheter-associated bloodstream infections among intensive care patients in a non-teaching hospital. *Critical Care Medicine*, 2006, vol. 34, 2084-9
- **S. CHAKRAVARTI** ; **S SRIVASTAVA** ; **A. J. C. MITNACHT.** Near Infrared Spectroscopy (NIRS) in Children. *Seminars in Cardiothorasic and Vascular Anesthesia*, 2008, vol. 12 (70)

- **TORTORIELLO TA** ; **STAYER SA** ; **MOTT AR** ; **MCKENZIE ED** ; **FRASER CD** ; **ANDROPOULOS DB** ; **CHANG AC.** A noninvasive estimation of mixed venous oxygen saturation using near-infrared spectroscopy by cerebral oximetry in pediatric cardiac surgery patients. *Pediatric Anesthesia*, 2005, vol. 15 (6), 495-503
- **P. M. KIRSHBOM** ; **J. M. FORBESS** ; **B. E. KOGON** ; **J. M. SIMSIC et al.** Cerebral Near Infrared Spectroscopy Is a Reliable Marker of Systemic Perfusion in Awake Single Ventricle Children. *PEDIATRIC CARDIOLOGY*, 2007, vol. 28 (1), 42-45
- **N. NAGDYMAN** ; **T. FLECK** ; **S. BARTH** ; **H. ABDUL-KHALIQ** ; **B. STILLER.** Relation of cerebral tissue oxygenation index to central venous oxygen saturation in children. *INTENSIVE CARE MEDICINE*, 2004, vol. 30 (3), 468-471
- **MCQUILLEN PS** ; **NISHIMOTO MS** ; **BOTTRELL CL** ; **FINEMAN LD** ; **HAMRICK SE** ; **GLIDDEN DV** ; **AZAKIE A** ; **ADATIA I** ; **MILLER SP.** Regional and central venous oxygen saturation monitoring following pediatric cardiac surgery: concordance and association with clinical variables. *Pediatric Critical Care Medicine*, 2007, vol. 8 (2), 154-160
- **BHUTTA AT** ; **FORD JW** ; **PARKER JG** ; **PRODHAN P** ; **FONTENOT EE** ; **SEIB PM** ; **STROOPE BI** ; **FRAZIER EA** ; **SCHMITZ ML** ; **DRUMMOND-WEBB JJ**. Noninvasive cerebral oximeter as a surrogate for mixed venous saturation in children. *Pediatric Cardiology*, 2007, vol. 28 (1), 34-41
- **TAEED, R.** ; **SCHWARTZ, S. M.** ; **J. M. PEARL et al.** Unrecognized Pulmonary Venous Desaturation Early After Norwood Palliation Confounds p:s Assessment and Compromises Oxygen Delivery. *Circulation*, 2001, vol. 103, 2699-2704